# EUROPEAN PATENT APPLICATION

(11) **EP 3 945 261 A1**
(43) Date of publication of application: **02.02.2022**
(21) Application number: 21187886.3
(22) Date of filing: 27.07.2021
(51) Int. Cl.: F24F 13/065, F24F 8/108, F24F 8/22, F24F 13/10, F24F 13/20, F24F 1/0287, F24F 1/0353, F24F 8/80

(54) **PORTABLE AIR CARING APPARATUS**

(30) Priority: 27.07.2020 KR 20200093402; 23.11.2020 KR 20200158166; 14.12.2020 KR 20200174527; 14.12.2020 KR 20200174898; 30.12.2020 KR 20200188334
(71) Applicant: LG Electronics Inc., Seoul 07336 (KR)
(72) Inventor: OH, Si Young, 08592 Seoul (KR); CHOI, Jinwook, 08592 Seoul (KR); KIM, Kidong, 08592 Seoul (KR); CHOI, Seok-Ho, 08592 Seoul (KR); KIM, Juhyun, 08592 Seoul (KR)
(74) Representative: Vossius & Partner Patentanwälte Rechtsanwälte mbB

(57) **Abstract**

Disclosed herein is a portable air caring apparatus. The portable air caring apparatus according to the present invention includes an inlet portion configured to form a path along which air is suctioned, a filter portion disposed at an upper side of the inlet portion and configured to purify air which enters through the inlet portion and moves upward, a sanitizing portion disposed at a lower side of the filter portion, installed at a height allowing the sanitizing portion to overlap the inlet portion, and configured to irradiate sanitizing light toward the filter portion, and a fan module portion disposed at an upper side of the filter portion and configured to rotate a fan to blow air in a direction toward the upper side of the filter portion. (Fig. 2)

## Description

### CROSS-REFERENCE TO RELATED APPLICATION

This application claims priority to and the benefit of Korean Patent Application No. 10-2020-0093402 filed on July 27, 2020, Korean Patent Application No. 10-2020-0158166 filed on November 23, 2020, Korean Patent Application No. 10-2020-0174527 filed on December 14, 2020, Korean Patent Application No. 10-2020-0174898 filed on December 14, 2020, and Korean Patent Application No. 10-2020-0188334 filed on December 30, 2020, the disclosure of which is incorporated herein by reference in its entirety.

### BACKGROUND

### 1. Field of the Invention

The present invention relates to a portable air caring apparatus, and more particularly, to a portable air caring apparatus capable of discharging purified air upward.

### 2. Discussion of Related Art

An air caring apparatus is an apparatus widely used in modern life that purifies air by filtering physical particles such as dust, fine dust, and ultrafine dust, chemical substances such as odorous particles and harmful gases, and microorganisms such as bacteria and viruses.

Due to the influence of urbanization, industrialization, and globalization, the air caring apparatus has become an indispensable apparatus also in general homes. Also, the demand for the air caring apparatus has also sharply increased due to the influence of an increase in fine dust levels, an increase in the number of allergy patients, an improvement in the standard of living, and the like.

The air caring apparatus may have a large size when targeting an environment larger than 100 m², such as a general home. In the apparatus, a filter that handles physical particles such as dust, a filter that handles chemical substances such as gases, and a filter that handles microorganisms such as bacteria and viruses may be used in combination. That is, in a large space, an air caring apparatus of a large size that can accommodate various filters together may be used.

However, using an air caring apparatus of a large size in a small space such as a studio apartment or the inside of a vehicle, an extremely large space such as a public library, or outdoors is inefficient in terms of space utilization, mobility, and energy consumption. Also, for users who often move from place to place, an air caring apparatus that has a small size and can be easily carried for use by an individual is more suitable than an air caring apparatus having a large size. Under such a background, a portable air caring apparatus that can be easily carried for use by an individual is being developed.

The portable air caring apparatus is provided in a small, lightweight form so that it is easy to carry. The portable air caring apparatus has an advantage in that it can easily be carried and used at a desired location by a user. That is, the portable air caring apparatus is an apparatus suitable for users who tend to frequently go out or move from place to place rather than staying in one place, such as their home, for a long period of time.

Related Art 1 (U.S. Patent Publication No. 2020-0061231 A1, Title of Invention: Air purifier) is an invention relating to an air cleaner in which outside air is suctioned through a lower side of a front surface and then moved upward through a body portion. The air moved upward through the inside of the body portion sequentially passes through a plurality of filters and a fan and then is discharged to an upper side of the body portion through an outlet.

However, since a suction port of Related Art 1 is formed in one side surface of a lower portion of the body portion, in a case in which the air cleaner is mounted on a structure such as a cup holder having the shape of a groove which is concave toward the lower side, there is a problem in that the suction port configured to suction air interferes with the structure and the amount of suctioned air is decreased such that an air purification rate is decreased.

Also, since the suction port configured to suction air is only formed at one side of the body portion in Related Art 1, there is a problem in that a flow rate of air being suctioned into the body portion is decreased, and an installation position of the product is limited in consideration of an air suctioning direction for air cleaning.

Also, since the suction port configured to suction air is only installed at one side of the filter and movement of air mostly occurs through one side of the filter that is close to the suction port in Related Art 1, there is a problem in that the air cleaning efficiency is degraded.

Also, since a sanitizing apparatus is disposed between the fan and the filter and is installed across the inside of the body portion in Related Art 1, there is a problem in that an area of the sanitizing apparatus that comes in contact with air moving toward the fan after passing through the filter is increased and resistance of an air flow path is increased such that the air cleaning efficiency is degraded.

In addition, since, despite the discharge of purified air being performed through the upper side of the body portion, a separate apparatus for controlling an air discharge direction is not provided in Related Art 1, there is a problem in that the purified air is not smoothly supplied toward the face of a user.

Related Art 2 (Korean Patent Publication No. 10-2010-0063548 A, Title of Invention: Air cleaner having diagonal flow fan) is an invention relating to an air cleaner in which outside air is suctioned through suction grilles disposed at a lower side of a main body case and then moved upward along the main body case. The air moved upward through the inside of the main body case sequentially passes through a plurality of filters and a diagonal flow fan and then is discharged to an upper side of the main body case through an outlet.

However, since a suction port of Related Art 2 is formed in a bottom surface of a lower portion of the main body case, in a case in which the air cleaner is mounted on a structure such as a cup holder having the shape of a groove which is concave toward the lower side, there is a problem in that the suction port configured to suction air interferes with the structure and the amount of suctioned air is decreased such that an air purification rate is decreased.

Also, since the suction port configured to suction air is formed along an edge of a lower surface of the main body case in Related Art 2, there is a problem in that, as compared to the product in which the suction port is formed along the entire outer periphery of the main body case, a flow rate of air being suctioned into the main body case is decreased.

Also, since a separate sanitizing apparatus for sanitizing the filter is not provided in Related Art 2, there is a problem in that the filter is prone to contamination.

In addition, since despite the discharge of purified air being performed through the upper side of the main body case, a separate apparatus for controlling an air discharge direction is not provided in Related Art 2, there is a problem in that the purified air is not smoothly supplied toward the face of a user.

### SUMMARY OF THE INVENTION

The present invention is directed to providing a portable air caring apparatus which is capable of being mounted on a structure such as a cup holder having the shape of a groove concave toward the lower side and in which an inlet portion configured to suction air does not interfere with the structure.

The present invention is also directed to providing a portable air caring apparatus capable of addressing a decrease in a flow rate of suctioned air and a limitation in an installation position of the product due to the inlet portion, which is configured to suction air, only being installed in one direction.

The present invention is also directed to providing a portable air caring apparatus allowing air entering through the inlet portion to evenly pass through a filter portion so that air cleaning efficiency is improved.

The present invention is also directed to providing a portable air caring apparatus capable of minimizing resistance of a flow path of a sanitizing apparatus that comes in contact with an air flow path passing through the filter portion.

The present invention is also directed to providing a portable air caring apparatus capable of easily controlling a discharge direction of air being discharged to an upper side of the product.

The present invention is also directed to providing a portable air caring apparatus capable of preventing a finger of a user from coming in contact with an inner side of a fan module.

The present invention is also directed to providing a portable air caring apparatus capable of decreasing air resistance generated between air moving into the fan module and a safety device installed at an outer side of the fan module.

The present invention is also directed to providing a portable air caring apparatus capable of providing a guide for switching the air discharge direction.

The present invention is also directed to providing a portable air caring apparatus having a separate guide for guiding a movement direction of air between an air blowing fan and a discharge portion.

Objectives of the present invention are not limited to the above-mentioned objectives, and other unmentioned objectives of the present invention and advantages thereof should be understood from the following description and should be more clearly understood from embodiments of the present invention. Also, it should be easily understood that the objectives and advantages of the present invention may be realized by means shown in the claims below and combinations thereof.

To achieve the above objectives, a portable air caring apparatus according to the present invention has a first case portion, which includes an inlet portion, installed at an upper side of a second case portion.

Specifically, since the first case portion is connected to the upper side of the second case portion, in which electronic components are installed, and the inlet portion configured to suction air is installed in the first case portion, air may be suctioned through the inlet portion in a state in which the first case portion is inserted into a structure such as a cup holder having the shape of a groove which is concave toward the lower side.

Also, in the portable air caring apparatus according to the present invention, the inlet portion configured to suction air is installed along an outer periphery of the first case portion.

Specifically, since the inlet portion configured to suction outside air is installed along the outer periphery of the first case portion, air outside the first case portion may move into the first case portion through the inlet portion, and thus a suction flow rate of air may be increased.

Also, in the portable air caring apparatus according to the present invention, in a state in which the inlet portion configured to suction air is installed along the outer periphery of the first case portion, a filter portion is installed at an upper side spaced apart from the inlet portion.

Specifically, the outside air that enters the first case portion through the inlet portion, which is installed along the outer periphery of the first case portion, moves upward and evenly passes through the filter portion.

Also, the portable air caring apparatus according to the present invention decreases resistance of a flow path of a sanitizing portion installed to sanitize the filter portion.

Specifically, since the sanitizing portion is disposed at the central portion of the first case portion and the air that enters through the inlet portion moves upward while moving around an outer periphery of the sanitizing portion, the resistance of the flow path of the sanitizing portion is decreased.

Also, in the portable air caring apparatus according to the present invention, the discharge portion installed at an upper side of the first case portion is rotatably installed.

Specifically, since the discharge portion which is open in the vertical direction is rotatably connected to the upper side of the first case portion, a discharge direction of air that has passed through a fan module portion may be controlled.

The portable air caring apparatus according to the present invention may include at least any one of a housing portion, the filter portion, the fan module portion, the discharge portion, and the sanitizing portion.

The housing portion includes the inlet portion, has the filter portion, the sanitizing portion, and the fan module portion disposed therein, and forms an air flow path in the vertical direction. Also, a cylindrical air flow path is formed inside the housing portion. Also, along a vertical reference line that passes through the center of the housing portion in the vertical direction, the center of the inlet portion, the center of the filter portion, the center of the sanitizing portion, and the center of the fan module portion may coincide in the vertical direction. Also, the housing portion may include the first case portion and the second case portion.

The first case portion may have an accommodation space formed therein and have the filter portion, the sanitizing portion, and the fan module portion installed therein.

The inlet portion configured to suction air may be disposed at a side surface of a lower portion of the first case portion. Also, an outlet configured to discharge air may be disposed at the upper side of the first case portion. Also, the first case portion may include at least any one of a first case, a second case, and an intermediate case.

The first case may include the inlet portion configured to suction air and may be coupled to the upper side of the second case portion.

Also, the inlet portion may be installed along the outer periphery of the first case portion. Also, the inlet portion may include a plurality of inlet holes provided to guide movement of air into the first case portion. Also, the inlet hole may be formed as a slot-shaped hole. Also, the inlet hole may be installed to be inclined in one direction along the outer periphery of the first case portion. Also, air that enters the first case portion through the inlet hole may rotate in a spiral and move to the filter portion at the upper side.

Also, the first case may further include a filter fixing protrusion configured to fix the filter portion. The filter fixing protrusion may protrude from an upper portion of the first case to the inside of the first case and support a lower portion of the filter portion.

Also, the second case may be disposed at an upper side of the first case and include an outlet configured to discharge purified air. Also, the second case may rotatably support the discharge portion. Also, the second case may include at least any one of a second case body, a tubular expansion member, and a rotation support portion.

The second case body may be installed in a shape that surrounds an outer periphery of the fan module portion. Also, the tubular expansion member may extend to an upper side of the second case body and have the shape of a tube in which an inner path gradually widens upward.

Also, the rotation support portion may be connected to at least any one of the second case body and the tubular expansion member. Also, the rotation support portion may rotatably support the discharge portion at the center of an outlet disposed in the tubular expansion member. Also, the rotation support portion may include at least any one of a core member, a support, and a ball joint.

The core member may be disposed at a lower side of the discharge portion and may extend in a direction from the center of the outlet toward the discharge portion. Also, the support may extend to an outer side of the core member and may be connected to at least any one of the second case body and the tubular expansion member to restrict movement of the core member. Also, the support may be provided as a plurality of supports and installed in the shape of a rod. Also, the ball joint may be coupled to the core member and rotatably support the discharge portion. Also, an end portion of the ball joint may be formed in a spherical shape and coupled to an inner side of the discharge portion to rotatably support the discharge portion.

The intermediate case may be installed in a shape that surrounds the outer side of the filter portion and may connect the first case and the second case. Also, the intermediate case may be formed in a cylindrical shape.

Also, the second case portion may be connected to the lower portion of the first case portion. Also, electronic components including a battery may be installed inside the second case portion. Also, an air flow path is formed inside the first case portion while an air flow path is not formed inside the second case portion. Also, at least any one of the first case portion and the second case portion may be formed as a cylindrical case. Both the first case portion and the second case portion may be formed in a cylindrical shape. Alternatively, only the second case portion may be formed in a cylindrical shape. Alternatively, only the first case portion may be formed in a cylindrical shape.

The filter portion may be installed inside the first case portion and purify air entering through the inlet portion. Also, the filter portion may be formed in a cylindrical shape that extends in the vertical direction. Also, the height of the filter portion may be set to be higher than the height of the inlet portion.

The fan module portion may be disposed between the filter portion and the outlet and may rotate a fan to blow air in a direction toward the outlet. Also, the fan module portion may include a fan housing, a fan member, and a fan base.

Also, the fan module portion may be disposed at a lower side of the rotation support portion. Also, the fan module portion may include the fan housing which is fixed to the first case portion. Also, the fan housing may include a support plate disposed at a lower side of the core member and a connection support which extends toward an outer side of the support plate in a radial direction. Also, the connection support and the support may overlap in the vertical direction to decrease resistance of an air flow path.

The fan housing may be fixed to an inner side of the first case portion and have a space formed therein to allow rotation of the fan member. Also, the fan member may be rotatably installed inside the fan housing and may move air in a direction toward the discharge portion. Also, a diagonal flow fan may be used as the fan member. Also, the fan member may include at least any one of a hub, a fan blade, and a shroud.

The hub may be disposed at the center of the fan housing and may receive external power and rotate. Also, the fan blade may be provided as a plurality of fan blades, and the plurality of fan blades may be disposed to be spaced apart at equal intervals along an outer peripheral surface of the hub. Also, the shroud may be connected to an end portion of the fan blade, installed in an annular shape, and spaced apart from the fan base. Also, an outer diameter of the hub and an inner diameter of the shroud may gradually become smaller in a direction from an upper side toward a lower side.

Also, the fan base may be coupled to a lower side of the fan housing and guide air, which passed through the filter portion, to enter the fan member.

The discharge portion may be rotatably installed in the first case portion and may control a discharge direction of air that has passed through the fan module portion. Also, the discharge portion may be rotatably installed at the spherical ball joint disposed in the first case portion. Also, the discharge portion may include a first discharge portion which is disposed at one side of the ball joint and has a plurality of vanes provided to guide discharge of air. Also, the discharge portion may include a second discharge portion which is disposed at the other side of the ball joint, connected to the first discharge portion, and configured to rotate about the ball joint along with the first discharge portion.

Also, the center of rotation of the discharge portion may coincide with the center of the fan module portion in the vertical direction. Also, air that enters through the inlet portion may move upward and sequentially pass through the filter portion, the fan module portion, and the discharge portion.

Also, the sanitizing portion may be disposed between the filter portion and the second case portion and may irradiate the filter portion with sanitizing light. Also, the sanitizing portion may be disposed at a lower side of the filter portion and may be installed at a height at which the sanitizing portion overlaps the inlet portion. Also, the sanitizing portion may include at least any one of a sanitization support portion, a pedestal portion, and an irradiating portion.

The sanitization support portion may be disposed between the first case portion and the second case portion. Also, the sanitization support portion may block the lower portion of the first case portion. Also, the sanitization support portion may be disposed at a lower side of the irradiating portion and connected to the housing portion such that movement of the sanitization support portion is restricted.

Also, the pedestal portion may protrude to an upper side of the sanitization support portion to support the lower side of the irradiating portion. Also, the pedestal portion may be disposed at the center of the inlet portion, and a transverse cross-section of the pedestal portion may be formed in a circular shape.

Also, the pedestal portion may be formed in a columnar shape that protrudes upward from the center of the sanitization support portion. Also, the pedestal portion may be formed in a cylindrical or conical shape. Also, the pedestal portion may be disposed at the center of the first case portion in which the inlet portion is formed, and a transverse cross-section of the pedestal portion may be formed in a circular shape.

Also, the irradiating portion may be mounted on an upper side of the pedestal portion and may irradiate the sanitizing light in a direction toward the filter portion. Also, the irradiating portion may be installed at a position that is level with or higher than an upper end of the inlet portion. Also, the irradiating portion may be disposed on a vertical reference line that passes through the center of the inlet portion in the vertical direction.

Also, when a distance between the fan module portion and the filter portion is L1 and a distance between the filter portion and the irradiating portion is L2, L1 may be shorter than L2.

Also, D1, which is a length of the inlet portion in the vertical direction, may be formed to be shorter than D2, which is a length of the filter portion in the vertical direction. Also, D1 may be formed to be greater than 0.57 times D2 and less than 0.77 times D2.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a front view of a portable air caring apparatus according to a first embodiment of the present invention;
FIG. 2 is a cross-sectional view of the portable air caring apparatus according to the first embodiment of the present invention;
FIG. 3 is an exploded cross-sectional view of the portable air caring apparatus according to the first embodiment of the present invention;
FIG. 4 is an exploded perspective view of the portable air caring apparatus according to the first embodiment of the present invention that is viewed from an upper side;
FIG. 5 is an exploded perspective view of the portable air caring apparatus according to the first embodiment of the present invention that is viewed from a lower side;
FIG. 6 is a perspective view illustrating a rotation support portion according to the first embodiment of the present invention;
FIG. 7 is a perspective view illustrating a state in which the rotation support portion is installed at an upper side of a fan module portion according to the first embodiment of the present invention;
FIG. 8 is a perspective view illustrating the fan module portion according to the first embodiment of the present invention;
FIG. 9 is an exploded perspective view of the fan module portion according to the first embodiment of the present invention;
FIG. 10 is a plan view of the fan module portion according to the first embodiment of the present invention;
FIG. 11 is a bottom view of the fan module portion according to the first embodiment of the present invention;
FIG. 12 is a cross-sectional view of the fan module portion according to the first embodiment of the present invention;
FIG. 13 is a perspective view of a fan member according to the first embodiment of the present invention;
FIG. 14 is a plan view of the fan member according to the first embodiment of the present invention;
FIG. 15 is a bottom view of the fan member according to the first embodiment of the present invention;
FIG. 16 is a front view of the fan member according to the first embodiment of the present invention;
FIG. 17 is a cross-sectional view of the fan member according to the first embodiment of the present invention;
FIG. 18 is a perspective view illustrating another fan module portion according to the first embodiment of the present invention;
FIG. 19 is an exploded perspective view illustrating the fan module portion according to the first embodiment of the present invention;
FIG. 20 is a cross-sectional view illustrating a flow of air passing through the portable air caring apparatus according to the first embodiment of the present invention;
FIG. 21 is a cross-sectional view in which the portable air caring apparatus according to the first embodiment of the present invention is divided in a vertical longitudinal direction;
FIG. 22 is a plan view illustrating a state in which air that has passed through an inlet portion moves while rotating in a spiral around a sanitizing portion according to the first embodiment of the present invention;
FIG. 23 is a cross-sectional view illustrating air passing through the fan module portion according to the first embodiment of the present invention;
FIG. 24 is a perspective view illustrating a fan module portion and a filter portion according to a second embodiment of the present invention;
FIG. 25 is a perspective view of the fan module portion according to the second embodiment of the present invention;
FIG. 26 is an exploded perspective view of the fan module portion according to the second embodiment of the present invention;
FIG. 27 is a plan view of a safety portion according to the second embodiment of the present invention;
FIG. 28 is a plan view illustrating a fan base and the safety portion according to the second embodiment of the present invention;
FIG. 29 is a plan view illustrating a fan member and the safety portion according to the second embodiment of the present invention;
FIG. 30 is a view in which portions of the fan member and the fan base that face a blocking portion are indicated according to the second embodiment of the present invention;
FIG. 31 is a plan view of the fan module portion according to the second embodiment of the present invention;
FIG. 32 is an exploded perspective view of a portable air caring apparatus according to the second embodiment of the present invention;
FIG. 33 is a cross-sectional view of the portable air caring apparatus according to the second embodiment of the present invention;
FIG. 34 is a view illustrating a flow of air passing through the portable air caring apparatus according to the second embodiment of the present invention;
FIG. 35 is a cross-sectional view of a portable air caring apparatus according to a third embodiment of the present invention;
FIG. 36 is an exploded cross-sectional view of the portable air caring apparatus according to the third embodiment of the present invention;
FIG. 37 is an exploded perspective view of the portable air caring apparatus according to the third embodiment of the present invention;
FIG. 38 is a perspective view illustrating a state in which a rotation support portion is installed in a housing portion according to the third embodiment of the present invention;
FIG. 39 is a cross-sectional view illustrating the state in which the rotation support portion is installed in the housing portion according to the third embodiment of the present invention;
FIGS. 40 and 41 are cross-sectional views illustrating a guide vane according to the third embodiment of the present invention; and
FIG. 42 is a perspective view illustrating a fan module portion installed at a lower side of the rotation support portion according to the third embodiment of the present invention.

### DETAILED DESCRIPTION OF EXEMPLARY EMBODIMENTS

The objectives, features, and advantages will be described in detail below with reference to the accompanying drawings, and accordingly, those of ordinary skill in the art to which the present invention pertains should be able to easily practice the technical idea of the present invention. In describing the present invention, when it is determined that detailed description of a known art relating to the present invention may unnecessarily obscure the gist of the present invention, the detailed description thereof will be omitted. Hereinafter, exemplary embodiments according to the present invention will be described in detail with reference to the accompanying drawings. In the drawings, the same reference numerals are used to indicate the same or similar elements.

Although terms such as first and second are used to describe various elements, of course, the elements are not limited by the terms. The terms are only used to distinguish one element from another element, and of course, a first element may also be a second element unless otherwise stated.

Hereinafter, when an arbitrary configuration is described as being disposed on an "upper portion (or lower portion)" of an element or being disposed "above (or below)" the element, this may not only mean that the arbitrary configuration is disposed in contact with an upper surface (or lower surface) of the element but may also mean that another configuration may be interposed between the element and the arbitrary configuration disposed above (or below) the element.

Also, when a certain element is described as being "connected," "coupled," or "linked" to another element, this may mean that the element is directly connected or linked to the other element but may also mean that the element is "connected," "coupled," or "linked" to the other element via another element "interposed" therebetween or the element and the other element are "connected," "coupled," or "linked" through different elements.

Throughout the specification, each element may be provided as a single element or a plurality of elements unless particularly described otherwise.

In the specification, a singular expression includes a plural expression unless the context clearly indicates otherwise. In the application, terms such as "comprise" or "include" should not necessarily be interpreted as indicating that all of various elements or various steps described in the specification are included and should be interpreted as indicating that some of the elements or some of the steps may not be included or additional elements or steps may be further included.

Throughout the specification, "A and/or B" may refer to A, B, or A and B unless particularly described otherwise, and "C to D" may refer to C or more and D or less unless particularly described otherwise.

### [First embodiment - Exterior of portable air caring apparatus]

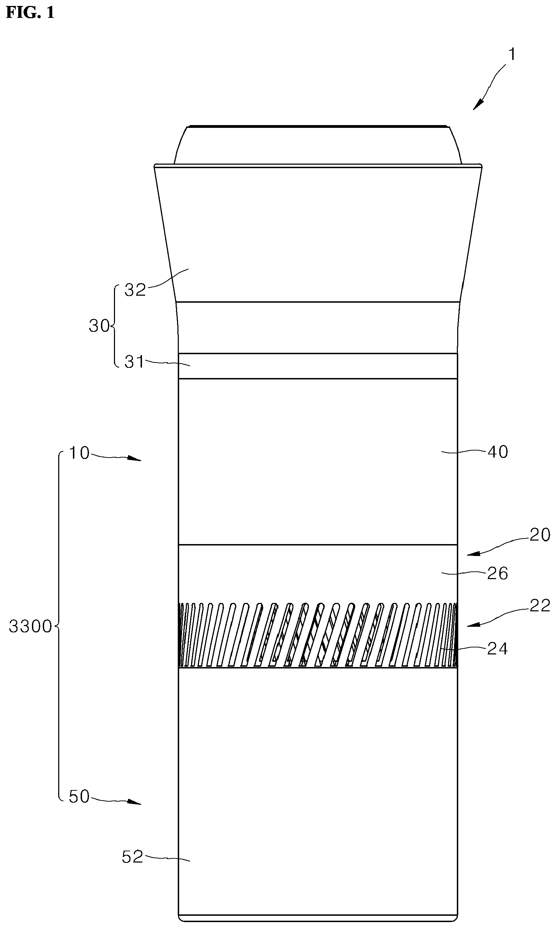

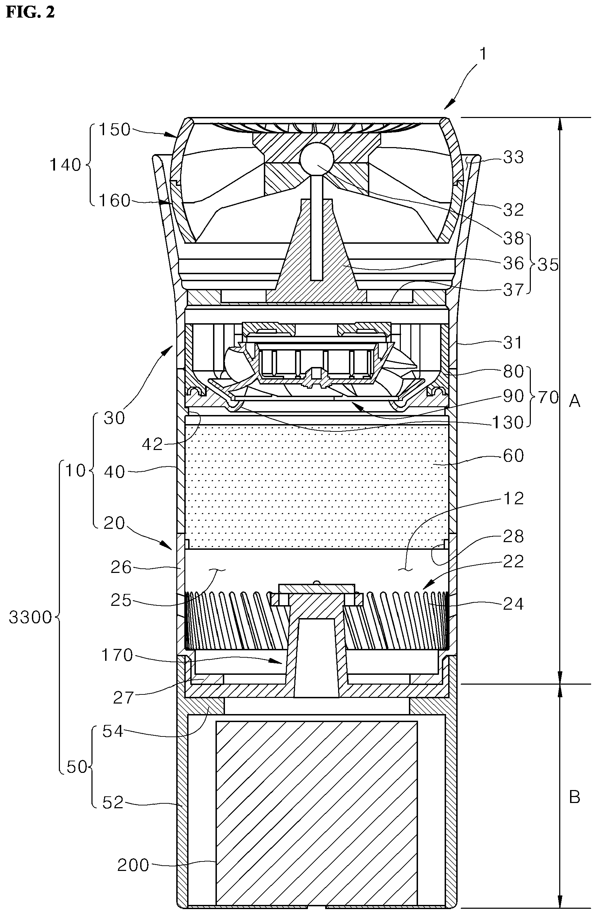

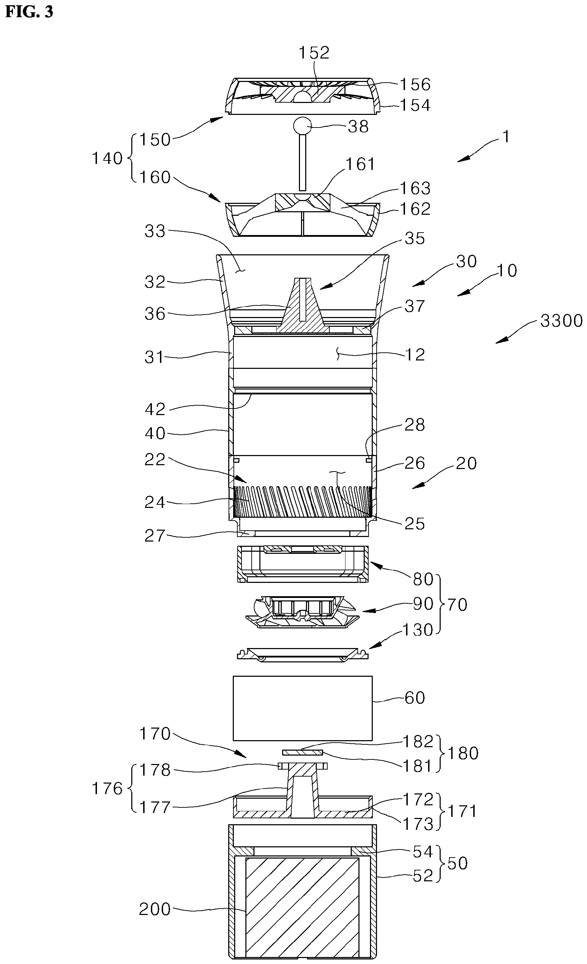

FIG. 1 is a front view of a portable air caring apparatus 1 according to a first embodiment of the present invention, FIG. 2 is a cross-sectional view of the portable air caring apparatus 1 according to the first embodiment of the present invention, and FIG. 3 is an exploded cross-sectional view of the portable air caring apparatus 1 according to the first embodiment of the present invention.

As illustrated in FIGS. 1 to 3, the portable air caring apparatus 1 according to an embodiment of the present invention may be formed in a substantially cylindrical shape. The portable air caring apparatus 1 may include at least any one of a housing portion 3300, a filter portion 60, a fan module portion 70, a discharge portion 140, and a sanitizing portion 170.

The housing portion 3300 may include an inlet portion 22, and the filter portion 60, the sanitizing portion 170, and the fan module portion 70 may be disposed in the housing portion 3300. Also, the housing portion 3300 forms an air flow path in a vertical direction. Since a cylindrical air flow path is formed inside the housing portion 3300, frictional resistance of air moving in the vertical direction may be reduced.

Also, along a vertical reference line that passes through the center of the housing portion 3300 in the vertical direction, the center of the inlet portion 22, the center of the filter portion 60, the center of the sanitizing portion 170, the center of the fan module portion 70, and the center of an outlet 33 may coincide in the vertical direction. Therefore, a flow of air, which moves from a lower side to an upper side along the housing portion 3300, moves in a straight line in the vertical direction, and a movement path of air is shortened. Thus, resistance of an air flow path is decreased, and air caring efficiency is improved.

In a case in which the portable air caring apparatus 1 is installed on a horizontal surface, the vertical reference line coincides with a vertical line.

Also, the housing portion 3300 may include a first case portion 10 and a second case portion 50. The portable air caring apparatus 1 according to the present embodiment is operated in a manner of suctioning air through a side surface of the first case portion 10 and discharging air upward.

The first case portion 10 and the second case portion 50 form a framework of an exterior of the portable air caring apparatus 1. The first case portion 10 and the second case portion 50 accommodate a plurality of components.

The first case portion 10 has an accommodation space formed therein and may have the filter portion 60, the sanitizing portion 170, and the fan module portion 70 installed therein. Also, in the first case portion 10, both sides may be formed to be open in a first direction. That is, an upper side and a lower side of the first case portion 10 may be open. The inlet portion 22 may be disposed at one side of the first case portion 10 in the first direction, and the outlet 33 and the discharge portion 140 may be disposed at the other side of the first case portion 10 in the first direction.

The second case portion 50 is connected to a lower side of the first case portion 10, and the second case portion 50 may be formed in a cylindrical shape.

The portable air caring apparatus 1 may be formed in a cylindrical shape that stands upright and is long in the vertical direction as a whole. Accordingly, a user may use the portable air caring apparatus 1 in a vertical state or a horizontal state. Also, in a place such as the inside of a vehicle where shaking of the portable air caring apparatus 1 may occur, since the portable air caring apparatus 1 is used in a state of being mounted in a groove portion such as a cup holder that is concave toward the lower side, the position of the portable air caring apparatus 1 may be stably maintained.

Directions will be defined. When a direction in which the discharge portion 140 is located from the first case portion 10 is referred to as "upper portion" and a direction in which the second case portion 50 is located from the first case portion 10 is referred to as "lower portion," "first direction" refers to a vertical direction or an axial direction. The first direction may also refer to a perpendicular direction. Also, "second direction" is a direction perpendicular to the first direction and refers to a lateral direction or a radial direction.

### [Overall structure of portable air caring apparatus]

The portable air caring apparatus 1 according to the present embodiment includes the first case portion 10, the second case portion 50, the filter portion 60, the fan module portion 70, the discharge portion 140, and the sanitizing portion 170.

The first case portion 10 and the second case portion 50 form the framework of the exterior of the portable air caring apparatus 1. The exterior of a side surface and a bottom surface of the portable air caring apparatus 1 are formed by the first case portion 10 and the second case portion 50. An accommodation space 12 is formed inside the first case portion 10 and the second case portion 50. The accommodation space 12 accommodates the filter portion 60, the fan module portion 70, the sanitizing portion 170, and electronic components including a battery 200. Preferably, the first case portion 10 and the second case portion 50 are formed to have a sufficient strength to protect the accommodated components from external impact.

The filter portion 60 is installed in the accommodation space 12 of the first case portion 10 and is disposed between the fan module portion 70 and the inlet portion 22. That is, the filter portion 60 is disposed at a lower portion of the fan module portion 70 and serves to purify air that is suctioned through the inlet portion 22 of the portable air caring apparatus 1. The air purified while passing through the filter portion 60 passes through the fan module portion 70 and the discharge portion 140 and is discharged to an upper portion of the portable air caring apparatus 1.

The filter portion 60 is installed inside the first case portion 10 and purifies air that enters through the inlet portion 22. Also, the filter portion 60 may be formed in a cylindrical shape extending in the vertical direction.

The filter portion 60 made be made of a single filter or, as necessary, installed in a state in which a plurality of filters are stacked. Also, the filter portion 60 may further include a separate filter case for fixing the filter.

The filter case is fixed to the inside of the first case portion 10, and an insertion space for accommodating the filter may be formed inside the filter case.

The fan module portion 70 is accommodated in the accommodation space 12 inside the first case portion 10 and may be disposed between the discharge portion 140 and the filter portion 60. More specifically, the fan module portion 70 may be disposed between the outlet 33 and the filter portion 60. That is, the fan module portion 70 is disposed at an upper portion of the filter portion 60, and the outlet 33 and the discharge portion 140 are disposed at an upper portion of the fan module portion 70. The fan module portion 70 serves to suction air, which enters a lower portion of the filter portion 60 through the inlet portion 22, and discharge the air to an upper portion of the first case portion 10.

The center of rotation of the discharge portion 140 may coincide with the center of the fan module portion 70 in the vertical direction. The air that enters through the inlet portion 22 moves upward, sequentially passes through the filter portion 60, the fan module portion 70, and the discharge portion 140, and is discharged to an upper side of the portable air caring apparatus 1.

In the present embodiment, the fan module portion 70 is illustrated as including a diagonal flow fan. The fan module portion 70 may suction air, which has passed through the filter portion 60, in the axial direction and discharge the air in a direction between the axial direction and radial direction.

The discharge portion 140 may be rotatably installed at the upper side of the first case portion 10 and may guide a discharge direction of air that has moved upward through the outlet 33. A rotation support portion 35 is disposed at the upper portion of the first case portion 10, and the discharge portion 140 is rotatably installed on the rotation support portion 35. Since both sides of the discharge portion 140 in the vertical direction are open, air that has moved to a lower portion of the discharge portion 140 through the outlet 33 may be discharged to the outside of the portable air caring apparatus 1 through an upper portion of the discharge portion 140.

The sanitizing portion 170 is disposed at the lower portion of the filter portion 60 and may be fixed to at least any one of the first case portion 10 and the second case portion 50. The sanitizing portion 170 is spaced a predetermined distance apart from the filter portion 60 and irradiates the filter portion 60 with sanitizing light. Since the sanitizing light irradiated by the sanitizing portion 170 is harmful to the human body, the installation position of the sanitizing portion 170 is set such that the sanitizing light does not leak to the outside of the portable air caring apparatus 1 through the inlet portion 22.

The battery 200 is installed in the accommodation space 12 provided inside the second case portion 50 and is disposed at a lower portion of the sanitizing portion 170. The battery 200 may supply power for driving the portable air caring apparatus 1.

### [Component arrangement structure of portable air caring apparatus]

The accommodation space 12 provided inside the portable air caring apparatus 1 may be divided into a first accommodation area A and a second accommodation area B. When the accommodation space 12 is divided in the vertical direction, an upper area is the first accommodation area A, and a lower area is the second accommodation area B. Note that the first accommodation area A and the second accommodation area B are not physically partitioned areas and are areas that are only conceptually divided.

In the first embodiment of the present invention, the accommodation space 12 of the first case portion 10 constituting the framework of the portable air caring apparatus 1 is set as the first accommodation area A, and the accommodation space 12 inside the second case portion 50 is set as the second accommodation area B.

Components relating to suctioning, purifying, and discharging air are disposed in the first accommodation area A. That is, since the inlet portion 22, the filter portion 60, the fan module portion 70, and the discharge portion 140 are disposed in the first accommodation area A, air flows from a lower side toward an upper side in the first accommodation area A.

In the first case portion 10, the inlet portion 22 having a plurality of inlet holes 24 formed therein is installed as a path for suctioning air. At the upper portion of the first case portion 10, the outlet 33 and the discharge portion 140 rotatably installed at the outlet 33 are installed as a path for discharging air that is purified in the first accommodation area A. Therefore, in the first case portion 10, an air flow path which connects the filter portion 60, the fan module portion 70, and the discharge portion 140 is formed.

That is, the inlet portion 22, the filter portion 60, the fan module portion 70, the discharge portion 140, and the outlet 33 are provided in the first accommodation area A, and a flow path necessary for the air, which is suctioned into the portable air caring apparatus 1, to pass through the air caring apparatus is formed in the first accommodation area A.

Components not directly related to a flow of air for purifying air are disposed in the second accommodation area B. That is, a controller, which includes a printed circuit board (PCB), and the battery 200 may be installed in the second accommodation area B.

According to the present embodiment, the first case portion 10 and the second case portion 50 are formed in a cylindrical shape in which a length in the vertical direction is longer than a length in the lateral direction. Also, the first accommodation area A disposed at an upper portion is formed to have a longer length in the vertical direction than the second accommodation area B disposed at a lower portion. That is, when the portable air caring apparatus 1 stands upright, the first accommodation area A at the upper portion occupies a larger area than the second accommodation area B at the lower portion.

The battery 200 may be installed inside the second case portion 50 in which the second accommodation area B is formed. The battery 200 may be provided to have a heavier weight than the sum of weights of the fan module portion 70, the filter portion 60, and the discharge portion 140.

Generally, since the weight per unit volume of the battery 200 is significantly heavier than the weight per unit volume of the fan module portion 70, the filter portion 60, and the discharge portion 140, even when the weight or size of the battery 200 is not intentionally increased, the battery 200 has a heavier weight than the fan module portion 70, the filter portion 60, and the discharge portion 140.

In this way, when the battery 200, which is a heavy object, is disposed in the lower portion of the portable air caring apparatus 1, the center of mass of the portable air caring apparatus 1 is biased toward the lower side from the center in the vertical direction. That is, the center of mass of the portable air caring apparatus 1 is moved toward the lower portion of the portable air caring apparatus 1 at which the battery 200 is disposed.

In this way, when the center of mass of the portable air caring apparatus 1 is biased toward the lower side of the portable air caring apparatus 1 at which the battery 200 is disposed, a risk of the portable air caring apparatus 1 falling down when the portable air caring apparatus 1 stands upright is reduced.

That is, when the portable air caring apparatus 1 stands upright, since the center of mass of the portable air caring apparatus 1 is located at the lower side due to the battery 200 being disposed at the lower portion of the portable air caring apparatus 1, the portable air caring apparatus 1 does not fall down easily.

Also, when the battery 200, which is a heavy object, is disposed in the lower portion of the portable air caring apparatus 1, other components constituting the portable air caring apparatus 1 should be disposed above the battery 200. That is, the components relating to suctioning, purifying, and discharging air should be disposed at a higher position than the battery 200.

In order to secure the charge capacity of the battery 200 that is necessary for smooth use of the portable air caring apparatus 1, the size of the battery 200 is required to be a predetermined size or larger. Therefore, an installation space of a predetermined size or more for installing the battery 200 is also required inside the portable air caring apparatus 1. Also, since it is difficult to form a flow path for an air flow in the space in which the battery 200 is installed, the components relating to suctioning, purifying, and discharging air are inevitably disposed at positions avoiding the battery 200, that is, positions higher than the battery 200.

Due to such an arrangement structure, the flow path for suctioning, purifying, and discharging air is formed in the first accommodation area A, which is at a higher position than the battery 200, in the portable air caring apparatus 1. Therefore, suctioning of air into the portable air caring apparatus 1 and discharge of air purified by the portable air caring apparatus 1 are also performed at a position higher than the position where the battery 200 is installed.

When the discharge of the purified air is performed at the upper portion of the portable air caring apparatus 1 as described above, it becomes easier for the air purified by the portable air caring apparatus 1 to reach the face of a user.

When the portable air caring apparatus 1 is used while placed on a floor surface at a lower position than the user's face, in order to increase an amount of air purified by the portable air caring apparatus 1 that reaches the user's face, using the portable air caring apparatus 1 in a vertical state is more advantageous than using the portable air caring apparatus 1 in a horizontal state.

To this end, when the portable air caring apparatus 1 stands upright, the amount of air purified by the portable air caring apparatus 1 that reaches the user's face may be further increased when the discharge of the purified air is performed at the upper portion of the portable air caring apparatus 1.

Also, the structure in which the battery 200, which is a heavy object, is disposed in the lower portion of the portable air caring apparatus 1 and, accordingly, the components relating to suctioning, purifying, and discharging air are disposed at a higher position than the battery 200 may also contribute to expanding a range of installation of the portable air caring apparatus 1.

For example, when the portable air caring apparatus 1 is used while inserted into a cup holder in a vehicle, an area where suctioning of air is performed and an area where purifying and discharging of air are performed may be disposed at a higher position than the cup holder. Accordingly, air caring performance may be maintained at a high level while the portable air caring apparatus 1 is stably mounted in the vehicle. To this end, preferably, the length of the second accommodation area B in the vertical direction in which the battery 200 is disposed may be set to be larger than or equal to a depth of the cup holder.

As another example, even in a case in which a lower area of the portable air caring apparatus 1 is fixed using a cradle in the form of tongs or the like, the portable air caring apparatus 1 can be stably fixed while an area of the portable air caring apparatus 1 where suctioning of air is performed and an area of the portable air caring apparatus 1 where discharging of the purified air is performed are not blocked.

Also, even in a case in which a user moves the portable air caring apparatus 1 while holding the lower portion of the portable air caring apparatus 1 by his or her hand, stable movement of the portable air caring apparatus 1 is possible without blocking the area of the portable air caring apparatus 1 where suctioning of air is performed and the area of the portable air caring apparatus 1 where discharging of the purified air is performed.

That is, since the components not directly related to a flow of air for purifying air are disposed at the lower portion of the portable air caring apparatus 1, and mounting and fixing of the portable air caring apparatus 1 are performed through the lower portion of the portable air caring apparatus 1, the portable air caring apparatus 1 may simultaneously provide air caring performance at a high level and be stably fixed.

In the portable air caring apparatus 1 according to the first embodiment of the present invention that has the above structure, the first case portion 10 including the inlet portion 22 is installed at an upper side of the second case portion 50. The first case portion 10 is connected to the upper side of the second case portion 50 in which electronic components are installed, and the inlet portion 22 configured to suction air is provided in the first case portion 10. Therefore, in a state in which the first case portion 10 is inserted into a structure such as a cup holder having the shape of a groove which is concave toward the lower side, air is easily suctioned into the first case portion 10 through the inlet portion 22 and then discharged to the upper side of the first case portion 10. Thus, air cleaning efficiency may be improved.

### [Detailed configuration of components of portable air caring apparatus 1]

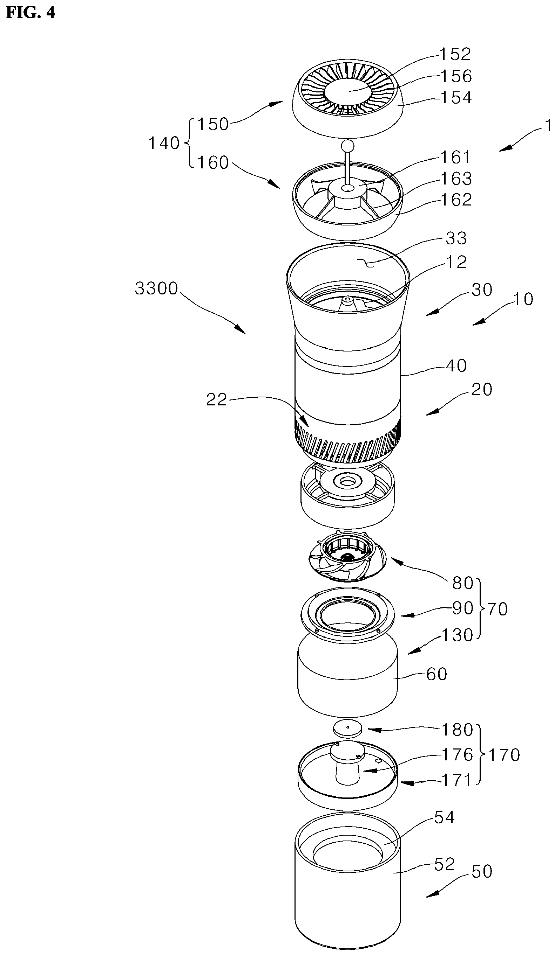

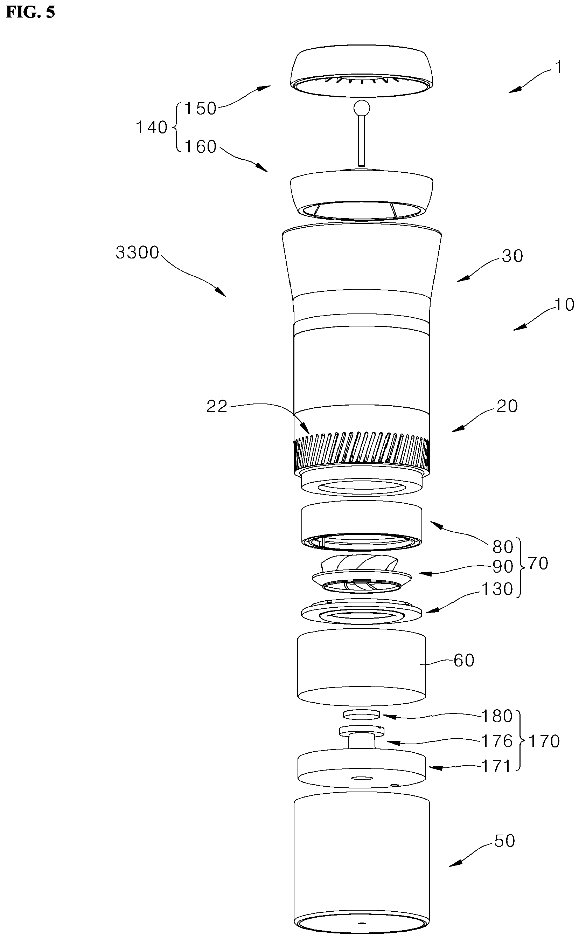

FIG. 4 is an exploded perspective view of the portable air caring apparatus 1 according to the first embodiment of the present invention that is viewed from an upper side, and FIG. 5 is an exploded perspective view of the portable air caring apparatus 1 according to the first embodiment of the present invention that is viewed from a lower side.

As illustrated in FIGS. 4 and 5, the portable air caring apparatus 1 according to the first embodiment of the present invention may include at least any one of the first case portion 10, the second case portion 50, the filter portion 60, the fan module portion 70, the discharge portion 140, the sanitizing portion 170, and the battery 200.

### [First case portion]

The accommodation space 12 may be formed inside the first case portion 10, and the inlet portion 22 configured to suction air may be provided at a lower side surface of the first case portion 10. The first case portion 10 may be formed in a cylindrical shape and installed in a shape in which the upper side and lower side thereof are open. The first case portion 10 may consist of a single member or may consist of a plurality of members as necessary. The first case portion 10 according to the first embodiment of the present invention may consist of a plurality of members, and each member may be coupled by fitting or coupled using an adhesive, or the members may be connected to each other using a fastening member such as a bolt. In this way, various modifications are possible.

Meanwhile, air is suctioned through the lower side surface of the first case portion 10, and air is discharged through the upper side of the first case portion 10. To this end, the inlet portion 22 including the inlet holes 24 is installed along a periphery of a lower portion of the first case portion 10. Also, the outlet 33 configured to discharge air may be disposed at the upper side of the first case portion 10.

The first case portion 10 according to the first embodiment of the present invention may include at least any one of a first case 20, a second case 30, and an intermediate case 40. The intermediate case 40 may be connected to an upper side of the first case 20, and the second case 30 may be connected to an upper side of the intermediate case 40.

Since the accommodation space 12 is formed to be continuous across the inside of the first case 20, the inside of the intermediate case 40, and the inside of the second case 30 in the vertical direction, which is the first direction, air that enters the first case 20 is guided to move upward along the intermediate case 40 and the second case 30.

### [First case]

The first case 20 includes the inlet portion 22 configured to suction air and may be modified in various ways in which the first case 20 is coupled to the upper side of the second case portion 50. The first case 20 according to the first embodiment of the present invention has a cylindrical shape and is continuous in the first direction.

Since the inlet portion 22 is formed along a periphery of the first case 20, air outside the first case 20 may move into the first case 20 through the inlet portion 22. The inlet portion 22 may be installed at a plurality of sites along the periphery of the first case 20. Since the inlet portion 22 according to the first embodiment of the present invention is continuously installed along the outer periphery of the first case portion 10, a suction flow rate of air may increase, and air cleaning efficiency may be improved.

The inlet portion 22 may be installed in a band-shaped region along the periphery of the first case 20, and the inlet portion 22 may include the plurality of inlet holes 24 configured to guide movement of air into the first case portion 10. The inlet holes 24 may be modified in various ways in which the inlet holes 24 guide a flow of air entering the first case 20 in a spiral shape. The inlet holes 24 according to the first embodiment of the present invention may be formed in the shape of a slot. Also, since the inlet holes 24 are installed to be inclined in one direction along the outer periphery of the first case portion 10, the inlet holes 24 guide the flow of air entering the first case 20 in a spiral shape.

In a case in which a virtual vertical line extends in the first direction, the inlet holes 24 are installed in a state of being tilted while forming a predetermined angle with the vertical line. Also, the inlet holes 24 are installed to be inclined in a direction in which a fan member 90 included in the fan module portion 70 rotates. Therefore, air that enters the first case 20 through the inlet holes 24 rotates clockwise and moves upward, and the fan member 90 included in the fan module portion 70 also rotates clockwise and suctions air that has passed through the filter portion 60. Thus, a flow of air that rotates in a spiral and moves upward may be facilitated, and air cleaning efficiency may be improved.

Also, since the inlet holes 24 are, instead of being installed in the first direction, formed in the shape of a slot that is tilted to form an acute angle with the virtual line extending in the first direction, a length of the inlet hole 24 is longer as compared to when the inlet hole 24 is installed in the first direction. Therefore, since an air flow is further facilitated when air enters through the slot-shaped inlet holes 24, air in a target space may be purified within a shorter time as compared to the related art.

In a case in which the fan member 90 included in the fan module portion 70 rotates clockwise, the inlet holes 24 are installed to be tilted clockwise. Also, in a case in which the fan member 90 included in the fan module portion 70 rotates counterclockwise, the inlet holes 24 are installed to be tilted counterclockwise.

Also, in a state in which the inlet portion 22 configured to suction air is installed along the outer periphery of the first case portion 10, since the filter portion 60 is installed at an upper side that is spaced apart from the inlet portion 22, air may move evenly throughout the entire area of the filter portion 60.

The first case 20 according to the first embodiment of the present invention may include the inlet portion 22 and a blocking body 26. The blocking body 26 is installed at an upper side of the inlet portion 22 configured to suction air. The blocking body 26 is formed in a cylindrical shape and guides the inlet portion 22 and the filter portion 60 to be spaced apart from each other at a predetermined gap.

The first case 20 that surrounds the outer periphery of the sanitizing portion 170 is formed in the shape of a pipe that is continuous in the first direction, and the blocking body 26 and the inlet portion 22 are consecutively installed in the vertical direction of the first case 20. An irradiating portion 180, which is a component of the sanitizing portion 170, may be disposed in the blocking body 26. Therefore, the sanitizing light irradiated by the sanitizing portion 170 comes in contact with the blocking body 26 and is blocked from being irradiated toward the outside of the first case portion 10. Since the sanitizing light is blocked from being irradiated toward the outside of the first case portion 10 through the inlet portion 22 of the first case portion 10, the sanitizing light may be prevented from being irradiated toward a user, and thus a safe usage environment may be provided.

Also, a length of the blocking body in the first direction is set in consideration of a movement space for allowing outside air, which enters the first case portion 10 through the inlet portion 22, to move upward and evenly pass through the filter portion 60. That is, the length of the blocking body in the first direction is set in consideration of a movement distance for allowing the outside air, which enters through the inlet portion 22, to rotate upward while rotating in a spiral and to evenly reach the entire area of the lower portion of the filter portion 60. Since the inlet portion 22 and the blocking body 26 constitute the first case 20, air that enters through the inlet portion 22 may be evenly supplied to the filter portion 60 disposed at an upper side, and thus air cleaning efficiency may be improved. Also, a service life of the filter portion 60 may be extended to reduce maintenance and repair costs.

The first case 20 according to the first embodiment of the present invention may further include an upper fixing portion 27 configured to fix the sanitizing portion 170. The first case 20 is disposed at the upper side of the second case portion 50, and a sanitization support portion 171 of the sanitizing portion 170, which will be described below, is installed between the first case 20 and the second case portion 50. Since the upper fixing portion 27, which extends to a lower side of the inlet portion 22, is disposed at an upper side of the sanitization support portion 171, the upper fixing portion 27 may restrict upward movement of the sanitizing portion 170 including the sanitization support portion 171. The sanitization support portion 171 according to the first embodiment of the present invention extends from a lower end of the inlet portion 22 toward an inner side of the inlet portion 22 in the radial direction and then extends downward in the first direction along an inner side surface of the sanitization support portion 171. Also, the upper fixing portion 27 may extend downward in the first direction along the inner side surface of the sanitization support portion 171 and then extend to the inner side of the inlet portion 22 in the radial direction to stably support the upper side of the sanitization support portion 171.

Also, the first case 20 according to the first embodiment of the present invention may further include a filter fixing protrusion 28 configured to fix the filter portion 60. The filter fixing protrusion 28 may protrude from an upper portion of the first case 20 toward an inner side of the first case 20 to support the lower portion of the filter portion 60. The filter fixing protrusion 28 may protrude to an inner side of the blocking body 26 and may be inserted into a groove portion disposed at the lower portion of the filter portion 60 to restrict downward movement of the filter portion 60.

### [Second case]

The second case 30 is disposed at the upper side of the first case 20 and may be modified in various ways in which the second case 30 includes the outlet 33 configured to discharge purified air and rotatably supports the discharge portion 140. A lower portion of the second case 30 may be formed in the shape of a cylindrical pipe, and an upper portion of the second case 30 may be formed in the shape of an expanding type pipe whose outlet gradually widens. The second case 30 according to the first embodiment of the present invention may include at least any one of a second case body 31, a tubular expansion member 32, and the rotation support portion 35.

The second case body 31 may be installed in a shape that surrounds the outer periphery of the fan module portion 70. The second case body 31 is formed in the shape of a cylindrical pipe extending in the first direction, and the accommodation space 12 formed in the second case body 31 is continuous in the first direction. The fan module portion 70 is disposed in the second case body 31.

The tubular expansion member 32 is formed in the shape of a pipe which extends to an upper side of the second case body 31 and in which an internal path gradually widens. The shape of the tubular expansion member 32 is designed in consideration of an angle of rotation of the discharge portion 140, a size of the discharge portion 140, and whether the tubular expansion member 32 interferes with the discharge portion 140. The tubular expansion member 32 may be integrally formed with the second case body 31, or as necessary, the tubular expansion member 32 and the second case body 31 may be manufactured as separate members and then coupled to each other. Also, the outlet 33, which is a path through which air is discharged, is disposed at an inner side of the tubular expansion member 32. Also, the discharge portion 140 is rotatably installed at an inner side of the outlet 33.

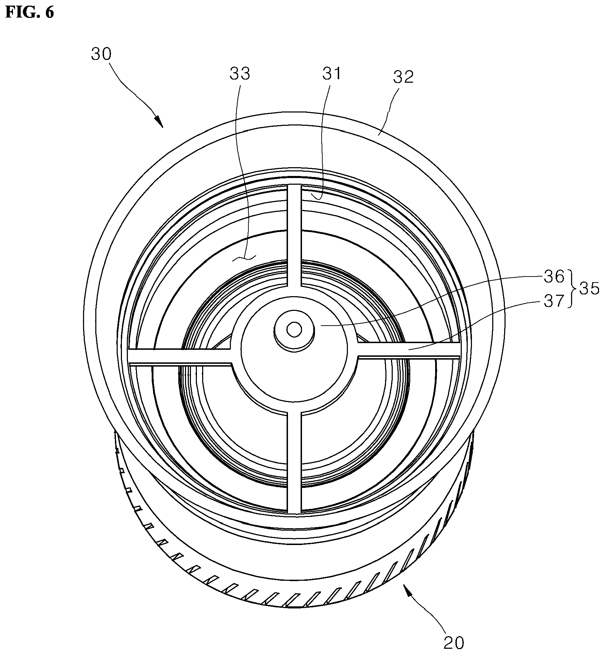

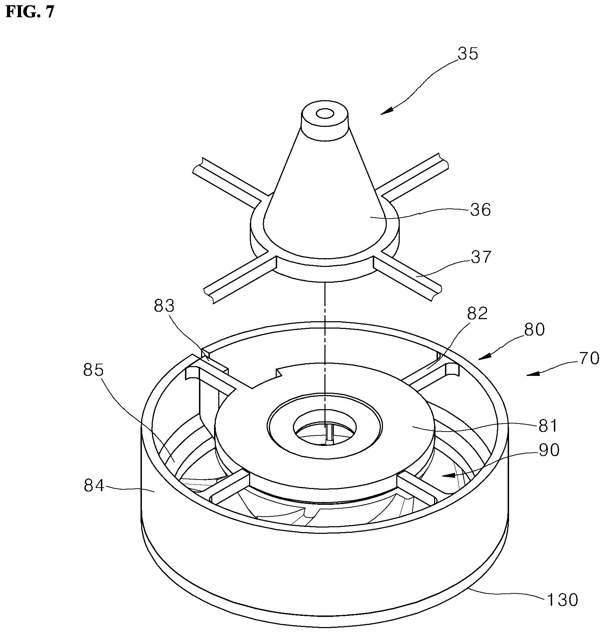

FIG. 6 is a perspective view illustrating the rotation support portion 35 according to the first embodiment of the present invention, and FIG. 7 is a perspective view illustrating a state in which the rotation support portion 35 is installed at an upper side of the fan module portion 70 according to the first embodiment of the present invention.

As illustrated in FIGS. 2, 6, and 7, the rotation support portion 35 is connected to at least any one of the second case body 31 and the tubular expansion member 32 and may be modified in various ways in which the rotation support portion 35 rotatably supports the discharge portion 140. Also, the rotation support portion 35 may rotatably support the discharge portion 140 at the center of the outlet 33 disposed at the inner side of the tubular expansion member 32. The rotation support portion 35 according to the first embodiment of the present invention may include at least any one of a core member 36, a support 37, and a ball joint 38.

The core member 36 is disposed at a lower side of the discharge portion 140 configured to control a discharge direction of air and may extend from the center of the outlet 33 in a direction toward the discharge portion 140. The core member 36 is disposed at the center of the second case 30 in the radial direction, and a lower side of the core member 36 is formed in a disk shape.

A transverse cross-section of a lower portion of the core member 36 is formed in a circular shape and coincides with a central portion of the fan module portion 70 which will be described below. That is, a support plate 81, which is disposed at the center of the fan module portion 70 in the radial direction, is disposed at the lower portion of the core member 36. Therefore, air that moves upward through the outer periphery of the support plate 81 moves upward along an outer side of the core member 36 and moves into the discharge portion 140. Thus, resistance of an air flow path is decreased, and air cleaning efficiency is improved.

Since a lower surface of the core member 36 that faces the support plate 81 of the fan module portion 70 has an area that is less than or equal to an area of the support plate 81, an increase in resistance of a flow path due to air, which has passed through the fan module portion 70, coming in contact with the lower portion of the core member 36 may be prevented. A cross-sectional area of a lower end portion of the core member 36 is less than or equal to a cross-sectional area of the support plate 81, and the core member 36 is disposed at an upper side of the support plate 81 in the first direction.

Also, since the core member 36 is formed in a conical shape toward an upper side, an area of the core member 36 that comes in contact with air moving outside the core member 36 is minimized. Thus, friction with air may be reduced, and air cleaning efficiency may be improved.

Also, a mounting hole portion to which the ball joint 38, which is configured to rotatably support the discharge portion 140, is connected is disposed at an upper end of the core member 36.

The support 37 extends to an outer side of the core member 36 and may be modified in various ways in which the support 37 is connected to at least any one of the second case body 31 and the tubular expansion member 32 to restrict movement of the core member 36. The support 37 according to the first embodiment of the present invention may be provided as a plurality of supports 37 and may be installed in the shape of a rod. The supports 37 extending radially outward from the lower portion of the core member 36 may be connected to the second case body 31. Alternatively, the supports 37 may be connected to the tubular expansion member 32 or connected to a connection site between the second case body 31 and the tubular expansion member 32.

The support 37 according to the first embodiment of the present invention is disposed at an upper side of a connection support 82 of the fan module portion 70 which will be described below. For example, in a case in which, as the connection support 82, four connection supports 82 are installed at 90° intervals about the support plate 81, the support 37 is also provided as four supports 37 installed at 90° intervals about the core member 36. The connection support 82 is disposed at a lower side of the support 37, and when viewed from the upper side of the support 37, the support 37 and the connection support 82 are installed to overlap each other.

Therefore, air, which passes through an outer side of the connection support 82 and moves upward, passes through an outer side of the support 37 disposed at the upper side of the connection support 82. Thus, friction that occurs during movement of air may be minimized, and air cleaning efficiency may be improved.

The support 37 according to the first embodiment of the present invention may also be provided as four or more supports 37 radially installed about the core member 36. Also, in a state in which the support 37 is connected to a separate ring-shaped edge member, the edge member may be fixed to the housing portion 3300. In this way, various modifications are possible.

The ball joint 38 is coupled to the core member 36 and may be modified in various ways in which the ball joint 38 rotatably supports the discharge portion 140. The ball joint 38 according to the first embodiment of the present invention has a spherical end portion, and the end portion may be coupled to an inner side of the discharge portion 140 to rotatably support the discharge portion 140. An upper side of the ball joint 38 has a spherical shape, and a bar-shaped body of the ball joint 38 that extends downward from the spherical upper side is inserted into and fixed to the core member 36 through a hole formed in an upper side of the core member 36. The ball joint 38 and the core member 36 may be fixed using various fixing methods such as screw fastening, pin fastening, and using an adhesive.

As illustrated in FIGS. 2 and 3, the first case portion 10 may further include the intermediate case 40 which is installed between the first case 20 and the second case 30. The intermediate case 40 is installed in a shape surrounding an outer side of the filter portion 60 and may be modified in various ways in which the intermediate case 40 connects the first case 20 and the second case 30 to each other. The intermediate case 40 according to the first embodiment of the present invention may be formed in the shape of a cylindrical pipe, and the upper and lower sides of the intermediate case 40 may be open.

Meanwhile, a case protrusion 42 that protrudes to an inner side of the intermediate case 40 may support the lower portion of the fan module portion 70 and may prevent the fan module portion 70 from coming in contact with the filter portion 60, which is disposed below the fan module portion 70. Since the case protrusion 42 maintains a separation distance between the filter portion 60 and the fan module portion 70, air that has passed through the filter portion 60 is collected at an inlet of the fan module portion 70, and thus a flow of air moving into the fan module portion 70 may be further facilitated.

Meanwhile, the inlet holes 24 are formed in the inlet portion 22, and the inlet holes 24 may be installed to be inclined in a diagonal shape or, as necessary, may be formed as holes each having the shape of an inequality sign in which the line is broken at the center. Also, in order to increase a flow rate of air entering the filter portion 60, the inlet holes 24 may be additionally formed in a side surface of the housing portion 3300 in which the filter portion 60 is installed. In this way, various modifications are possible.

Meanwhile, the housing portion 3300 may consist of three or more members. In this way, the housing portion 3300 may be modified to have various other shapes.

### [Second case portion]

The second case portion 50 is connected to the lower portion of the first case portion 10 and may be modified in various ways in which the second case portion 50 has a space formed therein to install electronic components including the battery 200.

At least any one of the first case portion 10 and the second case portion 50 may be made of a cylindrical case. Both the first case portion 10 and the second case portion 50 may be formed in a cylindrical shape, or only the second case portion 50 may be formed in a cylindrical shape. Alternatively, as necessary, only the first case portion 10 may be formed in a cylindrical shape.

In a case in which the second case portion 50 is formed in a cylindrical shape and extends in the vertical direction, it is convenient for a user to hold the outer periphery of the second case portion 50 with his or her hand, and the second case portion 50 may also be easily mounted on a cup holder of a vehicle that includes a groove portion having a substantially circular cross-section.

Also, in a case in which the first case portion 10 is formed in a cylindrical shape, friction, which is generated when air passing through the first case portion 10 and moving upward comes in contact with the inside of the first case portion 10 formed in a curved shape, may be reduced such that a flow of air is facilitated.

Meanwhile, since an air flow path is formed inside the first case portion 10 while an air flow path is not formed inside the second case portion 50, suction and discharge of air through the first case portion 10 may be smoothly performed even when the second case portion 50 is inserted into a cup holder or held by a user's hand. Thus, convenience in use may be improved.

The second case portion 50 according to the first embodiment of the present invention includes a lower case 52 and a lower pedestal portion 54. The lower case 52 is formed in a cylindrical shape, and electronic components including the battery 200 are installed in the lower case 52. An upper side of the lower case 52 is open, and a lower side of the lower case 52 may be blocked by a separate cover.

Also, the lower pedestal portion 54 which protrudes to an inner side of the lower case 52 may support a lower portion of the irradiating portion 180 and thus restrict downward movement of the irradiating portion 180.

### [Filter portion]

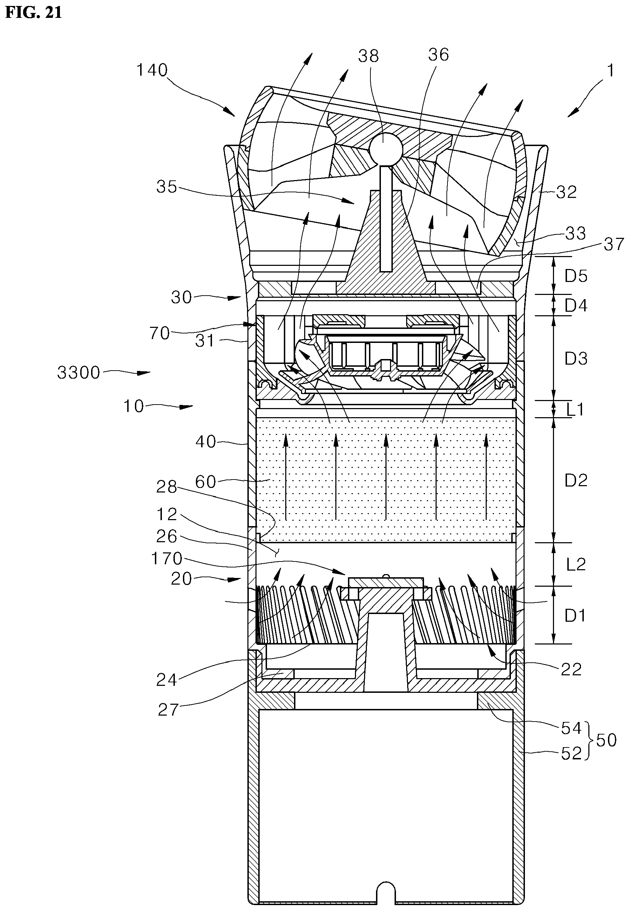

FIG. 21 is a cross-sectional view in which the portable air caring apparatus 1 according to the first embodiment of the present invention is divided in a vertical longitudinal direction.

As illustrated in FIG. 21, the filter portion 60 is installed in the first case portion 10 and may be modified in various ways in which the filter portion 60 purifies air that enters through the inlet portion 22. The filter portion 60 according to the first embodiment of the present invention may be formed in a cylindrical shape. The filter portion 60 may be installed in the intermediate case 40, and the filter portion 60 and the intermediate case 40 may constitute a module to facilitate disassembly and assembly thereof.

Since the intermediate case 40 is formed in the shape of a circular pipe and the filter portion 60, which is installed in the intermediate case 40, is also formed in a cylindrical shape that comes in contact with the inside of the intermediate case 40, impurities may be effectively removed from air passing through the intermediate case 40.

When a height of the filter portion 60 is D2 and a height of the inlet portion 22 is D1, the height D2 of the filter portion 60 may be set to be higher than the height D1 of the inlet portion 22. Since an air caring function is an important function of the portable air caring apparatus 1, a component that increases an air caring volume of the filter portion 60 is important. Accordingly, since D2 is formed to be longer than D1 in the portable air caring apparatus 1 according to the present invention, efficiency with which foreign substances are collected to the filter portion 60 from air that enters the first case portion 10 through the inlet portion 22 may be improved.

Also, a transverse cross-section of the filter portion 60 is formed in a circular shape such that the filter portion 60 has the largest area in the first case portion 10. Also, when the filter portion 60 is manufactured in the form of a cylinder and an upper end and a lower end of a material constituting the filter portion 60 are cut, pressure loss may be minimized, and thus performance of the filter portion 60 may be maximized.

Also, since an outer diameter of the filter portion 60 is formed to be greater than or equal to a suction diameter of a bell mouth 132 through which air is suctioned into the fan module portion 70, the volume of the filter portion 60 may be maximized.

Meanwhile, when a distance between the fan module portion and the filter portion 60 is L1 and a distance between the filter portion 60 and the irradiating portion 180 is L2, L1 may be shorter than L2. Since the distance L1 between the fan module portion and the filter portion 60 is greater than or equal to 1/8^{th} of a suction inner diameter of the fan module portion 70, the filter portion 60 may be designed so that suction flow resistance of air being suctioned into the fan module portion 70 is not increased.

Also, according to the present embodiment, the filter portion 60, the fan module portion 70, and the discharge portion 140 may be arranged in the vertical direction along the housing portion 3300, and an air flow may also occur in the vertical direction. That is, an air flow that occurs due to the operation of the fan module portion 70 may occur in a linear direction which is the same as the direction in which the filter portion 60, the fan module portion 70, and the discharge portion 140 are arranged.

When the air flow occurs in the linear direction as described above, resistance related to the air flow is lowered correspondingly, and thus the air flow may occur more smoothly. In this way, since suctioning a sufficient amount of air and discharging a sufficient amount of air, which corresponds to the amount of suctioned air, may be performed by the fan module portion 70, air caring performance of the portable air caring apparatus 1 may be improved correspondingly.

### [Fan module portion]

The fan module portion 70 is disposed between the filter portion 60 and the outlet 33 and may be modified in various ways in which the fan module portion 70 rotates a fan to blow air in a direction toward the outlet 33.

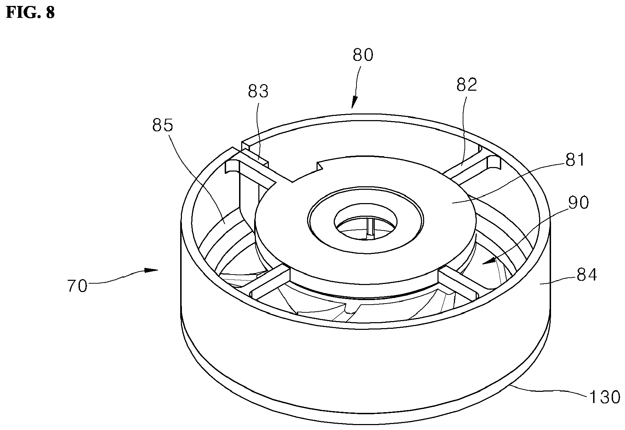

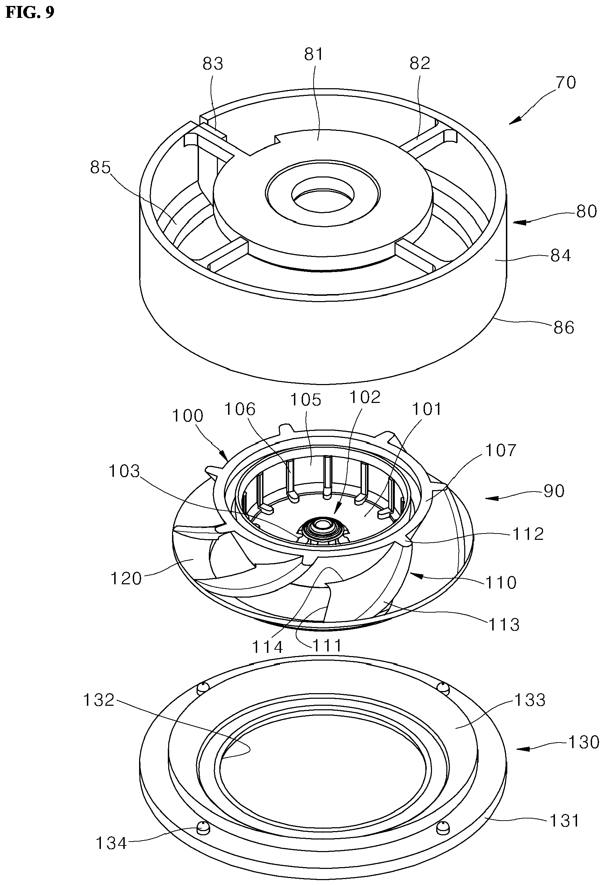

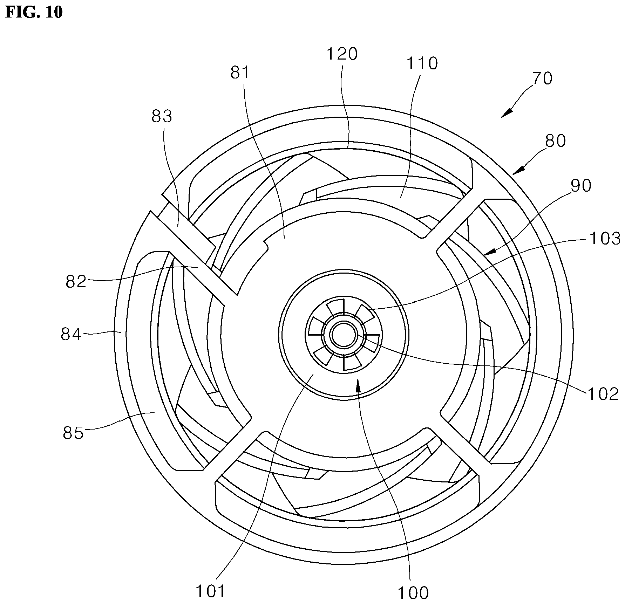

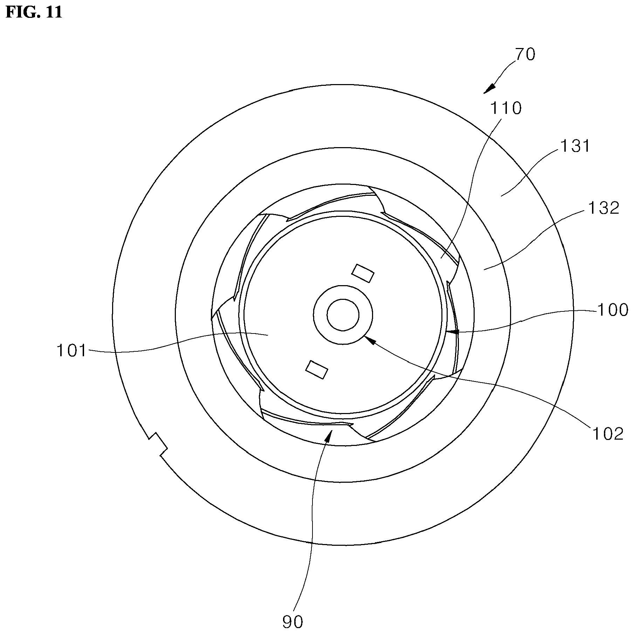

FIG. 8 is a perspective view illustrating the fan module portion 70 according to the first embodiment of the present invention, FIG. 9 is an exploded perspective view of the fan module portion 70 according to the first embodiment of the present invention, FIG. 10 is a plan view of the fan module portion 70 according to the first embodiment of the present invention, and FIG. 11 is a bottom view of the fan module portion 70 according to the first embodiment of the present invention.

As illustrated in FIGS. 2 and 8 to 11, when the fan module portion 70, which is a circular diagonal flow fan module, is applied, since the shape of the fan module portion 70 matches with the inner shape of the first case portion 10, which is a cylindrical shape, or corresponds thereto, the size of the first case portion 10 does not need to be enlarged due to fixing or fastening the fan module portion 70. In this way, reduction of product size is possible. Also, for use in a vehicle, the portable air caring apparatus 1 according to the present invention may be implemented to have a size that allows the portable air caring apparatus 1 to be inserted into a cup holder.

Also, since a circular diagonal flow fan module is applied as the fan module portion 70, a small-sized upward discharge type air cleaner that can maximize air flow performance may be provided. A type of fan of the fan module portion 70 is a diagonal flow fan, and an internal structure of the fan module portion 70 is changed to mount the diagonal flow fan.

The fan member 90 according to the present invention rotates due to operating a motor. Only a rotating shaft of the motor that rotates the fan member 90 may be connected to the fan member 90, a rotor may be installed at the fan member 90, and a stator may be installed in a fan housing 80 whose rotation is restricted. Since a magnetic field of the stator changes, the shaft that rotates along with the rotor may be connected to the fan member 90, and the rotor and the fan member 90 may rotate about the stator. Since the configuration of the motor rotating the fan member 90 is a known configuration, detailed description thereof will be omitted.

The fan module portion 70 according to the first embodiment of the present invention may include the fan housing 80, the fan member 90, and a fan base 130.

### [Fan housing]

The fan housing 80 is fixed to an inner side of the first case portion 10 and may be modified in various ways in which the fan housing 80 has a space formed therein to allow the fan member 90 to rotate. The fan housing 80 according to the first embodiment of the present invention may include at least any one of the support plate 81, the connection support 82, a wire guide 83, a side support portion 84, an inner side guiding portion 85, and a protruding boss 86.

The support plate 81 is formed in a disk shape, and a hole is formed at the center of the support plate 81. A motor may be installed at the center of the support plate 81, or a shaft connected to the motor may be installed in the first direction. The support plate 81 is disposed on the lower side of the core member 36.

The connection support 82 extends to the outside of the support plate 81 and is connected to the side support portion 84. The connection support 82 according to the first embodiment of the present invention may be provided as a plurality of connection supports 82 and may be installed in the shape of a rod. The connection supports 82 extending radially outward from the support plate 81 may be connected to the inner side guiding portion 85.

The connection support 82 according to the first embodiment of the present invention is disposed at a lower side of the support 37 of the rotation support portion 35. As the connection support 82, four connection supports 82 are installed at 90° intervals about the support plate 81, and the supports 37 are installed at upper sides of the connection supports 82 that face the supports 37.

Therefore, since the supports 37 of the rotation support portion 35 and the connection supports 82 of the fan module portion 70 are installed at positions that face and overlap each other, discharge flow resistance of air passing through outer sides of the connection supports 82 and the supports 37 and moving upward is decreased. Thus, an air flow may occur smoothly.

That is, the fan module portion 70 is disposed at a lower side of the rotation support portion 35, and the fan housing 80 forming an outer shape of the fan module portion 70 is fixed to the first case portion 10 and guides movement of air that moves upward. The fan housing 80 may include the support plate 81 disposed at the lower side of the core member 36 and the connection support 82 extending radially outward from the support plate 81.

Air moving upward through the fan member 90 moves upward through a space formed by the support plate 81, the connection support 82, and the side support portion 84. Here, since the connection support 82 and the support 37 overlap each other in the vertical direction, resistance of an air flow path along which air moves upward through the fan module portion 70 and the rotation support portion 35 may be reduced. That is, since the connection support 82 and the support 37 are installed to overlap each other, contact between the support 37 and air moving upward around the connection support 82 may be reduced, and frictional resistance of air may be reduced.

The wire guide 83 is continuously installed on the connection support 82 and supports a lower portion of a wire of an electronic device so that the wire may move along a side surface of the connection support 82. The wire guide 83 is formed in the shape of a protrusion, which is disposed on a lower portion of the side surface of the connection support 82, and guides a wire of the motor installed on the support plate 81 to be installed to extend to the outside of the fan housing 80. The wire guide 83 may be installed at the side surface of the connection support 82 and form a concave groove to allow the wire to be disposed therein. Therefore, since the wire installed in the wire guide 83 is disposed in the concave groove disposed at the side surface of the connection support 82, and the lower portion of the wire is supported by the wire guide 83, damage to the wire may be prevented.

The side support portion 84 is formed in the shape of a cylindrical pipe, and the upper and lower sides of the side support portion 84 are open. An outer side of the side support portion 84 comes in contact with the inside of the second case 30 and the intermediate case 40, and an inner side of the side support portion 84 is connected to the connection support 82.

The inner side guiding portion 85 forms an inclined surface that is installed to be inclined downward toward a radially inward side from a lower side of the side support portion 84. The inner side guiding portion 85 may be formed at the inner side of the side support portion 84 and may prevent a return air phenomenon in which air, which is blown upward by the fan member 90, moves to an inlet of the fan member 90 through an outer side surface of the fan member 90.

The protruding boss 86 extends to a lower end of the side support portion 84 and may be modified in various ways in which the protruding boss 86 includes a groove portion for inserting a coupling protrusion 134 of the fan base 130 which will be described below. The protruding boss 86 according to the first embodiment of the present invention may be provided as a plurality of protruding bosses 86 installed in a circumferential direction of the side support portion 84.

### [Fan member]

The fan member 90 is rotatably installed in the fan housing 80 and may be modified in various ways in which the fan member 90 is able to move air in the direction toward the discharge portion 140.

A diagonal flow fan may be used as the fan member 90, but this is only the first embodiment of the present invention, and other types of fans may also be used as the fan member 90 according to the present invention. The fan member 90 according to the first embodiment of the present invention may include at least any one of a hub 100, a fan blade 110, and a shroud 120.

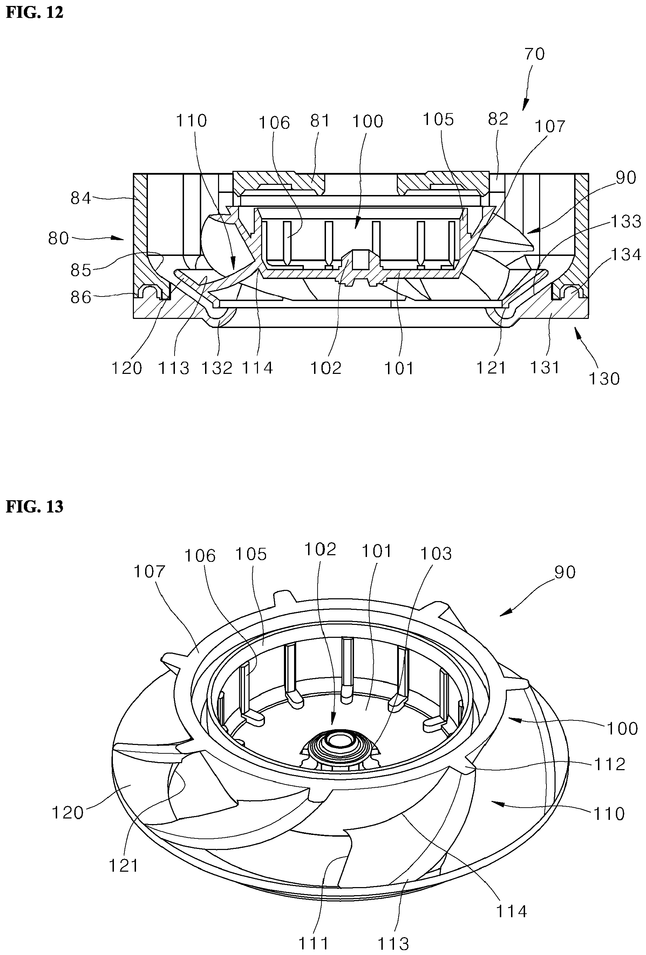

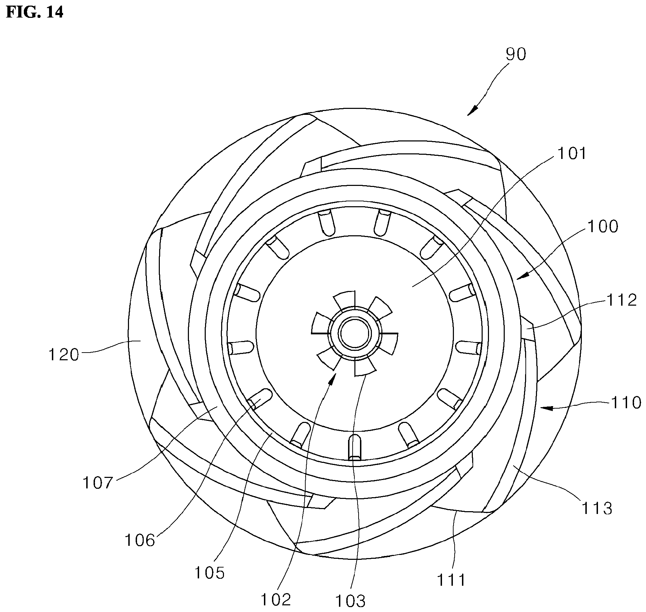

FIG. 12 is a cross-sectional view of the fan module portion 70 according to the first embodiment of the present invention, FIG. 13 is a perspective view of the fan member 90 according to the first embodiment of the present invention, and FIG. 14 is a plan view of the fan member 90 according to the first embodiment of the present invention.

As illustrated in FIGS. 12 to 14, the hub 100 is disposed at the center of the fan housing 80 and may be modified in various ways in which the hub 100 receives external power and rotates.

The hub 100 is disposed at the center of the fan member 90 in the radial direction and may rotate along with the rotor and the shaft, which is an output shaft of the motor, that constitute the motor. The hub 100 according to the first embodiment of the present invention may include at least any one of a hub plate portion 101, an axial coupling portion 102, an inner side protruding portion 105, and a skirt portion 107.

The hub plate portion 101 may be formed in the shape of a disk that is parallel to the support plate 81. The axial coupling portion 102 may be provided on the hub plate portion 101. The axial coupling portion 102 may be disposed at the center of the hub plate portion 101 in the radial direction. The axial coupling portion 102 may be formed to protrude to an upper side and a lower side of the hub plate portion 101.

The axial coupling portion 102 may be coupled to an axial end portion of a shaft that transmits rotary power. For example, coupling between the axial coupling portion 102 and the shaft may be performed in a form in which the shaft is fitted to the axial coupling portion 102.

First reinforcing protrusions 103 are installed at predetermined intervals along the outer periphery of the axial coupling portion 102. The first reinforcing protrusions 103 are radially installed about the center of the axial coupling portion 102 and are formed as band-shaped protrusions at an outer side of the axial coupling portion 102. Therefore, since stress concentrated on the axial coupling portion 102 is distributed through the first reinforcing protrusions 103, structural rigidity of the axial coupling portion 102 may be improved.

The inner side protruding portion 105 may protrude in a direction from the hub plate portion 101 toward an upper portion on which the support plate 81 is installed. The inner side protruding portion 105 according to the first embodiment of the present invention is installed in an arc shape along the outer side edge of the hub plate portion 101. The inner side protruding portion 105 is formed in the shape of a pipe that extends in the vertical direction.

Also, second reinforcing protrusions 106 are installed at predetermined intervals along the inner periphery of the inner side protruding portion 105. The second reinforcing protrusions 106 are installed in the first direction along an inner side surface of the inner side protruding portion 105, and lower sides of the second reinforcing protrusions 106 are formed as band-shaped protrusions that extend toward the axial coupling portion 102. Therefore, since stress concentrated on the inner side protruding portion 105 is distributed through the second reinforcing protrusions 106, structural rigidity of the inner side protruding portion 105 may be improved. As necessary, the rotor of the motor may be fixed to the inner side of the inner side protruding portion 105.

The skirt portion 107 may protrude in a direction from an edge of the hub plate portion 101 toward the support plate 81. The skirt portion 107 may form an inclined surface that is further inclined outward in the second direction away from the hub plate portion 101 in the first direction. The skirt portion 107 is disposed at an outer side of the inner side protruding portion 105, and an inner diameter of the skirt portion 107 gradually becomes larger from a lower side toward an upper side.

For example, the hub plate portion 101 and the skirt portion 107 may be connected in the shape of a truncated cone in which a hollow is formed and whose one side is open. The skirt portion 107 may be formed in the shape of a funnel in which an upper side is open and a lower side is blocked by the hub plate portion 101.

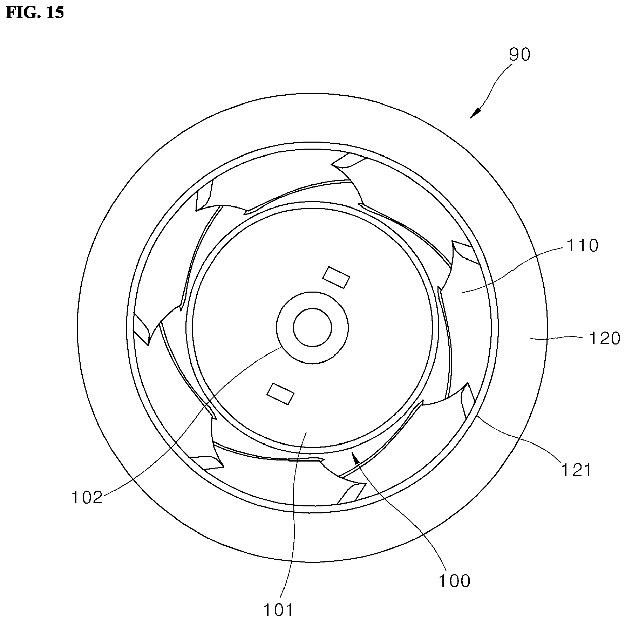

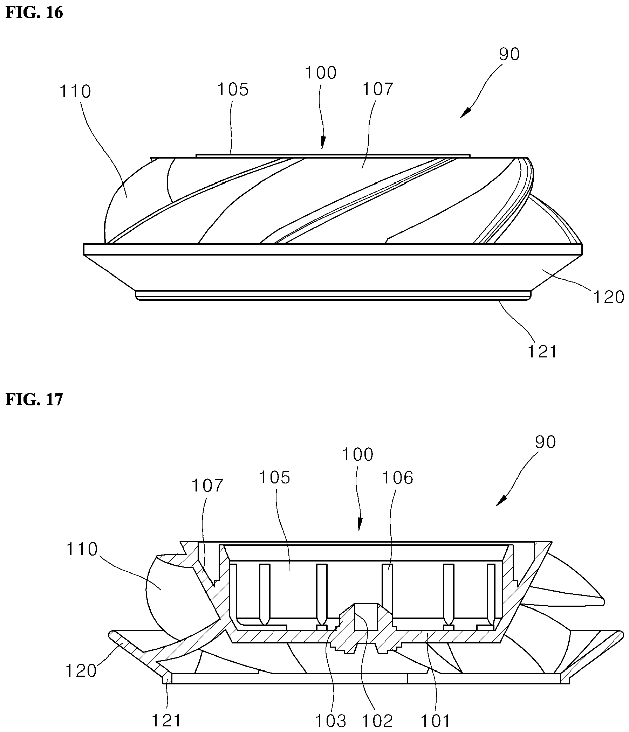

FIG. 15 is a bottom view of the fan member 90 according to the first embodiment of the present invention, FIG. 16 is a front view of the fan member 90 according to the first embodiment of the present invention, and FIG. 17 is a cross-sectional view of the fan member 90 according to the first embodiment of the present invention.

As illustrated in FIGS. 15 to 17, the shroud 120 is connected to an end portion of the fan blade 110 and installed in an annular shape and may be modified in various ways in which the shroud 120 may be spaced apart from the fan base 130.

The shroud 120 is installed along the outer periphery of the skirt portion 107, and the shroud 120 and the skirt portion 107 are connected to each other by the fan blade 110. Also, an outer diameter of the hub 100 and an inner diameter of the shroud 120 may gradually decrease in a direction from an upper side toward a lower side.

The shroud 120 may be spaced a predetermined distance apart from the hub 100 in the radial direction and may be disposed at an outer side of the hub 100 in the radial direction. Also, the shroud 120 may be spaced apart from the hub 100 by as much as a distance that corresponds to a length of the fan blade 110 in the radial direction. Also, each fan blade 110 may connect the skirt portion 107, which is disposed at the hub 100, and the shroud 120 to each other.

The shroud 120 may form an inclined surface that is substantially parallel to the skirt portion 107. In the present embodiment, the skirt portion 107 and the shroud 120 are illustrated as being arranged in a form in which a distance between the skirt portion 107 and the shroud 120 gradually increases in a direction toward an upper side of the shroud 120.

An inlet protrusion 121 disposed at a lower side of the shroud 120 is a ring-shaped protrusion and extends in the first direction from the lower side of the funnel-shaped shroud 120. Since the inlet protrusion 121 is disposed at an inner side of the bell mouth 132 which will be described below, the inlet protrusion 121 may prevent the returning flow of air, which enters through an inlet provided in the lower side of the shroud 120, along an outer side of the shroud 120.

The fan blade 110 may be provided as a plurality of fan blades 110, and the plurality of fan blades 110 may be disposed to be spaced apart from each other at equal intervals along an outer peripheral surface of the hub 100. The fan blades 110 protrude to the outside of the hub 100 with respect to the center of the hub 100 and extend in a spiral shape. Also, the plurality of fan blades 110 may be disposed to be spaced apart from each other at predetermined intervals in a peripheral direction of the hub 100.

The fan blades 110 according to the first embodiment of the present invention may protrude to the outside of the skirt portion 107 in a centrifugal direction extending in a spiral shape from the center of the axial coupling portion 102. Also, when a direction from the outside of the axial coupling portion 102 toward the axial coupling portion 102 is the radial direction, the inner side of the fan blades 110 in the radial direction may be connected to the skirt portion 107, and the outer side of the fan blades 110 in the radial direction may be connected to the shroud 120 which will be described below.

The skirt portion 107 is a portion of the hub 100 that is directly connected to the fan blades 110 and is a portion that also comes in direct contact with air passing through the fan blades 110. The skirt portion 107 is also closely related to a flow path of air passing through the fan module portion 70.

As illustrated in FIGS. 13 and 14, each fan blade 110 that connects the shroud 120 and the skirt portion 107 to each other may include a first end portion 111, a second end portion 112, a first edge 113, and a second edge 114.

The first end portion 111 is disposed at a front end of the fan blade 110 in a rotational direction thereof and may be formed in a linear shape that extends in the radial direction. The rotational direction is defined as a direction in which rotation of the fan member 90 occurs.

The second end portion 112 is disposed at a rear end of the fan blade 110 in the rotational direction thereof and may be radially formed about the axial coupling portion 102.

The first edge 113 may connect one end of the first end portion 111 and one end of the second end portion 112. The first edge 113 may be connected to an inner peripheral surface of the shroud 120.

The second edge 114 may connect the other end of the first end portion 111 and the other end of the second end portion 112. The second edge 114 may be connected to the outer peripheral surface of the hub 100.

That is, the one end of the first end portion 111 and the one end of the second end portion 112 may be connected to the inner peripheral surface of the shroud 120. Also, the other end of the first end portion 111 and the other end of the second end portion 112 may be connected to an outer peripheral surface of the skirt portion 107.

The one end of the first end portion 111 may be disposed closer to the center of the hub plate portion 101 in the radial direction than the one end of the second end portion 112. Also, the other end of the second end portion 112 may be disposed closer to the center of the hub plate portion 101 in the radial direction than the other end of the first end portion 111. This is because the one end and the other end of the first end portion 111 are disposed more toward the front in the rotational direction than the one end and the other end of the second end portion 112, and the skirt portion 107 is formed such that a radius thereof gradually becomes smaller toward the front in the rotational direction.

According to the present embodiment, the fan blade 110 is connected to the skirt portion 107 of the hub 100. In order to guide a flow of air entering the fan module portion 70 in a direction that is inclined upward, the skirt portion 107 forms an inclined surface that is inclined upward.

### [Fan base]

As illustrated in FIGS. 9 to 12, the fan base 130 is coupled to a lower side of the fan housing 80 and may be modified in various ways in which the fan base 130 guides air, which has passed through the filter portion 60, to enter the fan member 90.

The fan base 130 may be disposed between the filter portion 60 and the fan member 90. Also, an edge of the fan base 130 may be formed in a shape that corresponds to the shape of an edge of the filter portion 60. For example, when the filter portion 60 is formed in a cylindrical shape and the edge of the filter portion 60 is formed in a circular shape, the fan base 130 may be installed in an annular shape having a hollow formed therein.

A base plate 131 may be disposed between the filter portion 60 and the fan member 90. The base plate 131 is formed in the shape of a plate that extends in an annular shape and has a hollow formed at the center to allow movement of air.

The bell mouth 132 is installed in an annular shape at an inner side of the base plate 131 that faces the hollow. The bell mouth 132 extends in the circumferential direction and has a longitudinal cross-section formed in a concave shape that surrounds a lower side of the inlet protrusion 121 of the shroud 120.

The bell mouth 132 may be formed in a shape that surrounds an outer peripheral surface of the hollow formed at the center of the base plate 131. The bell mouth 132 is formed to be convex toward the lower side and may form a groove portion that is concave toward the upper side.

At least one portion of the bell mouth 132 may be inserted into the shroud 120 in the radial direction. The bell mouth 132 may guide a suctioning flow at the inlet of the fan module portion 70 to contribute to an improvement in suctioning and discharging performance of the fan module portion 70.

The coupling protrusion 134 protrudes to an upper side of the base plate 131 and is coupled to the groove portion of the protruding boss 86, which is disposed in the fan housing 80, by being fitted thereto to fix the fan base 130 to the lower side of the fan housing 80.

The fan base 130 and the fan housing 80 may be coupled to each other at a plurality of points due to coupling performed between the coupling protrusion 134 and the protruding boss 86. When coupling between the fan base 130 and the fan housing 80 is performed as described above, the fan member 90 may be rotatably installed between the fan base 130 and the fan housing 80.

A protruding rib 133 protrudes from the base plate 131 and may be disposed at an outer side of the bell mouth 132 in the radial direction. The protruding rib 133 according to the first embodiment of the present invention is located at the outer side of the bell mouth 132 in the radial direction and is installed in an annular shape that surrounds the outer periphery of the bell mouth 132. Also, the protruding rib 133 may be integrally formed with the base plate 131. More specifically, the base plate 131, the bell mouth 132, and the protruding rib 133 may be integrally formed.

Also, the protruding rib 133 may be installed to be inclined at the same angle as an outer side surface of the shroud 120, and a distance between the protruding rib 133 and the shroud 120 may be maintained to be constant. The protruding rib 133 may protrude in a shape forming an inclined surface. The inclined surface of the protruding rib 133 may be formed as an inclined surface that is spaced a predetermined distance apart from the shroud 120 and is parallel to the inclined surface of the shroud 120.

Also, the inclined surface of the protruding rib 133 may have the same angle of inclination as an inclined surface of the inner side guiding portion 85 disposed in the fan housing 80. Therefore, it is possible to prevent a return air phenomenon in which a portion of air, which has moved upward through a space between the shroud 120 and the skirt portion 107, moves to the inlet of the fan member 90 through a space between the shroud 120 and the protruding rib 133.

### [Discharge portion]

As illustrated in FIGS. 2 to 4, the discharge portion 140 is rotatably installed in the first case portion 10 and may be modified in various ways in which the discharge portion 140 controls a discharge direction of air that has passed through the fan module portion 70. The discharge portion 140 according to the present invention may smoothly rotate due to being rotatably installed on the spherical ball joint 38 disposed in the first case portion 10.

Since the discharge portion 140 installed at an upper side of the first case portion 10 is open in the vertical direction and rotatably connected to the upper side of the first case portion 10, the discharge portion 140 may control the discharge direction of air which has passed through the fan module portion 70. The discharge portion 140 according to the first embodiment of the present invention may include a first discharge portion 150 and a second discharge portion 160.

The first discharge portion 150 is disposed at one side (the upper side in FIG. 3) of the ball joint 38 and may be modified in various ways in which the first discharge portion 150 includes a plurality of vanes 156 configured to guide discharge of air. The first discharge portion 150 according to the first embodiment of the present invention may include a first discharge core 152, a first discharge body 154, and the vanes 156.

The first discharge core 152 is installed in a shape that surrounds the spherical upper side of the ball joint 38 and is disposed at the upper side of the core member 36. Also, the first discharge body 154 is installed in an annular shape that surrounds an outer side of the first discharge core 152, and an outer side of the first discharge body 154 is formed in a curved shape.

Also, since the first discharge core 152 and the first discharge body 154 are connected to each other by the plurality of vanes 156, the first discharge core 152, the first discharge body 154, and the vanes 156 may rotate together.

The second discharge portion 160 is disposed at the other side (the lower side in FIG. 3) of the ball joint 38 and may be modified in various ways in which the second discharge portion 160 is connected to the first discharge portion 150 and rotates about the ball joint 38 along with the first discharge portion 150. The second discharge portion 160 according to the first embodiment of the present invention may include a second discharge core 161, a second discharge body 162, and discharge supports 163.

The second discharge core 161 is installed in a shape that surrounds the spherical lower side of the ball joint 38 and is disposed at a lower side of the first discharge core 152. Also, the second discharge body 162 is installed in an annular shape that surrounds an outer side of the second discharge core 161, and an outer side of the second discharge body 162 is formed in a curved shape.

Also, since the second discharge core 161 and the second discharge body 162 are connected to each other by the plurality of discharge supports 163, the second discharge core 161, the second discharge body 162, and the discharge supports 163 may rotate together.

### [Sanitizing portion]

The sanitizing portion 170 is disposed between the filter portion 60 and the second case portion 50 and may be modified in various ways in which the sanitizing portion 170 irradiates the sanitizing light toward the filter portion 60. The sanitizing portion 170 according to the first embodiment of the present invention may include at least any one of the sanitization support portion 171, a pedestal portion 176, and the irradiating portion 180.

### [Sanitization support portion]

The sanitization support portion 171 is disposed between the first case portion 10 and the second case portion 50 and blocks the lower portion of the first case portion 10. Also, the sanitization support portion 171 is disposed at a lower side of the irradiating portion 180 and may be modified in various ways in which the sanitization support portion 171 is connected to the housing portion 3300 such that movement of the sanitization support portion 171 is restricted. The sanitization support portion 171 according to the first embodiment of the present invention may include a support base 172 and a fixing edge 173. The support base 172 has a disk shape, an upper side of the support base 172 is fixed by the upper fixing portion 27, and a lower side of the support base 172 is supported by the lower pedestal portion 54 of the second case portion 50.

The fixing edge 173 protrudes upward from an edge of the support base 172 and is disposed between the lower case 52 and the upper fixing portion 27.

Therefore, the sanitization support portion 171 is disposed between the first case portion 10 and the second case portion 50, and when the first case portion 10 and the second case portion 50 are coupled to each other, movement of the sanitization support portion 171 is also restricted. Also, movement of air, which enters the first case portion 10 through the inlet portion 22, toward the second case portion 50 is blocked by the sanitization support portion 171, and thus a flow rate of air moving toward the fan module portion 70 may be increased, and air caring performance of the portable air caring apparatus 1 may be improved.

The pedestal portion 176 may be modified in various ways in which the pedestal portion 176 protrudes upward from the center of the sanitization support portion 171 to support the lower portion of the irradiating portion 180. Also, the pedestal portion 176 is disposed at the center of the inlet portion 22 in the radial direction, and a transverse cross-section of the pedestal portion 176 may be formed in a circular shape to reduce friction with air.

The pedestal portion 176 according to the first embodiment of the present invention may include a pedestal column 177 and a pedestal plate 178.

The pedestal column 177 may be formed in the shape of a column that protrudes upward from the center of the sanitization support portion 171. Also, the pedestal column 177 may be formed in a cylindrical or conical shape. The pedestal column 177 according to the first embodiment of the present invention is formed in a conical shape, whose transverse cross-section gradually narrows from a lower side toward an upper side, and is disposed at the center of the first case portion 10 in which the inlet portion 22 is formed. Thus, friction with air may be minimized.

A transverse cross-section of the sanitization support portion 171, which is installed to sanitize the filter portion 60, has a circular shape, and air that enters through the inlet portion 22 rotates in a spiral due to the inclined shape of the inlet holes 24, rotates along an outer side of the pedestal column 177, and moves to an upper side where the filter portion 60 is installed. That is, since the sanitizing portion 170 is disposed at a central portion of the first case portion 10 and air, which enters through the inlet portion 22, moves upward while rotating along the outer periphery of the sanitizing portion 170, resistance of a flow path of the sanitizing portion 170 is decreased.

Since the pedestal portion 176, the center of rotation of the fan member 90, and the core member 36 are disposed in a straight line in the perpendicular direction, resistance related to a flow of air moving from a lower side toward an upper side is decreased, and thus the air flow may occur more smoothly. As a result, air caring performance of the portable air caring apparatus 1 is improved.

The pedestal plate 178 according to the first embodiment of the present invention is formed in the shape of a plate that is installed in the horizontal direction at an upper side of the pedestal column 177. The irradiating portion 180 is installed at an upper side of the pedestal plate 178, and the pedestal plate 178 has a cross-sectional area that is larger than or equal to a cross-sectional area of the irradiating portion 180 to prevent light irradiated from the irradiating portion 180 from moving downward.

The irradiating portion 180 is mounted on an upper side of the pedestal portion 176 and may irradiate the sanitizing light in a direction toward the filter portion 60. Also, the irradiating portion 180 may be disposed on a vertical reference line that passes through the radial center of the inlet portion 22 in the vertical direction. Therefore, in a case in which the filter portion 60 is disposed at an upper side of the irradiating portion 180, the entire area of a lower end of the filter portion 60 may be sanitized by a relatively small number of sanitizing light sources 182, and thus production costs and maintenance and repair costs may be reduced.

Also, the irradiating portion 180 may be modified in various ways in which the irradiating portion 180 is installed at a position that is level with or higher than an upper end of the inlet portion 22. The irradiating portion 180 according to the first embodiment of the present invention may include a printed circuit board (PCB) 181 and the sanitizing light source 182. The PCB 181 is installed on the upper side of the pedestal plate 178, and the sanitizing light source 182 configured to irradiate the sanitizing light is installed on an upper side of the PCB 181. The sanitizing light source 182 may be an ultraviolet-C light emitting diode (UVC LED), or various other types of sanitizing apparatuses may be used as the sanitizing light source 182 in which the sanitizing light source 182 sterilizes the filter portion 60.

Meanwhile, since the sanitizing light source 182 of the sanitizing portion 170 is disposed at the upper side of the inlet portion 22, the sanitizing light source 182 may be prevented from irradiating the sanitizing light to the outside of the first case portion 10 through the inlet portion 22.

### [Air flow of portable air caring apparatus]

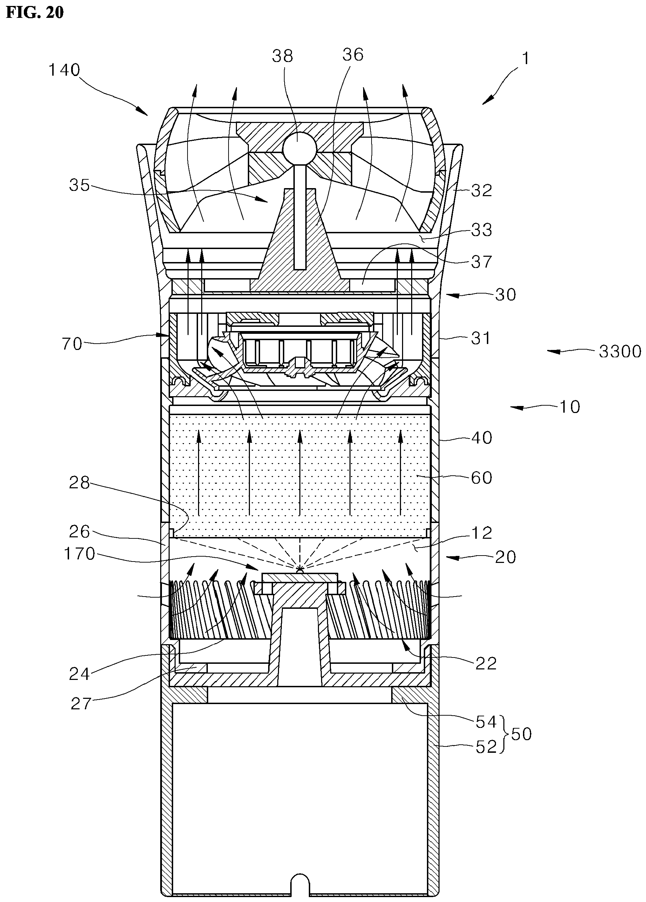

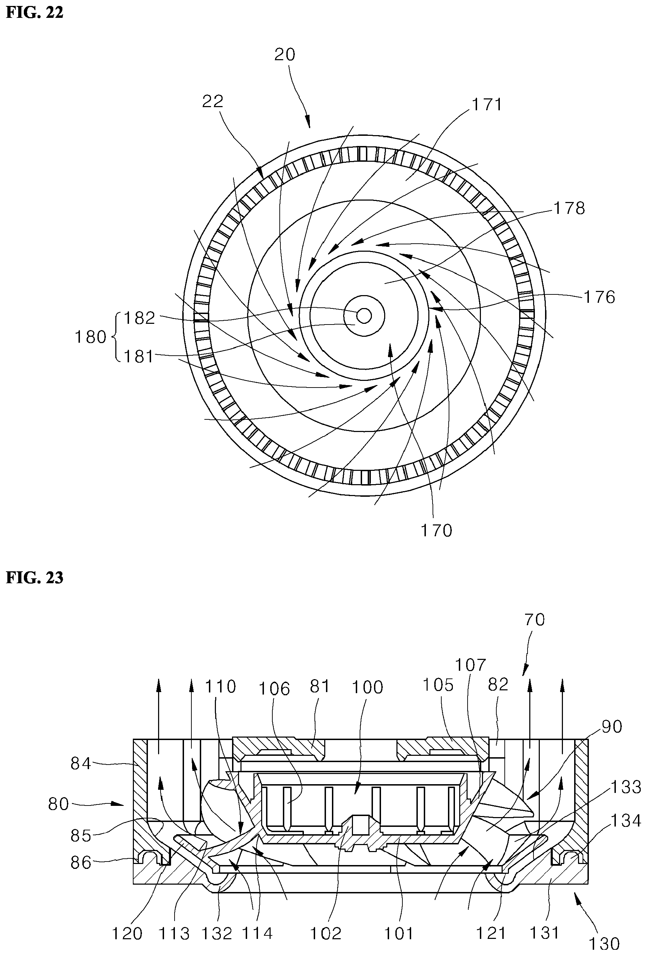

FIG. 20 is a cross-sectional view illustrating a flow of air passing through the portable air caring apparatus 1 according to the first embodiment of the present invention, FIG. 22 is a plan view illustrating a state in which air that has passed through the inlet portion 22 moves while rotating in a spiral around the sanitizing portion 170 according to the first embodiment of the present invention, and FIG. 23 is a cross-sectional view illustrating air passing through the fan module portion 70 according to the first embodiment of the present invention.

Hereinafter, aspects of air flow of the portable air caring apparatus 1 according to the present embodiment will be described with reference to FIGS. 20 and 22.

As illustrated in FIGS. 20 and 22, since the inlet portion 22 configured to suction outside air is installed along the outer periphery of the first case portion 10, air outside the first case portion 10 may move into the first case portion 10 through the inlet portion 22, and thus a suction flow rate of air may increase.

Due to the operation of the fan module portion 70, air outside the portable air caring apparatus 1 enters the portable air caring apparatus 1. Here, the air outside the portable air caring apparatus 1 passes through the inlet holes 24, which are installed in an inclined shape, and forms a spiral air flow that rotates along the outer periphery of the sanitization support portion 171.

Air, which enters the first case 20 and moves upward while rotating in a spiral, passes through the filter portion 60, and in this process, physical particles such as dust, fine dust, and ultrafine dust, chemical substances such as odorous particles and harmful gases, and microorganisms such as bacteria and viruses that are contained in the air may be filtered.

Since the filter portion 60 and the fan module portion 70 are disposed in a straight line in the vertical direction, suctioning and filtering of air may be effectively performed while flow loss of air is minimized.

Air that has passed through the filter portion 60, that is, purified air, may enter the fan module portion 70. A flow of air may be guided by the bell mouth 132, and in this way, air may be effectively guided to smoothly enter the fan module portion 70.

The air that enters the fan module portion 70 is discharged to the upper side of the fan module portion 70. The air discharged to the upper side of the fan module portion 70 may be discharged in a diagonal flow direction. Here, the diagonal flow direction may be defined as an upward diagonal direction.

Air suctioned into a central portion of the lower side of the fan module portion 70 is moved upward through a discharge port provided in an annular shape along an inner side of an edge of the fan module portion 70 and is moved upward through a space formed between the supports 37 of the rotation support portion 35. That is, since a diagonal flow fan is applied to the fan module portion 70, the air that enters the lower portion of the fan module portion 70 may be discharged in a direction that is inclined upward, and an air movement path of the rotation support portion 35 may coincide with a flow path direction. Thus, flow loss of air may be reduced.

In this way, air discharged to the upper side of the fan module portion 70, that is, purified air, enters the discharge portion 140 through a lower side thereof and is discharged to an upper side of the discharge portion 140.

Since the discharge portion 140 rotates within a predetermined angle range, a direction in which air is discharged may be controlled according to an angle at which the discharge portion 140 is installed.

Also, since the inner side of the discharge portion 140 forms a concave groove portion, an increase in discharge resistance of air, whose direction is switched through the discharge portion 140, may be reduced. Also, since the filter portion 60, the fan module portion 70, and the discharge portion 140 are disposed in a straight line in the vertical direction, suctioning and filtering of air and discharging of purified air may be effectively performed while flow loss of air is minimized.

### [Arrangement of components and spacing between components]

As illustrated in FIG. 21, a length of the inlet portion 22 in the first direction is set as D1, and a length of the blocking body 26, which is disposed at the upper side of the inlet portion 22, in the first direction is set as L2. Also, a length of the filter portion 60, which is disposed at an upper side of the blocking body, in the first direction is set as D2, and a length between the fan module portion 70, which is disposed at an upper side of the filter portion 60, and the filter portion 60 in the first direction is set as L1.

Also, a length of the fan module portion 70 in the first direction is set as D3, a distance between the support 37 of the rotation support portion 35, which is disposed at the upper side of the fan module portion 70, and the fan module portion 70 is set as D4, and a distance between the support 37 and a lower end of the discharge portion 140, which is installed to be inclined, is set as D5.

D2 is in a range of about 1.8 times D1 to 2.2 times D1. Since air that enters through the inlet portion 22 moves while rotating in a spiral, D1, which is the length of the inlet portion 22 in the first direction is shortened, and thus the size of the portable air caring apparatus 1 may be reduced. Also, since it is possible to sufficiently secure a space for installing the filter portion 60, air cleaning efficiency may be improved.

Also, D1, which is the length of the inlet portion 22 in the vertical direction, is formed to be shorter than D2, which is the length of the filter portion 60 in the vertical direction. Preferably, D1 may be formed to be greater than 0.57 times D2 and less than 0.77 times D2.

The filter portion 60 is formed in a cylindrical shape and is installed in the housing portion 3300 formed in a cylindrical shape. Therefore, an area of the filter portion 60 that comes in contact with air, which moves upward through the housing portion 3300, may be maximally secured, and the space inside the housing portion 3300 may be maximally utilized. Thus, air filtering performance may be improved.

Also, since the filter portion 60 is installed to be closer to the inlet portion 22 than the fan module portion 70, air, from which foreign substances are removed due to the air passing through the filter portion 60, is supplied to the fan module portion 70. Therefore, occurrence of a phenomenon, in which foreign substances such as dust are stuck in the fan module portion 70, is prevented to improve the durability of the fan module portion 70, and maintenance and repair costs for the fan module portion 70 may be reduced.

Also, resistance of air passing through the filter portion 60, which is at the lower side of the fan module portion 70, is higher than resistance of air being discharged to the upper side of the fan module portion 70. Therefore, since the fan module portion 70 is installed at the upper side of the filter portion 60 and suctions air into the upper side of the filter portion 60, air may be discharged to the upper side of the fan module portion 70 to facilitate an air blowing operation.

Also, since D2 is greater than D1 and since D2 is in a range of about 1.8 times D1 to 2.2 times D1, an installation space of the filter portion 60 is larger than an installation space of the inlet portion 22. Therefore, even when the amount of suctioned air, which has entered through the inlet portion 22, is large, an air caring ability of the filter portion 60 may be improved because an installation area of the filter portion 60 is larger than an installation area of the inlet portion 22.

Meanwhile, in a case in which the fan module portion 70 is disposed at the lower side of the filter portion 60 instead of being disposed at the upper side thereof, operational noise of the fan module portion 70 is transmitted to the outside of the housing portion 3300 through the inlet portion 22, and thus the number of customer complaints may be increased. Also, since air containing foreign substances is suctioned and immediately moves to the fan module portion 70, a phenomenon in which foreign substances such as dust are stuck in the fan module portion 70 may occur and the durability of the fan module portion 70 may be degraded, and maintenance and repair costs for the fan module portion 70 may also increase.

Also, a return air phenomenon may occur in which air discharged upward from the fan module portion 70 is blocked by the filter portion 60 and returns to the fan module portion 70, and thus air blowing performance and air caring performance of the portable air caring apparatus 1 may be significantly degraded.

Therefore, in order to reduce the operational noise of the portable air caring apparatus 1, reduce the maintenance and repair costs for the fan module portion 70, and improve the air blowing performance and air caring performance of the portable air caring apparatus 1, the fan module portion 70 is disposed at the upper side of the filter portion 60.

Also, L2 is formed to be less than D1. L2 is an essential length for air, which enters through the inlet portion 22, to evenly reach the entire area of the lower portion of the filter portion 60. However, since the vertical length of the portable air caring apparatus 1 increases as L2 becomes longer, the size of the portable air caring apparatus 1 may be further reduced as L2 becomes shorter.

In the present invention, since air entering through the inlet portion 22 enters the first case 20 while rotating in a spiral and moves upward, the air may be evenly distributed even when L2 is relatively short. Setting L2 as a length in a range of 0.5 times D1 to 0.7 times D1 may be suitable in consideration of a space in which the air is distributed.

Also, L1 is an essential length for air, which is discharged from the filter portion 60, to move through an inlet disposed at the center of the fan module portion 70. However, since the vertical length of the portable air caring apparatus 1 increases as L1 becomes longer, the size of the portable air caring apparatus 1 may be further reduced as L1 becomes shorter. According to the first embodiment of the present invention, L1 is less than L2. Meanwhile, D3 is formed to be less than D2 and greater than D1.

D4 and D5 are lengths necessary for air, which is discharged upward through the fan module portion 70, to enter the discharge portion 140. Since the discharge portion 140 avoids interference with the second case 30 and a direction of an air flow path is switched to a direction that is inclined upward, values of D4 and D5 are set in consideration of an extent to which resistance of the flow path increases.

Meanwhile, since the inlet portion 22 and the sanitizing portion 170 are installed to overlap each other such that space utilization is enhanced, compact design of the portable air caring apparatus 1 becomes possible. The portable air caring apparatus 1 is manufactured in a small size to be mounted in a cup holder of a vehicle or the like. Therefore, installing various components at optimal positions in the housing portion 3300 having a predetermined size may be of interest.

Accordingly, since the sanitizing portion 170 of the present invention is installed at a height at which the sanitizing portion 170 overlaps the inlet portion 22, as compared to an air caring apparatus in which the inlet portion 22 and the sanitizing portion 170 are installed at different heights, the length of the portable air caring apparatus 1 in the first direction, which is the vertical direction, may be reduced, and an installation area for the filter portion 60 and the fan module portion 70 may be increased. Thus, air cleaning efficiency may also be increased.

Also, since heat generated during operation of the sanitizing portion 170 is cooled by air entering through the inlet portion 22, heat dissipation of the sanitizing portion 170 may be promptly performed, and thus the durability of the sanitizing portion 170 may be improved.

Also, in order to reduce the size of the portable air caring apparatus 1, the housing portion 3300 is formed in a cylindrical shape extending in the vertical direction, and the center of the inlet portion 22, the center of the filter portion 60, the center of the sanitizing portion 170, the center of the fan module portion 70, the center of the rotation support portion, and the center of the discharge portion 140 in the radial direction coincide with each other along a vertical reference line that passes through the radial center of the housing portion 3300 in the vertical direction.

Therefore, air entering through the inlet portion 22 moves upward, sequentially passes through the filter portion 60, the fan module portion 70, the rotation support portion, and the discharge portion 140, and is discharged to the outside of the portable air caring apparatus 1, and such an air flow is formed in an upward linear direction. Since the sanitizing portion 170, the core member, the support plate, and the like, which are likely to interfere with movement of air, are disposed at the radial center of the housing portion 3300, an air movement path is formed in portions of the housing portion 3300 excluding a radial central portion thereof. Therefore, the housing portion 3300 may maximally secure the linearity of air moving in the vertical direction, and thus an air flow path may be optimized, an air flow speed may also be increased as compared to other air caring apparatuses, and air caring performance may also be improved.

### [Another example of fan module portion]

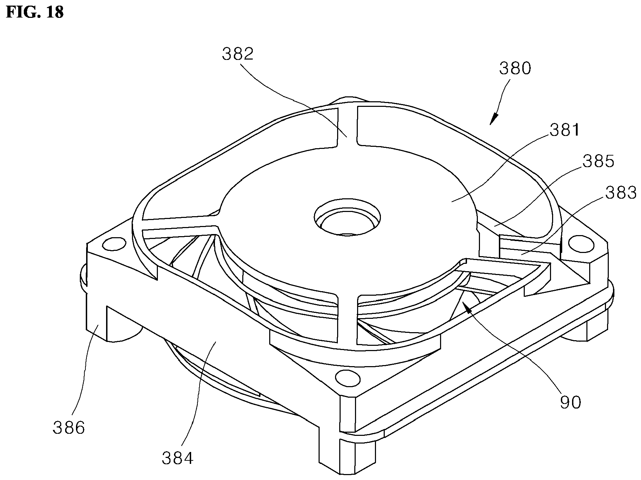

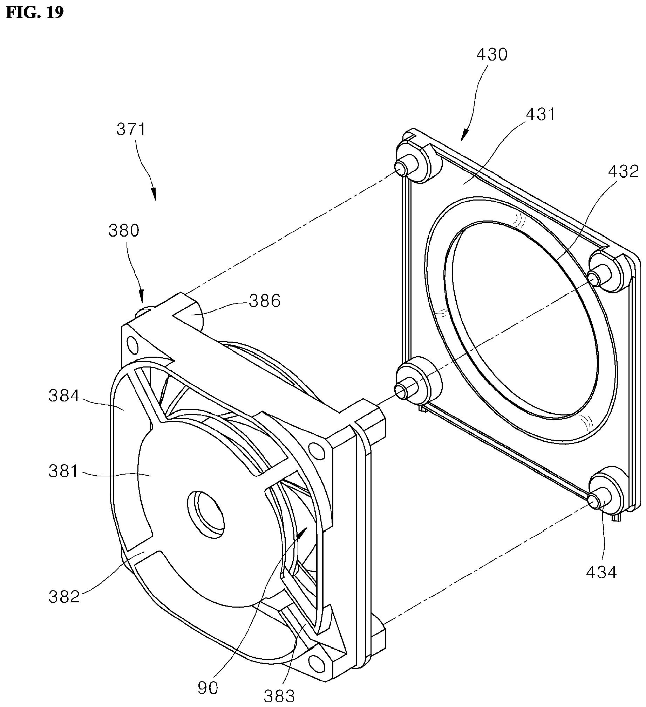

FIG. 18 is a perspective view illustrating another fan module portion 371 according to the first embodiment of the present invention, and FIG. 19 is an exploded perspective view illustrating the fan module portion 371 according to the first embodiment of the present invention.

As illustrated in FIGS. 18 and 19, an outer shape of the fan module portion 371 may be a quadrilateral shape. Also, since a circular diagonal flow fan module is applied to the inside of the fan module portion 371, a small-sized upward discharge type air cleaner that can maximize air flow performance may be provided. The fan module portion 371 according to another embodiment of the present invention may include a fan housing 380, the fan member 90, and a fan base 430.

### [Fan housing]

An outer shape of the fan housing 380 may be modified in various ways in which the fan housing 380 includes a quadrilateral edge. The fan housing 380 according to the first embodiment of the present invention may include at least any one of a support plate 381, a connection support 382, a wire guide 383, a side support portion 384, an inner side guiding portion 385, and a protruding boss 386.

The support plate 381 is formed in a disk shape, and a hole is formed at the center of the support plate 381. A motor may be installed at the center of the support plate 381, or a shaft connected to the motor may be installed in the first direction.

The connection support 382 extends to the outside of the support plate 381 and is connected to the side support portion 384. The connection support 382 according to the first embodiment of the present invention may be provided as a plurality of connection supports 382 and may be installed in the shape of a rod. The connection supports 382 extending radially outward from the support plate 381 may be connected to the side support portion 384.

The wire guide 383 is continuously installed on the connection support 382 and supports a lower portion of a wire of an electronic device so that the wire may move along a side surface of the connection support 382. The wire guide 383 is formed in the shape of a protrusion, which is disposed on a lower portion of a side surface of the connection support 382, and guides a wire of the motor installed on the support plate 381 to be installed to extend to the outside of the fan housing 380. The wire guide 383 may be installed at the side surface of the connection support 382 and form a concave groove to allow the wire to be disposed therein. Therefore, since the wire installed in the wire guide 383 is disposed in the concave groove disposed at the side surface of the connection support 382, and the lower portion of the wire is supported by the wire guide 383, damage to the wire may be prevented.

The side support portion 384 is formed in the shape of a quadrilateral frame, and the upper and lower sides of the side support portion 384 are open. An outer side of the side support portion 384 is formed in the shape of a quadrilateral frame, and an air movement path and a quadrilateral or circular inner space is formed in the side support portion 384.

The inner side guiding portion 385 forms an inclined surface that is installed to be inclined downward toward a radially inward side from a lower side of the side support portion 384. The inner side guiding portion 385 may be formed at the inner side of the side support portion 384 and may prevent a return air phenomenon in which air, which is blown upward by the fan member 90, moves to an inlet of the fan member 90 through an outer side surface of the fan member 90.

The protruding boss 386 extends to a lower end of the side support portion 384 and may be modified in various ways in which the protruding boss 386 includes a groove portion for inserting the coupling protrusion 134 of the fan base 430 which will be described below. The protruding boss 386 according to the first embodiment of the present invention is installed at each corner of the side support portion 384.

A diagonal flow fan is used as the fan member 90. Since description relating thereto has been given above, detailed description thereof will be omitted.

### [Fan base]

The quadrilateral fan base 430 is coupled to a lower side of the quadrilateral fan housing 380 and may be modified in various ways in which the fan base 430 guides air, which has passed through the filter portion 60, to enter the fan member 90.

An edge of the fan base 430 may be formed in a shape that corresponds to the shape of an edge of the fan housing 380. For example, in a case in which the fan housing 380 is formed in a quadrilateral shape, the fan base 430 may be installed in the shape of a quadrilateral plate having a hollow formed therein.

A base plate 431 is formed in the shape of a quadrilateral plate and has a hollow formed at the center to allow movement of air.

A bell mouth 432 is installed in an annular shape at an inner side of the base plate 431 that faces the hollow. The bell mouth 432 extends in the circumferential direction and has a longitudinal cross-section formed in a concave shape that surrounds a lower side of the inlet protrusion 121 disposed at the shroud 120. The bell mouth 432 may be formed in a shape that surrounds an outer peripheral surface of the hollow formed at the center of the base plate 431. The bell mouth 432 is formed to be convex toward the lower side and may form a groove portion that is concave toward the upper side.

A coupling protrusion 434 protrudes to an upper side of the base plate 431 and is coupled to the groove portion of the protruding boss 386, which is disposed in the fan housing 380, by being fitted thereto to fix the fan base 430 to a lower side of the fan housing 380. The fan base 430 and the fan housing 380 may be coupled to each other at a plurality of points due to coupling performed between the coupling protrusion 434 and the protruding boss 386. When coupling between the fan base 430 and the fan housing 380 is performed as described above, the fan member 90 may be rotatably installed between the fan base 430 and the fan housing 380.

### [Second embodiment - Fan module portion]

Hereinafter, a portable air caring apparatus according to a second embodiment of the present invention will be described with reference to the drawings.

For convenience of description, elements which have the same configuration and effect as in the first embodiment of the present invention will be denoted by the same reference numerals as in the first embodiment and may be described using the same drawings as in the first embodiment. As necessary, description of such elements may be omitted.

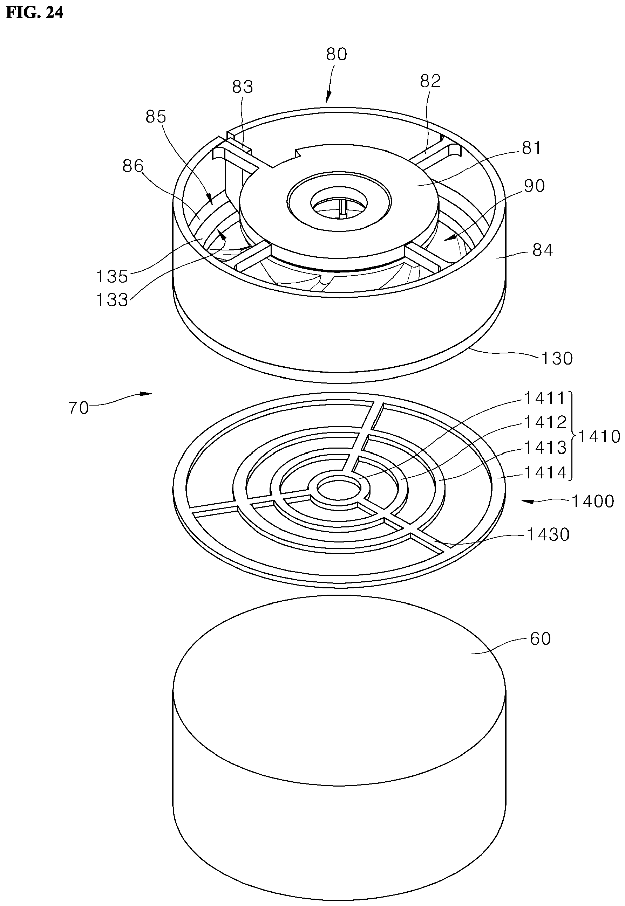

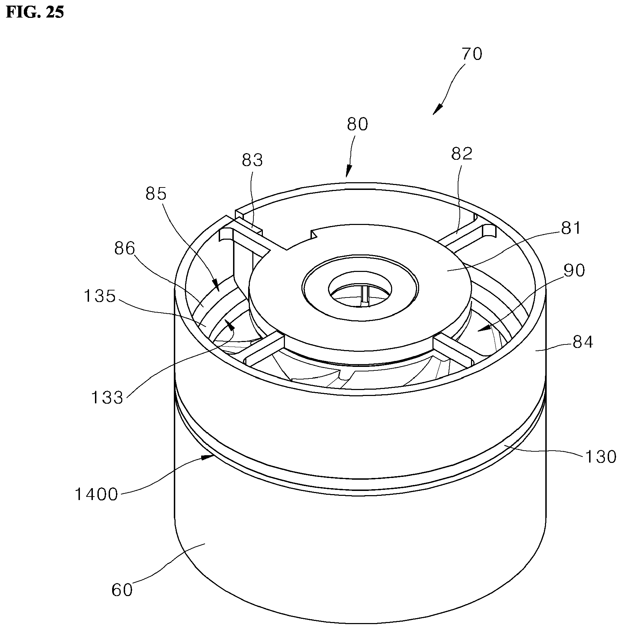

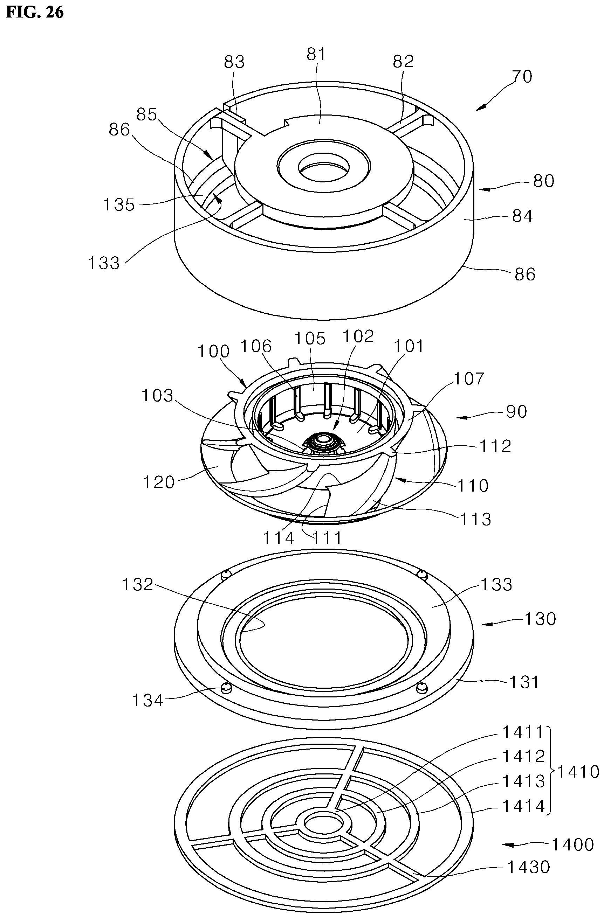

FIG. 24 is a perspective view illustrating a fan module portion 70 and a filter portion 60 according to the second embodiment of the present invention, FIG. 25 is a perspective view of the fan module portion 70 according to the second embodiment of the present invention, and FIG. 26 is an exploded perspective view of the fan module portion 70 according to the second embodiment of the present invention.

As illustrated in FIGS. 24 to 26, in the fan module portion 70 according to the present invention, a safety portion 1400 is installed at an outer side of a fan base 130 to form a protective mesh at an outer side of a suction portion 136. Since the safety portion 1400 including a blocking portion 1410 and a safety support 1430 is installed at the outer side of the fan base 130, when replacing the filter portion 60, even when a user moves his or her fingers in a direction toward the suction portion 136, the fingers are caught on the blocking portion 1410 and the safety support 1430 and are blocked from coming in contact with the suction portion 136.

Also, since the blocking portion 1410 is not installed at a position facing the suction portion 136, and the blocking portion 1410 is installed in an area that does not face the suction portion 136, an area in which air moving to the suction portion 136 through the filter portion 60 comes in contact with the safety portion 1400 is reduced, and thus the air flow may occur more smoothly.

Also, the fan module portion 70 is disposed between an inlet portion 22, which is disposed at one side of a housing portion 3300, and an outlet 33, which is disposed at the other side of the housing portion 3300, and may be modified in various ways in which the fan module portion 70 rotates a fan to blow air in a direction toward the outlet 33.

Since a type of fan of the fan module portion 70 is a diagonal flow fan and an internal structure of the fan module portion 70 is changed to mount the diagonal flow fan, a small-sized air cleaner that can maximize air flow performance may be provided.

The fan module portion 70 according to the second embodiment of the present invention may include a fan member 90 which is rotatably installed, the fan base 130 which is continuously installed on the fan member 90 and configured to guide air to enter in a direction toward the fan member 90, and the safety portion 1400 which includes the blocking portion 1410, which is continuously installed on the fan base 130 and configured to block movement of an external object toward an inner side of the suction portion 136 disposed in the fan base 130, and the safety support 1430, which is configured to support the blocking portion 1410.

Also, since a fan housing 80, the fan member 90, and the fan base 130 may be coupled to form a module, or the fan housing 80, the fan member 90, the fan base 130, and the safety portion 1400 may be coupled to form a module, assembly and disassembly may be easily performed, and production costs and maintenance and repair costs may be reduced.

The fan member 90 according to the present invention rotates due to operation of a motor. Only a rotating shaft of the motor that rotates the fan member 90 may be connected to the fan member 90, a rotor may be installed at the fan member 90, and a stator may be installed in the fan housing 80 whose rotation is restricted. Since a magnetic field of the stator changes, the shaft that rotates along with the rotor may be connected to the fan member 90, and the rotor and the fan member 90 may rotate about the stator. Since the configuration of the motor rotating the fan member 90 is a known configuration, detailed description thereof will be omitted.

Directions will be defined. When a direction in which a discharge portion 140 is located from a first case portion 10 is referred to as "upper portion" and a direction in which a second case portion 50 is located from the first case portion 10 is referred to as "lower portion," "first direction" refers to a vertical direction or an axial direction. The first direction may also refer to a perpendicular direction. Also, "second direction" is a direction perpendicular to the first direction and refers to a lateral direction, a horizontal direction, or a radial direction.

### [Fan housing]

The fan housing 80 has an operating space provided therein. The fan housing 80 according to the second embodiment of the present invention is fixed to the inside of the housing portion 3300 and may be modified in various ways in which an operating space for allowing the fan member 90 to rotate is provided inside the fan housing 80. The fan housing 80 according to the second embodiment of the present invention may include at least any one of a support plate 81, a connection support 82, a wire guide 83, a side support portion 84, an inner side guiding portion 85, and a protrusion mounting groove portion 87.

The support plate 81 is disposed at the center of the fan housing 80 and may be formed in a disk shape. Also, a hole is formed at the center of the support plate 81. A motor may be installed at the center of the support plate 81, or a shaft connected to the motor may be installed in the vertical direction, which is the first direction.

The connection support 82 extends radially outward from the support plate 81 and is connected to the side support portion 84. The connection support 82 according to the second embodiment of the present invention may be provided as a plurality of connection supports 82 and may be installed in the shape of a rod. The connection supports 82 extending radially outward from the support plate 81 may be connected to the side support portion 84.

The connection support 82 according to the second embodiment of the present invention is disposed at a lower side of a core support portion 350 of a rotation support portion 300 which will be described below. As the connection support 82, four connection supports 82 are installed at 90° intervals about the support plate 81, and the core support portion 350 is installed at upper sides of the connection supports 82 that face each other.

The wire guide 83 is continuously installed on the connection support 82 and supports a lower portion of a wire of an electronic device so that the wire may move along a side surface of the connection support 82. The wire guide 83 is formed in the shape of a protrusion, which is disposed on a lower portion of a side surface of the connection support 82, and guides a wire of the motor installed on the support plate 81 to be installed to extend to the outside of the fan housing 80. The wire guide 83 may be installed at the side surface of the connection support 82 and form a concave groove to allow the wire to be disposed therein. Therefore, since the wire installed in the wire guide 83 is disposed in the concave groove disposed at the side surface of the connection support 82, and the lower portion of the wire is supported by the wire guide 83, damage to the wire may be prevented.

The side support portion 84 is formed in the shape of a cylindrical pipe, and the upper and lower sides of the side support portion 84 are open. An outer side of the side support portion 84 comes in contact with the inside of the housing portion 3300, and an inner side of the side support portion 84 is connected to the connection support 82. Also, the side support portion 84 is spaced apart from the support plate 81 and connected to the connection support 82 and may be modified in various ways in which the side support portion 84 forms a circular curved surface along the outer periphery thereof or has a polygonal shape.

The inner side guiding portion 85 forms a first inclined surface 88 that is installed to be inclined downward toward a radially inward side from a lower side of the side support portion 84. The inner side guiding portion 85 may be formed at the inner side of the side support portion 84 and may prevent a return air phenomenon in which air, which is blown upward by the fan member 90, moves to an inlet of the fan member 90 through an outer side surface of the fan member 90. Also, the inner side guiding portion 85 protrudes to the inner side of the side support portion 84, and the first inclined surface 88, whose inner diameter gradually narrows in a direction toward where the fan base 130 is installed, is provided. Also, the inner side guiding portion 85 may be installed at the lower side of the side support portion 84 that faces the fan base 130.

The protrusion mounting groove portion 87 forms a concave groove shape in a lower side surface of the side support portion 84, and a coupling protrusion 134 disposed on the fan base 130 is connected to the protrusion mounting groove portion 87. The protrusion mounting groove portion 87 may be modified in various ways in which the protrusion mounting groove portion 87 includes a groove portion for inserting the coupling protrusion 134 of the fan base 130. The protrusion mounting groove portion 87 according to the second embodiment of the present invention is provided as a plurality of protrusion mounting groove portions 87 installed in a circumferential direction of the side support portion 84.

### [Fan member]

The fan member 90 is rotatably installed in the fan housing 80 and may be modified in various ways in which the fan member 90 is able to move air in a direction toward the discharge portion 140.

A diagonal flow fan may be used as the fan member 90, but this is only the second embodiment of the present invention, and other types of fans may also be used as the fan member 90 according to the present invention. The fan member 90 according to the second embodiment of the present invention may include a hub 100 which is disposed at the center of the fan housing 80 and configured to receive external power and rotate, a plurality of fan blades 110 which are disposed to be spaced apart from each other at equal intervals along an outer peripheral surface of the hub 100, and a shroud 120 which is connected to the other side end portion of the fan blade 110, installed in an annular shape, and spaced apart from the fan base 130.

### [Hub]

The hub 100 is disposed at the center of the fan housing 80 and may be modified in various ways in which the hub 100 receives external power and rotates. A lower side of the hub 100 is formed in the shape of a circular plate, and the suction portion 136 is formed between the hub 100 and the fan base 130. Therefore, air outside the fan module portion 70 may move into the fan member 90 through the suction portion 136.

The hub 100 is disposed at the center of the fan member 90 in the radial direction and may rotate along with the rotor and the shaft, which is an output shaft of the motor, that constitute the motor. The hub 100 according to the second embodiment of the present invention may include at least any one of a hub plate portion 101, an axial coupling portion 102, an inner side protruding portion 105, a skirt portion 107, first reinforcing protrusions 103, and second reinforcing protrusions 106.

The hub plate portion 101 is rotatably installed in the fan housing 80 and is disposed at the center of the fan housing 80 in the radial direction. Also, the hub plate portion 101 may be formed in a disk shape that is parallel to the support plate 81. The axial coupling portion 102 may be provided on the hub plate portion 101.

The axial coupling portion 102 may be disposed at the center of the hub plate portion 101 in the radial direction. The axial coupling portion 102 may be formed to protrude in at least any one direction of a direction toward an upper side of the hub plate portion 101 and a direction toward a lower side of the hub plate portion 101. Also, the axial coupling portion 102 may be coupled to an axial end portion of a shaft, which is configured to transmit rotary power, to receive the rotary power. For example, coupling between the axial coupling portion 102 and the shaft may be performed in a form in which the shaft is fitted to the axial coupling portion 102.

The first reinforcing protrusions 103 are installed at predetermined intervals along the outer periphery of the axial coupling portion 102. The first reinforcing protrusions 103 are radially installed about the center of the axial coupling portion 102 and are formed as band-shaped protrusions at an outer side of the axial coupling portion 102. Therefore, since stress concentrated on the axial coupling portion 102 is distributed through the first reinforcing protrusions 103, structural rigidity of the axial coupling portion 102 may be improved.

The inner side protruding portion 105 extends from the hub plate portion 101 toward an upper side at which the support plate 81 is installed. The inner side protruding portion 105 according to the second embodiment of the present invention is installed in an arc shape along the outer side edge of the hub plate portion 101. The inner side protruding portion 105 according to the second embodiment of the present invention may be formed in the shape of a pipe that extends in the vertical direction.

Also, the second reinforcing protrusions 106 are installed at predetermined intervals along the inner periphery of the inner side protruding portion 105. The second reinforcing protrusions 106 extend in the vertical direction along an inner side surface of the inner side protruding portion 105, and lower sides of the second reinforcing protrusions 106 are formed in the shape of a band that is bent in a direction toward the axial coupling portion 102 and connected to the hub plate portion 101. The second reinforcing protrusions 106 are installed in the first direction along an inner side surface of the inner side protruding portion 105, and lower sides of the second reinforcing protrusions 106 are formed as band-shaped protrusions that extend toward the axial coupling portion 102. Therefore, since stress concentrated on the inner side protruding portion 105 is distributed through the second reinforcing protrusions 106, structural rigidity of the inner side protruding portion 105 may be improved. As necessary, the rotor of the motor may be fixed to the inner side of the inner side protruding portion 105.

The skirt portion 107 may protrude upward from the inner side protruding portion 105 or the hub plate portion 101 toward the support plate 81. The skirt portion 107 according to the second embodiment of the present invention extends from an outer side of the inner side protruding portion 105 so as to be inclined upward. Also, the skirt portion 107 may form an inclined surface that is further inclined outward in the radial direction away from the hub plate portion 101 in the first direction. The skirt portion 107 is disposed at an outer side of the inner side protruding portion 105, and an inner diameter of the skirt portion 107 gradually increases from a lower side toward an upper side.

For example, the hub plate portion 101 and the skirt portion 107 may be connected in the shape of a truncated cone in which a hollow 137 is formed and whose one side is open. The skirt portion 107 may be formed in the shape of a funnel in which an upper side is open and a lower side is blocked by the hub plate portion 101.

### [Fan blade]

The fan blade 110 may be provided as a plurality of fan blades 110, and the plurality of fan blades 110 may be disposed to be spaced apart from each other at equal intervals along an outer peripheral surface of the hub 100. The fan blades 110 protrude to the outside of the hub 100 with respect to the center of the hub 100 and extend in a spiral shape. Also, the plurality of fan blades 110 may be disposed to be spaced apart from each other at predetermined intervals in a peripheral direction of the hub 100.

The fan blades 110 according to the second embodiment of the present invention may protrude to the outside of the skirt portion 107 in a centrifugal direction extending in a spiral shape from the center of the axial coupling portion 102. Also, when a direction from the outside of the axial coupling portion 102 toward the axial coupling portion 102 is the radial direction, the inner side of the fan blades 110 in the radial direction may be connected to the skirt portion 107, and the outer side of the fan blades 110 in the radial direction may be connected to the shroud 120 which will be described below. Also, one side end portion of the fan blade 110 is connected to the hub 100, connects the skirt portion 107 and the shroud 120 to each other, and extends in a spiral shape.

The skirt portion 107 is a portion of the hub 100 that is directly connected to the fan blades 110 and is a portion that also comes in direct contact with air passing through the fan blades 110. The skirt portion 107 is also closely related to a flow path of air passing through the fan module portion 70.

Each fan blade 110 that connects the shroud 120 and the skirt portion 107 to each other may include a first end portion 111, a second end portion 112, a first edge 113, and a second edge 114.

The first end portion 111 is disposed at a front end of the fan blade 110 in a rotational direction thereof and may be formed in a linear shape that extends in the radial direction. The rotational direction is defined as a direction in which rotation of the fan member 90 occurs.

The second end portion 112 is disposed at a rear end of the fan blade 110 in the rotational direction thereof and may be radially formed about the axial coupling portion 102.

The first edge 113 may connect one end of the first end portion 111 and one end of the second end portion 112. The first edge 113 may be connected to an inner peripheral surface of the shroud 120.

The second edge 114 may connect the other end of the first end portion 111 and the other end of the second end portion 112. The second edge 114 may be connected to the outer peripheral surface of the hub 100.

That is, the one end of the first end portion 111 and the one end of the second end portion 112 may be connected to the inner peripheral surface of the shroud 120. Also, the other end of the first end portion 111 and the other end of the second end portion 112 may be connected to an outer peripheral surface of the skirt portion 107.

The one end of the first end portion 111 may be disposed closer to the center of the hub plate portion 101 in the radial direction than the one end of the second end portion 112. Also, the other end of the second end portion 112 may be disposed closer to the center of the hub plate portion 101 in the radial direction than the other end of the first end portion 111. This is because the one end and the other end of the first end portion 111 are disposed more toward the front in the rotational direction than the one end and the other end of the second end portion 112, and the skirt portion 107 is formed such that a radius thereof gradually decreases toward the front in the rotational direction.

According to the present embodiment, the fan blade 110 is connected to the skirt portion 107 of the hub 100. In order to guide a flow of air entering the fan module portion 70 in a direction that is inclined upward, the skirt portion 107 forms an inclined surface that is inclined upward.

### [Shroud]

The shroud 120 is connected to the other side end portion of the fan blade 110 and installed in an annular shape and may be modified in various ways in which the shroud 120 may be spaced apart from the fan base 130.

The shroud 120 is installed along the outer periphery of the skirt portion 107, and the shroud 120 and the skirt portion 107 are connected to each other by the fan blade 110. Also, an outer diameter of the hub 100 and an inner diameter of the shroud 120 may gradually decrease in a direction from an upper side toward a lower side.

Also, since a distance between the shroud 120 and the skirt portion 107, which are connected to each other by the fan blade 110, gradually increases from a lower side toward an upper side, air blowing using a diagonal flow fan which discharges air in a direction that is inclined upward may occur more smoothly, and thus the amount of blown air may be increased.

The shroud 120 may be spaced a predetermined distance apart from the hub 100 in the radial direction and may be disposed at an outer side of the hub 100 in the radial direction. Also, the shroud 120 may be spaced apart from the hub 100 by as much as a distance that corresponds to a length of the fan blade 110 in the radial direction. Also, each fan blade 110 may connect the skirt portion 107, which is disposed at the hub 100, and the shroud 120 to each other.

The shroud 120 may form an inclined surface that is substantially parallel to the skirt portion 107. In the present embodiment, the skirt portion 107 and the shroud 120 are illustrated as being arranged in a form in which a distance between the skirt portion 107 and the shroud 120 gradually increases in a direction toward an upper side of the shroud 120.

The shroud 120 according to the second embodiment of the present invention further includes an inlet protrusion 121 disposed at a lower side of the shroud 120. The inlet protrusion 121 disposed at the lower side of the shroud 120 is a ring-shaped protrusion that extends in the first direction from the lower side of the funnel-shaped shroud 120. Also, the inlet protrusion 121 extends downward and is spaced a predetermined distance apart from the fan base 130. Since the inlet protrusion 121 is disposed at an inner side of the bell mouth 132 which will be described below, the inlet protrusion 121 may prevent the returning flow of air, which enters through an inlet provided in the lower side of the shroud 120, along an outer side of the shroud 120.

### [Fan base]

The fan base 130 may be modified in various ways in which the fan base 130 is coupled to the fan housing 80 and guides air to enter in a direction toward the fan member 90. The fan base 130 may include a base plate 131, which is formed in the shape of a plate that extends in an annular shape and has the hollow 137 formed at the center to allow movement of air and a bell mouth 132, which is disposed in an annular shape along an inner side of the base plate 131 that faces the hollow 137 and which forms a groove portion, which is concave upward, and surrounds a lower side of the inlet protrusion 121. The fan base 130 according to the second embodiment of the present invention may include at least any one of the base plate 131, the bell mouth 132, a protruding rib 133, and the coupling protrusion 134.

The fan base 130 may be disposed between the filter portion 60 and the fan member 90. Also, an edge of the fan base 130 may be formed in a shape that corresponds to the shape of an edge of the filter portion 60. For example, when the filter portion 60 is formed in a cylindrical shape and the edge of the filter portion 60 is formed in a circular shape, the fan base 130 may be installed in an annular shape having the hollow 137 formed therein.

Also, the safety portion 1400 may be additionally installed between the fan base 130 and the filter portion 60. The safety portion 1400 blocks the filter portion 60 from being suctioned into the suction portion 136, which is disposed in the fan base, to prevent a phenomenon in which an external object, including a finger of a user, accesses the inside of the suction portion 136.

The base plate 131 may be disposed between the filter portion 60 and the fan member 90 or disposed between the safety portion 1400 and the fan member 90. The base plate 131 is formed in the shape of a plate extending in an annular shape and has the hollow 137 formed at the center to allow movement of air.

The bell mouth 132 is installed in an annular shape at an inner side of the base plate 131 that faces the hollow 137. The bell mouth 132 may be installed in a shape that surrounds an end portion of a lower side of the fan member 90. The bell mouth 132 according to the second embodiment of the present invention extends in the circumferential direction and has a longitudinal cross-section formed in a concave shape that surrounds a lower side of the inlet protrusion 121 of the shroud 120 that protrudes downward.

The bell mouth 132 may be formed in a shape that surrounds an outer peripheral surface of the hollow 137 formed at the center of the base plate 131. The bell mouth 132 is formed to be convex toward the lower side and may form a groove portion that is concave toward the upper side.

At least one portion of the bell mouth 132 may be inserted into the shroud 120 in the radial direction. The bell mouth 132 may guide a suctioning flow at the inlet of the fan module portion 70 to contribute to an improvement in suctioning and discharging performance of the fan module portion 70.

The coupling protrusion 134 protrudes to an upper side of the base plate 131 and is coupled to the groove portion of the protrusion mounting groove portion 87, which is disposed in the fan housing 80, by being fitted thereto to fix the fan base 130 to the lower side of the fan housing 80.

The fan base 130 and the fan housing 80 may be coupled to each other at a plurality of points due to coupling performed between the coupling protrusion 134 and the protrusion mounting groove portion 87. When coupling between the fan base 130 and the fan housing 80 is performed as described above, the fan member 90 may be rotatably installed between the fan base 130 and the fan housing 80.

The protruding rib 133 protrudes from the base plate 131 and may be disposed at an outer side of the bell mouth 132 in the radial direction. The protruding rib 133 according to the second embodiment of the present invention is located at the outer side of the bell mouth 132 in the radial direction and is installed in an annular shape that surrounds the outer periphery of the bell mouth 132. Also, the protruding rib 133 may be integrally formed with the base plate 131. More specifically, the base plate 131, the bell mouth 132, and the protruding rib 133 may be integrally formed.

The protruding rib 133 according to the second embodiment of the present invention is spaced apart from the shroud 120 and has a second inclined surface 135 whose inner diameter gradually increases upward. Also, the second inclined surface 135 is installed to be parallel to an outer side surface of the shroud 120. Also, the first inclined surface 88 and the second inclined surface 135 may form the same angle with a horizontal line.

Also, the protruding rib 133 may be installed to be inclined at the same angle as an outer side surface of the shroud 120, and a distance between the protruding rib 133 and the shroud 120 may be maintained to be constant. The protruding rib 133, which faces the shroud 120, protrudes to an upper side of the base plate 131 and forms the second inclined surface 135 at an upper side thereof. The second inclined surface 135 of the protruding rib 133 may be installed to be spaced a predetermined distance apart from the shroud 120 and be parallel to the outer side of the shroud 120.

Also, the second inclined surface 135 of the protruding rib 133 may have the same angle of inclination as the first inclined surface 88 of the inner side guiding portion 85 disposed in the fan housing 80. Therefore, it is possible to prevent a return air phenomenon in which a portion of air, which has moved upward through a space between the shroud 120 and the skirt portion 107, moves to the inlet of the fan member 90 through a space between the shroud 120 and the protruding rib 133.

Since a path between the fan member 90 and the fan base 130 is formed to be narrow, and since the bell mouth 132 disposed at the fan base 130 is installed in a shape that surrounds the lower end of the shroud 120, a flow of air moving to the inlet of the fan member 90 through the outside of the fan member 90 is reduced or blocked. Thus, the return air phenomenon may be prevented.

The suction portion 136, which is a space in which air is suctioned into the fan member 90, is formed between an inner diameter of the bell mouth 132 and an outer diameter of the hub plate portion 101. The suction portion 136 is a ring-shaped area disposed at an outer side of the hub plate portion 101. The suction portion 136 may be disposed between the hub 100, which is disposed at the center of the fan member 90, and the fan base 130 and form a path along which air is suctioned into the fan member 90.

### [Safety portion]

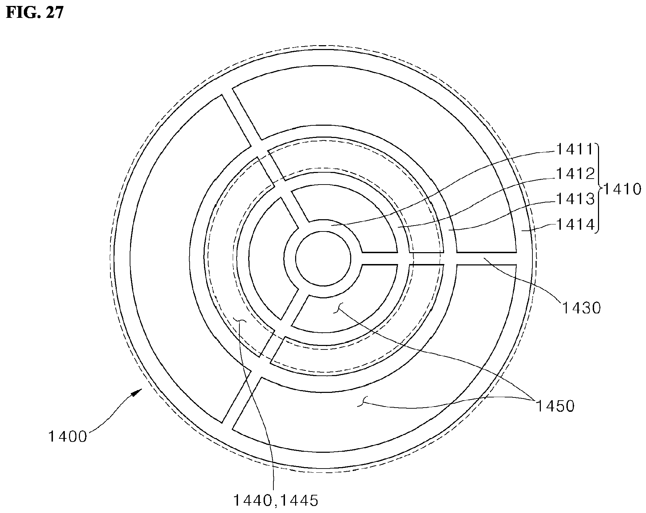

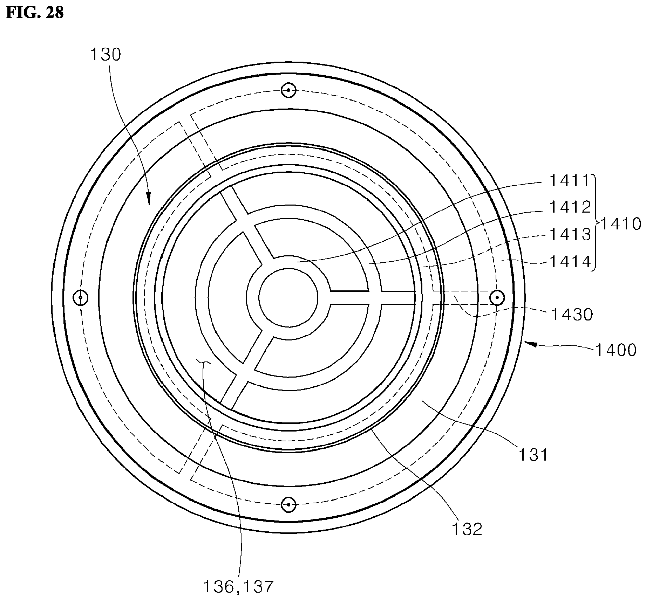

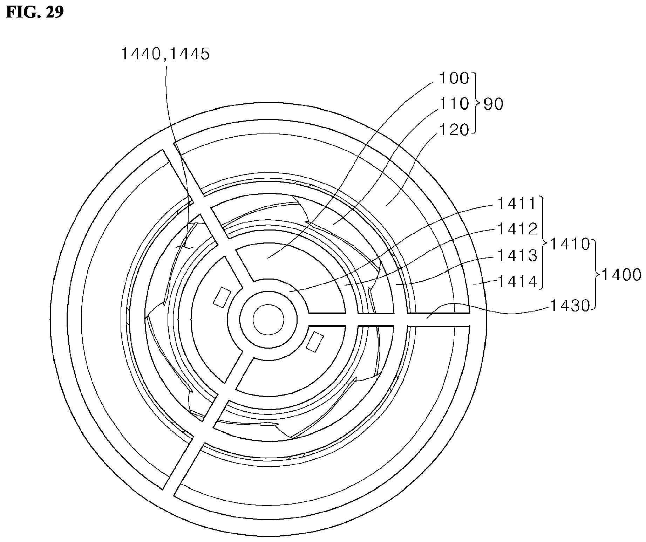

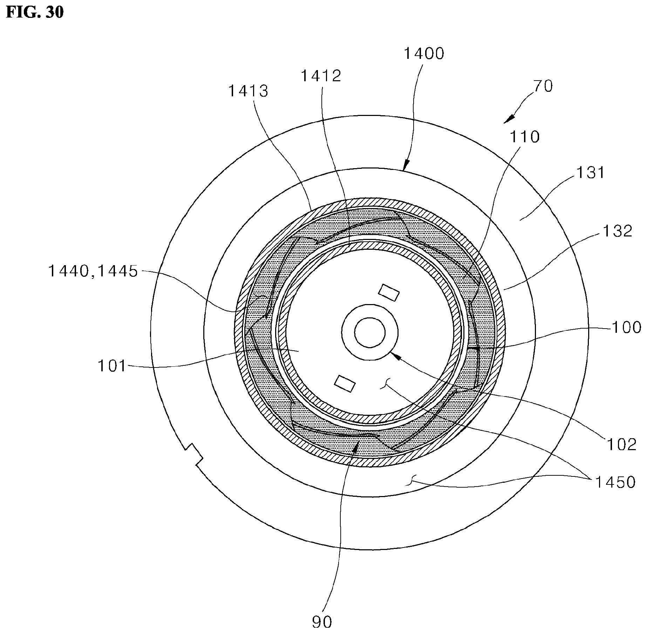

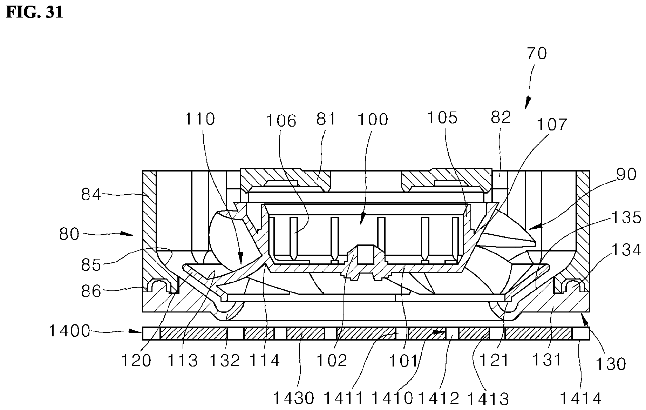

FIG. 27 is a plan view of the safety portion 1400 according to the second embodiment of the present invention, FIG. 28 is a bottom view illustrating the fan base 130 and the safety portion 1400 according to the second embodiment of the present invention, FIG. 29 is a bottom view illustrating the fan member 90 and the safety portion 1400 according to the second embodiment of the present invention, FIG. 30 is a view in which portions of the fan member 90 and the fan base 130 that face the blocking portion 1410 are indicated according to the second embodiment of the present invention, and FIG. 31 is a plan view of the fan module portion 70 according to the second embodiment of the present invention.

As illustrated in FIGS. 27 to 31, the safety portion 1400 is continuously installed on the fan base 130 and includes the blocking portion 1410 configured to block movement of an external object into the suction portion 136 disposed in the fan base 130 and the safety support 1430 configured to support the blocking portion 1410. The safety portion 1400 may be modified in various ways in which the safety portion 1400 minimizes flow resistance of air, which enters the suction portion 136 disposed between the fan base 130 and the hub 100 of the fan member 90, and blocks entry of a foreign object through the suction portion 136. The safety portion 1400 may be formed in the shape of a mesh that includes a plurality of through-holes. Also, the safety portion 1400 may be coupled to a lower portion of the fan base 1300 or may be spaced apart from the fan base 130 and fixed to the inside of the housing portion 3300. Also, the safety portion 1400 may be fixed to a filter case that faces the fan base 130. In this way, various modifications are possible.

The safety portion 1400 may be installed in a disk shape and have a predetermined thickness, and a hole is formed in the safety portion 1400 to allow air, which is moved toward the suction portion 136, to pass therethrough. The safety portion 1400 is designed to reduce flow resistance of air being suctioned into the fan member 90. Also, the safety portion 1400 may be integrally formed with a separate filter case, on which the filter portion 60 is mounted, and may, when replacing the filter portion 60, prevent an external object, including a finger, from coming in contact with the fan blade 110 to prevent occurrence of accidents.

The safety portion 1400 according to the second embodiment of the present invention may be divided into a first area 1440 that faces the suction portion 136 and a second area 1450 that does not face the suction portion 136. Since the first area 1440 is formed so that a flow rate of air moving toward the suction portion 136 is higher than in the second area 1450, the blocking portion 1410 may only be disposed in the second area 1450. Since the blocking portion 1410 is disposed in the second area 1450, an increase in frictional resistance of air passing through the safety portion 1400 and entering the suction portion 136 may be minimized.

For example, in a case in which the safety portion 1400 is installed in a disk-shaped area, the first area 1440 is a ring-shaped area that faces the suction portion 136, and the second area 1450 is an area disposed at the center of the first area 1440 and the outer periphery of the first area 1440.

The portable air caring apparatus 1 includes the filter portion 60 that serves to collect dust, and the fan module portion 70 is installed at a rear end of the filter portion 60 to generate an air flow. The mesh-type safety portion 1400 is installed on an outer side of the fan base 130 to prevent fingers or the like from coming in contact with the fan member 90 when replacing the filter portion 60. In this way, a contact preventing structure that satisfies safety standards may be provided. [Blocking portion]

In the blocking portion 1410, ring-shaped members having different diameters may be continuously installed about the center of the safety portion 1400 in the radial direction. The blocking portion 1410 is installed at a position that does not face an air flowing area 1445 which is formed between the hub 100, which is disposed at the center of the fan member 90, and the fan base 130. Also, the blocking portion 1410 is a support that extends in a ring shape and may be modified in various ways within the technical spirit in which the blocking portion 1410 is installed between the fan base 130 and the filter portion 60. The blocking portion 1410 according to the second embodiment of the present invention may include at least any one of a central member 1411, a first blocking member 1412, a second blocking member 1413, and an outer side member 1414.

The central member 1411 is disposed at the center of the hub 100 and disposed radially inward from the first blocking member 1412. The central member 1411 disposed at a central portion of the hub 100 may be formed in a ring shape.

The first blocking member 1412 may have an outer diameter less than an outer diameter of the hub 100. The first blocking member 1412 according to the second embodiment of the present invention may have an outer diameter that is greater than an outer diameter of the central member 1411 and less than the outer diameter of the hub 100. The first blocking member 1412 is installed in a ring shape, and the center of the first blocking member 1412 and the center of the central member 1411 are the same. Also, since the first blocking member 1412 and the central member 1411 are installed in the second area 1450 that faces the hub 100, an increase in frictional resistance of air moving toward the suction portion 136 may be minimized.

In a case in which the size of the suction portion 136 is large, the first blocking member 1412 may be installed in the first area 1440 that faces the suction portion 136. The first area 1440 is the air flowing area 1445 and is a path along which air entering the suction portion 136 mainly moves. It is preferable not to install the blocking portion 1410, including the first blocking member 1412, or the safety support 1430 in the first area 1440, but in a case in which a width of the suction portion 136 is wide, the blocking portion 1410 or the safety support 1430 may be installed in the first area 1440.

In a case in which the first blocking member 1412 is installed in the first area 1440, the ring-shaped first blocking member 1412 may be installed at an intermediate position between the hub plate portion 101 and the fan base 130. In a case in which the first blocking member 1412 is installed at the center of the first area 1440, since air passing through the first blocking member 1412 moves toward the suction portion 136 in a state in which streams of air are combined again, a decrease in an air flow speed may be minimized.

The second blocking member 1413 may have an inner diameter greater than an inner diameter of the fan base 130. The second blocking member 1413 according to the second embodiment of the present invention may have an inner diameter that is greater than the outer diameter of the first blocking member 1412 and greater than the inner diameter of the fan base 130. The inner diameter of the second blocking member 1413 is greater than the inner diameter of the bell mouth 132 facing the hollow 137 of the fan base 130. The second blocking member 1413 is installed in a ring shape, and the center of the second blocking member 1413 and the center of the central member 1411 are the same. Also, since the second blocking member 1413 is installed in the second area 1450 facing the bell mouth 132, an increase in frictional resistance of air moving toward the suction portion 136 may be minimized.

The outer side member 1414 is larger than an outer diameter of the second blocking member 1413 and is disposed at the outer periphery of the second blocking member 1413. The outer side member 1414 is installed in a ring shape, and the center of the outer side member 1414 and the center of the central member 1411 are the same. Also, the outer side member 1414 may be fixed to the fan base 130 or fixed to the inside of the housing portion 3300 using a separate fastening device, and as necessary, the outer side member 1414 may be connected to a filter case configured to support the filter portion 60. Since the outer side member 1414 is installed in the second area 1450, which faces the fan base 130, together with the second blocking member 1413, an increase in frictional resistance of air moving toward the suction portion 136 may be minimized.

In the safety portion 1400 facing the suction portion 136 disposed in the fan module portion 70, a cross-sectional area facing the suction portion 136 is minimized, and thus a decrease in an air flow speed may be minimized. To this end, a cross-sectional area of the safety portion 1400 installed in the air flowing area 1445, which is the first area 1440, is minimized. In a case in which the first area 1440 is formed between the inner diameter of the bell mouth 132 and the outer diameter of the hub plate portion 101, the blocking portion 1410 may not be installed in the first area 1440.

However, in a case in which a distance between the inner diameter of the bell mouth 132 and the outer diameter of the hub plate portion 101 is a predetermined distance or more, the blocking portion 1410 may be disposed at the center between the inner diameter of the bell mouth 132 and the outer diameter of the hub plate portion 101. The predetermined distance may be set as 12 mm and may be increased or decreased from 12 mm in consideration of an average finger thickness or the like.

In a case in which the distance between the inner diameter of the bell mouth 132 and the outer diameter of the hub plate portion 101 is within the predetermined distance, the central member 1411 and the first blocking member 1412 are installed at positions facing the hub 100, and the second blocking member 1413 and the outer side member 1414 are installed at positions facing the fan base 130.

### [Safety support]

The safety support 1430 may radially extend about the central member 1411 and may be connected to the first blocking member 1412, the second blocking member 1413, and the outer side member 1414. As the safety support 1430, a plurality of safety supports 1430 may be installed, and the plurality of safety supports 1430 may be disposed to be coplanar.

As described above, the safety portion 1400 may minimize a cross-sectional area of the blocking portion 1410 disposed in the air flowing area 1445 to minimize a decrease in an air flow speed. In a case in which a distance between the inner diameter and outer diameter of the suction portion 136 is small, the blocking portion 1410 is not installed in the first area 1440. Also, in a case in which the distance between the inner diameter and outer diameter of the suction portion 136 is large and is a predetermined distance or more, the blocking portion 1410 is installed at the center of the first area 1440. Therefore, a phenomenon, in which a flow of air passing through both sides of the blocking portion 1410, which is installed in the first area 1440, and moving toward the suction portion 136 occurs unevenly due to the blocking portion 1410, may be minimized, and thus a decrease in the flow rate and flow speed of air flowing to the suction portion 136 may be minimized.

### [Portable air caring apparatus]

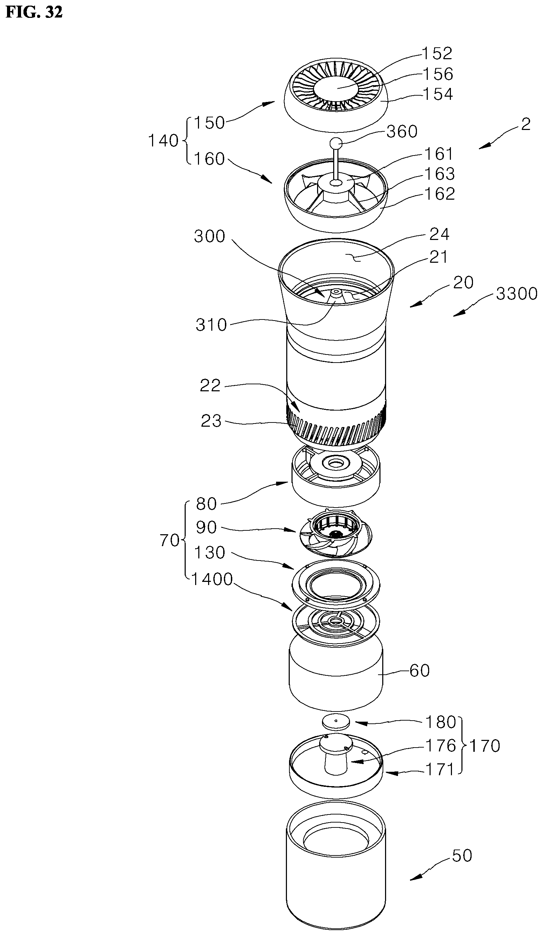

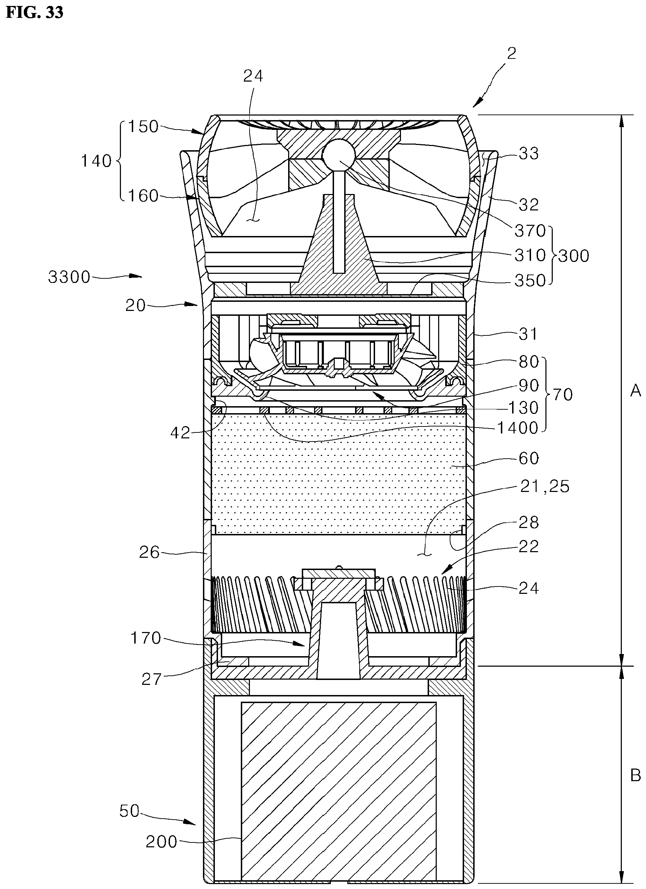

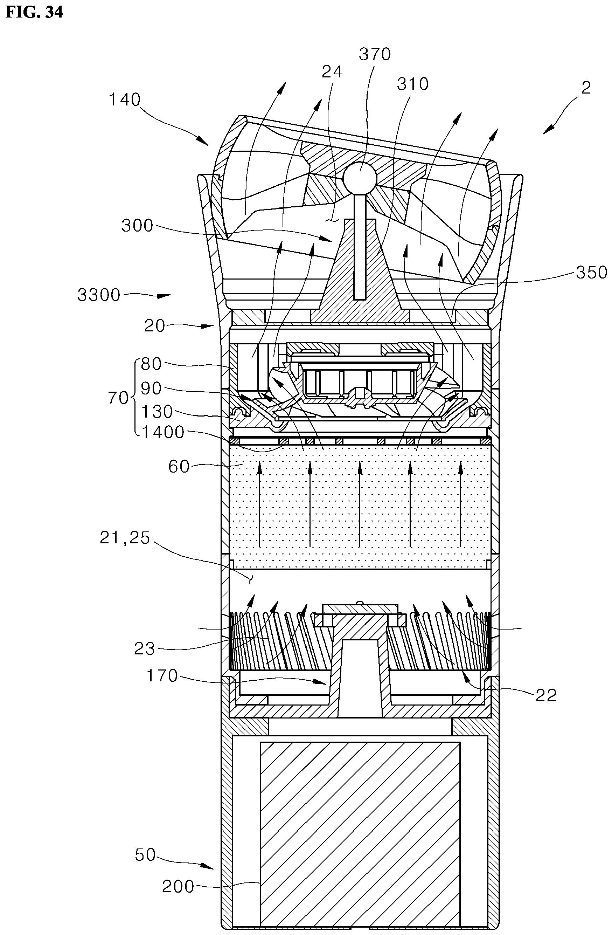

FIG. 32 is an exploded perspective view of the portable air caring apparatus 1 according to the second embodiment of the present invention, FIG. 33 is a cross-sectional view of the portable air caring apparatus 1 according to the second embodiment of the present invention, and FIG. 34 is a view illustrating a flow of air passing through the portable air caring apparatus 1 according to the second embodiment of the present invention.

As illustrated in FIGS. 32 to 34, the portable air caring apparatus 1 according to the second embodiment of the present invention may include at least any one of the housing portion 3300 which includes the inlet portion 22 through which air enters and the outlet 33 through which air is discharged and has an air flow path 25 formed therein, the fan housing 80 which is fixed to the inside of the housing portion 3300 and has an operating space formed therein, the fan member 90 which is rotatably installed in the fan housing 80, the fan base 130 which is configured to guide air to enter in a direction toward the fan member 90, the filter portion 60 which is disposed between the fan base 130 and the inlet portion 22 and configured to purify air entering through the inlet portion 22, and the safety portion 1400 which includes the blocking portion 1410, which is configured to block movement of an external object toward an inner side of the suction portion 136 disposed in the fan base 130, and the safety support 1430, which is configured to support the blocking portion 1410. Also, the portable air caring apparatus 1 may include at least any one of the discharge portion 140, the sanitizing portion 170, the battery 200, and the rotation support portion 300.

### [Housing portion]

The housing portion 3300 according to the second embodiment of the present invention includes the first case portion 10 and the second case portion 50. The first case portion 10 and the second case portion 50 form the framework of the exterior of the portable air caring apparatus 1. The inside of the housing portion 3300 forms the air flow path 25 in a cylindrical shape and guides movement of air in a direction from a lower side toward an upper side. Also, the fan module portion 70, whose edge is formed in a circular shape, is mounted in the housing portion 3300. Therefore, since formation of a gap between the fan module portion 70 and the housing portion 3300 is prevented, movement of air is blocked between the fan module portion 70 and the housing portion 3300.

Also, air entering through the lower portion of the housing portion 3300 may be discharged to an upper side of the housing portion 3300 through the fan module portion 70 and may easily reach a user located above the portable air caring apparatus 1.

The exterior of the side surface and bottom surface of the portable air caring apparatus 1 are formed by the first case portion 10 and the second case portion 50. An accommodation space 21 is formed inside the first case portion 10 and the second case portion 50. The accommodation space 21 accommodates the filter portion 60, the fan module portion 70, the sanitizing portion 170, the rotation support portion 300, and electronic components including the battery 200. Preferably, the first case portion 10 and the second case portion 50 are formed to have a sufficient strength to protect the accommodated components from external impact.

### [Filter portion]

The filter portion 60 is installed in the accommodation space 21 of the first case portion 10 and is disposed between the fan module portion 70 and the inlet portion 22. That is, the filter portion 60 is disposed at a lower portion of the fan module portion 70 and serves to purify air that is suctioned through the inlet portion 22 of the portable air caring apparatus 1. The air purified while passing through the filter portion 60 passes through the fan module portion 70 and the discharge portion 140 and is discharged to an upper portion of the portable air caring apparatus 1.

The filter portion 60 is installed inside the first case portion 10 and purifies air that enters through the inlet portion 22. Also, the filter portion 60 may be formed in a cylindrical shape extending in the vertical direction.

The filter portion 60 made be made of a single filter or, as necessary, installed in a state in which a plurality of filters are stacked. Also, the filter portion 60 may further include a separate filter case for fixing the filter.

The filter case is fixed to the inside of the first case portion 10, and an insertion space for accommodating the filter may be formed inside the filter case.

The fan module portion 70 is accommodated in the accommodation space 21 inside the first case portion 10 and may be disposed between the discharge portion 140 and the filter portion 60. More specifically, the fan module portion 70 may be disposed between the outlet 33 and the filter portion 60. That is, the fan module portion 70 is disposed at an upper portion of the filter portion 60, and the outlet 33, the rotation support portion 300, and the discharge portion 140 are disposed at an upper portion of the fan module portion 70. The fan module portion 70 serves to suction air, which enters a lower portion of the filter portion 60 through the inlet portion 22, and discharge the air to an upper portion of the first case portion 10.

The safety portion 1400 disposed at the fan module portion 70 may be fixed to the filter case or integrally formed with the filter case. In this way, various modifications are possible.

### [Discharge portion]

The center of rotation of the discharge portion 140 may coincide with the center of the fan module portion 70 in the vertical direction. Air entering through the inlet portion 22 moves upward, sequentially passes through the filter portion 60, the fan module portion 70, and the discharge portion 140, and is discharged to an upper side of the portable air caring apparatus 1.

The fan module portion 70 may suction air, which has passed through the filter portion 60, in the axial direction and may discharge the air in a direction between the axial direction and radial direction.

The discharge portion 140 may be rotatably installed at the upper side of the first case portion 10 and may guide a discharge direction of air that has moved upward through the outlet 33. The rotation support portion 300 is disposed at the upper portion of the first case portion 10, and the discharge portion 140 is rotatably installed on the rotation support portion 300. Since both sides of the discharge portion 140 in the vertical direction are open, air that has moved to a lower portion of the discharge portion 140 through the outlet 33 may be discharged to the outside of the portable air caring apparatus 1 through an upper portion of the discharge portion 140.

The sanitizing portion 170 is disposed at the lower portion of the filter portion 60 and may be fixed to at least any one of the first case portion 10 and the second case portion 50. The sanitizing portion 170 is spaced a predetermined distance apart from the filter portion 60 and irradiates the filter portion 60 with the sanitizing light. Since the sanitizing light irradiated by the sanitizing portion 170 is harmful to the human body, the installation position of the sanitizing portion 170 is set such that the sanitizing light does not leak to the outside of the portable air caring apparatus 1 through the inlet portion 22.

The battery 200 is installed in the accommodation space 21 provided inside the second case portion 50 and is disposed at a lower portion of the sanitizing portion 170. The battery 200 may supply power for driving the portable air caring apparatus 1.

The housing portion 3300 includes the inlet portion 22, and the filter portion 60, the sanitizing portion 170, and the fan module portion 70 may be disposed in the housing portion 3300. Also, the housing portion 3300 forms an air flow path in the vertical direction. Since the cylindrical air flow path 25 is formed in the housing portion 3300, frictional resistance of air moving in the vertical direction may be reduced.

Also, along a vertical reference line that passes through the center of the housing portion 3300 in the vertical direction, the center of the inlet portion 22, the center of the filter portion 60, the center of the sanitizing portion 170, the center of the fan module portion 70, the center of the outlet 33, and the center of the rotation support portion 300 may coincide in the vertical direction. Therefore, a flow of air, which moves from a lower side to an upper side along the housing portion 3300, moves in a straight line in the perpendicular direction, and a movement path of air is shortened. Thus, resistance of an air flow path is decreased, and air caring efficiency is improved.

In a case in which the portable air caring apparatus 1 is installed on a horizontal surface, the vertical reference line coincides with a vertical line. Also, the housing portion 3300 may consist of a single member, but preferably, the housing portion 3300 may consist of a plurality of members.

The portable air caring apparatus 1 may be formed in a cylindrical shape that stands upright and is long in the vertical direction as a whole. Accordingly, a user may use the portable air caring apparatus 1 in a vertical state or a horizontal state. Also, in a place such as the inside of a vehicle where shaking of the portable air caring apparatus 1 may occur, since the portable air caring apparatus 1 is used in a state of being mounted in a groove portion such as a cup holder that is concave toward the lower side, the position of the portable air caring apparatus 1 may be stably maintained.

### [Detailed configuration of components of portable air caring apparatus]

The portable air caring apparatus 1 according to the second embodiment of the present invention may include the housing portion 3300, the filter portion 60, and the fan module portion 70. Also, the portable air caring apparatus 1 according to the second embodiment of the present invention may further include the discharge portion 140, the rotation support portion 300, the sanitizing portion 170, and the battery 200.

### [First case portion]

The housing portion 3300 includes the first case portion 10 and the second case portion 50, the first case portion 10 has the accommodation space 21 formed therein, and the inlet portion 22 configured to suction air may be disposed at a side surface of a lower portion of the first case portion 10. The first case portion 10 is formed in a cylindrical shape and may be installed in a shape in which the upper and lower sides thereof are open. The first case portion 10 may consist of a single member or may consist of a plurality of members as necessary. The first case portion 10 according to the second embodiment of the present invention may consist of a plurality of members, and each member may be coupled by fitting or coupled using an adhesive or by welding, or the members may be connected to each other using a fastening member such as a bolt. In this way, various modifications are possible.

Meanwhile, air is suctioned through the lower side surface of the first case portion 10, and air is discharged through the upper side of the first case portion 10. To this end, the inlet portion 22 including inlet holes 23 is installed along a periphery of a lower portion of the first case portion 10. Also, the outlet 33 configured to discharge air may be disposed at the upper side of the first case portion 10.

Also, in a state in which the inlet portion 22 configured to suction air is installed along the outer periphery of the first case portion 10, since the filter portion 60 is installed at an upper side that is spaced apart from the inlet portion 22, air may move evenly throughout the entire area of the filter portion 60.

Meanwhile, the plurality of inlet holes 23 are formed in the inlet portion 22, and the inlet holes 23 may be installed to be inclined in a diagonal shape or, as necessary, may be formed as holes each having the shape of an inequality sign in which the line is broken at the center. Also, in order to increase a flow rate of air entering the filter portion 60, the inlet holes 23 may be additionally formed in a side surface of the housing portion 3300 in which the filter portion 60 is installed. In this way, various modifications are possible.

Meanwhile, the housing portion 3300 may consist of three or more members. In this way, the housing portion 3300 may be modified to have various other shapes.

### [Second case portion]

The second case portion 50 is connected to the lower portion of the first case portion 10 and may be modified in various ways in which the second case portion 50 has a space formed therein to install electronic components including the battery 200.

At least any one of the first case portion 10 and the second case portion 50 may be made of a cylindrical case. Both the first case portion 10 and the second case portion 50 may be formed in a cylindrical shape, or only the second case portion 50 may be formed in a cylindrical shape. Alternatively, as necessary, only the first case portion 10 may be formed in a cylindrical shape.

In a case in which the second case portion 50 is formed in a cylindrical shape and extends in the vertical direction, it is convenient for a user to hold the outer periphery of the second case portion 50 with his or her hand, and the second case portion 50 may also be easily mounted on a cup holder of a vehicle that includes a groove portion having a substantially circular cross-section.

Also, in a case in which the first case portion 10 is formed in a cylindrical shape, friction, which is generated when air passing through the first case portion 10 and moving upward comes in contact with the inside of the first case portion 10 formed in a curved shape, may be reduced such that a flow of air is facilitated.

Meanwhile, since the air flow path 25 is formed inside the first case portion 10 while the air flow path 25 is not formed inside the second case portion 50, suction and discharge of air through the first case portion 10 may be smoothly performed even when the second case portion 50 is inserted into a cup holder or held by a user's hand. Thus, convenience in use may be improved.

### [Filter portion]

The filter portion 60 is installed inside the first case portion 10 and may be modified in various ways in which the filter portion 60 purifies air that enters through the inlet portion 22. The filter portion 60 according to the second embodiment of the present invention may be formed in a cylindrical shape.

Since the first case portion 10 is formed in the shape of a circular pipe and the filter portion 60, which is installed in the first case portion 10, is also formed in a cylindrical shape that comes in contact with the inside of the first case portion 10, impurities may be effectively removed from air passing through the first case portion 10.

Also, a transverse cross-section of the filter portion 60 is formed in a circular shape such that the filter portion 60 has the largest area in the first case portion 10. Also, when the filter portion 60 is manufactured in the form of a cylinder and an upper end and a lower end of a material constituting the filter portion 60 are cut, pressure loss may be minimized, and thus performance of the filter portion 60 may be maximized.

Also, since an outer diameter of the filter portion 60 is formed to be greater than or equal to a suction diameter of the bell mouth 132 through which air is suctioned into the fan module portion 70, the volume of the filter portion 60 may be maximized.

Also, according to the present embodiment, the filter portion 60, the fan module portion 70, and the discharge portion 140 may be arranged in the vertical direction along the housing portion 3300, and an air flow may also occur in the vertical direction. That is, an air flow that occurs due to the operation of the fan module portion 70 may occur in a linear direction which is the same as the direction in which the filter portion 60, the fan module portion 70, and the discharge portion 140 are arranged.

When the air flow occurs in the linear direction as described above, resistance related to the air flow is lowered correspondingly, and thus the air flow may occur more smoothly. In this way, since suctioning a sufficient amount of air and discharging a sufficient amount of air, which corresponds to the amount of suctioned air, may be performed by the fan module portion 70, air caring performance of the portable air caring apparatus 1 may be improved correspondingly.

The safety portion 1400 is installed between the filter portion 60 and the fan base 130. The safety portion 1400 may be fixed to the inside of the first case portion 10 or may be connected to the filter case in which the filter portion 60 is stored. Alternatively, the safety portion 1400 may be fixed to the fan base 130 through a separate fastening device. In this way, various modifications are possible.

### [Discharge portion]

The discharge portion 140 is disposed at the outlet 33 of the housing portion 3300 and may be modified in various ways in which the discharge portion 140 is rotatably installed at the rotation support portion 300 and guides a discharge direction of air that has passed through the fan module portion 70. The discharge portion 140 according to the present invention may smoothly rotate due to being rotatably installed on a spherical ball joint 370 disposed in the rotation support portion 300.

Since the discharge portion 140 installed at an upper side of the housing portion 3300 is open in the vertical direction and rotatably connected to the rotation support portion 300, the discharge portion 140 may control the discharge direction of air which has passed through the fan module portion 70. The discharge portion 140 according to the second embodiment of the present invention may include a first discharge portion 150 and a second discharge portion 160.

### [First discharge portion]

The first discharge portion 150 is disposed at one side of the ball joint 370 and may be modified in various ways in which the first discharge portion 150 includes a plurality of vanes 156 configured to guide discharge of air. The first discharge portion 150 according to the second embodiment of the present invention may include a first discharge core 152, a first discharge body 154, and the vanes 156.

The first discharge core 152 is disposed at an upper side of the ball joint 370 and may be formed in various shapes including a disk shape. Also, the first discharge body 154 is spaced apart from the first discharge core 152 and may be installed in an annular shape that surrounds an outer side of the first discharge core 152. Also, since an outer side of the first discharge body 154 is formed in a curved shape and installed in a state of being spaced apart from the housing portion 3300, contact of the first discharge portion 150 with the housing portion 3300 may be prevented during rotation of the first discharge portion 150.

Also, since the first discharge core 152 and the first discharge body 154 are connected to each other by the plurality of vanes 156, the first discharge core 152, the first discharge body 154, and the vanes 156 may rotate together.

### [Second discharge portion]

The second discharge portion 160 is disposed at the other side of the ball joint 370 and may be modified in various ways in which the second discharge portion 160 is connected to the first discharge portion 150 and rotates about the ball joint 370 along with the first discharge portion 150. The second discharge portion 160 according to the second embodiment of the present invention may include a second discharge core 161, a second discharge body 162, and discharge supports 163.

The second discharge core 161 is installed in a shape that surrounds the spherical lower side of the ball joint 370 and is disposed at a lower side of the first discharge core 152. Also, the second discharge body 162 is installed in an annular shape that surrounds an outer side of the second discharge core 161, and an outer side of the second discharge body 162 is formed in a curved shape.

Also, since the second discharge core 161 and the second discharge body 162 are connected to each other by the plurality of discharge supports 163, the second discharge core 161, the second discharge body 162, and the discharge supports 163 may rotate together.

### [Sanitizing portion]

The sanitizing portion 170 is disposed between the filter portion 60 and the second case portion 50 and may be modified in various ways in which the sanitizing portion 170 irradiates the sanitizing light toward the filter portion 60. The sanitizing portion 170 according to the second embodiment of the present invention may include at least any one of a sanitization support portion 171, a pedestal portion 176, and an irradiating portion 180.

### [Sanitization support portion]

The sanitization support portion 171 is disposed between the first case portion 10 and the second case portion 50 and blocks the lower portion of the first case portion 10. Also, the sanitization support portion 171 is disposed at a lower side of the irradiating portion 180 and may be modified in various ways in which the sanitization support portion 171 is connected to the housing portion 3300 such that movement of the sanitization support portion 171 is restricted.

Movement of air, which enters the first case portion 10 through the inlet portion 22, toward the second case portion 50 is blocked by the sanitization support portion 171, and thus a flow rate of air moving toward the fan module portion 70 may be increased, and air caring performance of the portable air caring apparatus 1 may be improved.

The pedestal portion 176 may be modified in various ways in which the pedestal portion 176 protrudes upward from the center of the sanitization support portion 171 to support the lower portion of the irradiating portion 180. Also, the pedestal portion 176 is disposed at the center of the inlet portion 22 in the radial direction, and a transverse cross-section of the pedestal portion 176 may be formed in a circular shape to reduce friction with air.

The pedestal portion 176 may be formed in the shape of a column that protrudes upward from the center of the sanitization support portion 171. Also, the pedestal portion 176 may be formed in a cylindrical or conical shape. The pedestal portion 176 according to the second embodiment of the present invention is formed in a shape whose transverse cross-section gradually narrows from a lower side toward an upper side, and is disposed at the center of the first case portion 10 in which the inlet portion 22 is formed. Thus, friction with air may be minimized.

A transverse cross-section of the sanitization support portion 171, which is installed to sanitize the filter portion 60, has a circular shape, and air that enters through the inlet portion 22 rotates in a spiral due to the inclined shape of the inlet holes 23, rotates along an outer side of a pedestal column 177, and moves to an upper side where the filter portion 60 is installed. That is, since the sanitizing portion 170 is disposed at a central portion of the first case portion 10 and air, which enters through the inlet portion 22, moves upward while rotating along the outer periphery of the sanitizing portion 170, resistance of a flow path of the sanitizing portion 170 is decreased.

Since the pedestal portion 176, the center of rotation of the fan member 90, and a core member 310 of the rotation support portion 300, which will be described below, are disposed in a straight line in the perpendicular direction, resistance related to a flow of air moving from a lower side toward an upper side is decreased, and thus the air flow may occur more smoothly. As a result, air caring performance of the portable air caring apparatus 1 is improved.

The irradiating portion 180 is mounted on an upper side of the pedestal portion 176 and may irradiate the sanitizing light in a direction toward the filter portion 60. Also, the irradiating portion 180 may be disposed on a vertical reference line that passes through the radial center of the inlet portion 22 in the vertical direction. Therefore, in a case in which the filter portion 60 is disposed at an upper side of the irradiating portion 180, the entire area of a lower end of the filter portion 60 may be sanitized by a relatively small number of sanitizing light sources 182, and thus production costs and maintenance and repair costs may be reduced.

Also, the irradiating portion 180 may be modified in various ways in which the irradiating portion 180 is installed at a position that is level with or higher than an upper end of the inlet portion 22. The irradiating portion 180 according to the second embodiment of the present invention may include a printed circuit board (PCB) 181 and the sanitizing light source 182. The PCB 181 is installed on the upper side of the pedestal plate 178, and the sanitizing light source 182 configured to irradiate the sanitizing light is installed on an upper side of the PCB 181. The sanitizing light source 182 may be a UVC LED, or various other types of sanitizing apparatuses may be used as the sanitizing light source 182 in which the sanitizing light source 182 sterilizes the filter portion 60.

Meanwhile, since the sanitizing light source 182 of the sanitizing portion 170 is disposed at the upper side of the inlet portion 22, the sanitizing light source 182 may be prevented from irradiating the sanitizing light to the outside of the first case portion 10 through the inlet portion 22.

### [Rotation support portion]

The rotation support portion 300 is connected to at least any one of a second case body 31 and a tubular expansion member 32 and may be modified in various ways in which the rotation support portion 300 rotatably supports the discharge portion 140. Also, the rotation support portion 300 may rotatably support the discharge portion 140 at the center of the outlet 33 disposed at the inner side of the tubular expansion member 32. The rotation support portion 300 according to the second embodiment of the present invention may include at least any one of the core member 310, the core support portion 350, and the ball joint 370.

The core member 310 is disposed at a lower side of the discharge portion 140 configured to control a discharge direction of air and may extend from the center of the outlet 33 in a direction toward the discharge portion 140. The core member 310 is disposed at the center of a second case 30 in the radial direction.

A transverse cross-section of a lower portion of the core member 310 is formed in a circular shape and coincides with a central portion of the fan module portion 70 which will be described below. That is, the support plate 81, which is disposed at the center of the fan module portion 70 in the radial direction, is disposed at the lower portion of the core member 310. Therefore, air that moves upward through the outer periphery of the support plate 81 moves upward along an outer side of the core member 310 and moves into the discharge portion 140. Thus, resistance of an air flow path is decreased, and air cleaning efficiency is improved.

Since a lower surface of the core member 310 that faces the support plate 81 of the fan module portion 70 has an area that is less than or equal to an area of the support plate 81, an increase in resistance of a flow path due to air, which has passed through the fan module portion 70, coming in contact with the lower portion of the core member 310 may be prevented. A cross-sectional area of a lower end portion of the core member 310 is less than or equal to a cross-sectional area of the support plate 81, and the core member 310 is disposed at an upper side of the support plate 81 in the first direction.

Also, since the core member 310 is formed in a conical shape toward an upper side, an area of the core member 310 that comes in contact with air moving outside the core member 310 is minimized. Thus, friction with air may be reduced, and air cleaning efficiency may be improved.

Since the core support portion 350 extends to the outside of the core member 310 and is connected to the housing portion 3300, movement of the core support portion 350 and the core member 310 is restricted.

Also, the core support portion 350 according to the second embodiment of the present invention may be disposed at an upper side of the connection support 82 of the fan module portion 70 which will be described below. For example, in a case in which, as the connection support 82, four connection supports 82 are installed at 90° intervals about the support plate 81, the core support portion 350 is also provided as four core support portions 350 installed at 90° intervals about the core member 310. The connection support 82 is disposed at a lower side of the core support portion 350, and when viewed from the upper side of the core support portion 350, the core support portion 350 and the connection support 82 are installed to overlap each other.

Therefore, air, which passes through an outer side of the connection support 82 and moves upward, passes through an outer side of the core support portion 350 disposed at the upper side of the connection support 82. Thus, friction that occurs during movement of air may be minimized, and air cleaning efficiency may be improved.

The core support portion 350 according to another embodiment of the present invention may also be provided as four or more core support portions 350 radially installed about the core member 310. Also, in a state in which the core support portion 350 is connected to a separate ring-shaped edge member, the edge member may be fixed to the housing portion 3300. In this way, various modifications are possible.

The ball joint 370 is coupled to the core member 310 and may be modified in various ways in which the ball joint 370 rotatably supports the discharge portion 140. The ball joint 370 according to the second embodiment of the present invention has a spherical end portion, and the end portion may be coupled to an inner side of the discharge portion 140 to rotatably support the discharge portion 140. An upper side of the ball joint 370 has a spherical shape, and a bar-shaped body of the ball joint 370 that extends downward from the spherical upper side is inserted into and fixed to the core member 310 through a hole formed in an upper side of the core member 310. The ball joint 370 and the core member 310 may be fixed using various fixing methods such as screw fastening, pin fastening, and using an adhesive.

### [Air flow of portable air caring apparatus]

Since the inlet portion 22 configured to suction outside air is installed along the outer periphery of the first case portion 10, air outside the first case portion 10 may move into the first case portion 10 through the inlet portion 22, and thus a suction flow rate of air may increase.

Due to the operation of the fan module portion 70, air outside the portable air caring apparatus 1 enters the portable air caring apparatus 1. Here, the air outside the portable air caring apparatus 1 passes through the inlet holes 23, which are installed in an inclined shape, and forms a spiral air flow that rotates along the outer periphery of the sanitization support portion 171.

Air, which enters a first case 20 and moves upward while rotating in a spiral, passes through the filter portion 60, and in this process, physical particles such as dust, fine dust, and ultrafine dust, chemical substances such as odorous particles and harmful gases, and microorganisms such as bacteria and viruses that are contained in the air may be filtered.

Since the filter portion 60 and the fan module portion 70 are disposed in a straight line in the vertical direction, suctioning and filtering of air may be effectively performed while flow loss of air is minimized.

Air that has passed through the filter portion 60, that is, purified air, passes through the safety portion 1400, which is disposed at a lower portion of the fan module portion 70, and moves to the suction portion 136. Here, since a cross-sectional area of the safety portion 1400 that is disposed in the air flowing area 1445 is minimized, a decrease in a flow speed of air passing through the filter portion 60 and moving to the suction portion 136 may be minimized.

Also, since an object that moves from the filter portion 60 to the suction portion 136 is caught on the blocking portion 1410 and the safety support 1430 which are included in the safety portion 1400, damage to the fan member 90 may be prevented, and occurrence of accidents due to fingers being caught on the fan member 90 may also be prevented.

Meanwhile, movement of air toward the suction portion 136 may be guided by the bell mouth 132, and in this way, air may be guided to smoothly enter the fan module portion 70. The air entering the fan module portion 70 is discharged to the upper side of the fan module portion 70. The air discharged to the upper side of the fan module portion 70 may be discharged in a diagonal flow direction. Here, the diagonal flow direction may be defined as an upward diagonal direction.

Air suctioned into a central portion of the lower side of the fan module portion 70 is moved in an upward, inclined direction through a space between the hub 100, at which the fan blade 110 is disposed, and the shroud 120. Also, air that is moved upward along the side support portion 84 installed in the vertical direction has linearity in an upward direction.

The bell mouth 132 is installed in order to prevent a return air phenomenon in which a portion of air, which has moved to the rear of the fan member 90 due to rotation of the fan member 90, returns to an inlet of the fan member 90 through a space between the fan member 90 and the fan base 130. The bell mouth 132 surrounds the inlet protrusion 121 of the shroud 120 in a hemispherical shape and is installed to be spaced a predetermined distance apart from the inlet protrusion 121.

Also, the second inclined surface 135 of the protruding rib 133 and the shroud 120 are installed to be parallel to each other, and the distance between the second inclined surface 135 and the shroud 120 is formed to be small as possible to prevent the return air phenomenon.

Also, the first inclined surface 88 formed at the upper side of the inner side guiding portion 85 is installed to be inclined at the same angle as the second inclined surface 135 and connects the side support portion 84 and the protruding rib 133 to each other.

Therefore, since a path formed by the first inclined surface 88, the second inclined surface 135, and the shroud 120 is smaller than the space formed between the fan member 90 and the side support portion 84, the air that is moved to the rear of the fan member 90 due to rotation of the fan member 90 is discharged to the upper side of the fan member 90 without the return air phenomenon.

Since a path between the bell mouth 132 and the inlet protrusion 121 is also formed to be narrow, even when a portion of air enters between the second inclined surface 135 and the shroud 120, a phenomenon in which the air returns to the space between the bell mouth 132 and the inlet protrusion 121 may be prevented.

Meanwhile, since the first inclined surface 88 extends to be inclined toward the side support portion 84, air moving toward the upper side of the fan housing 80 through the fan member 90 is guided to move in an upward, inclined direction along the first inclined surface 88 and then move upward along the side support portion 84. Thus, the return air phenomenon due to air moving to the lower side of the first inclined surface 88 may be prevented.

Meanwhile, air discharged to the upper side of the fan module portion 70 enters the discharge portion 140 through a lower side thereof and is discharged to the upper side of the discharge portion 140. Since the discharge portion 140 rotates within a predetermined angle range, a direction in which air is discharged may be controlled according to an angle at which the discharge portion 140 is installed.

Also, since the inner side of the discharge portion 140 forms a concave groove portion, an increase in discharge resistance of air, whose direction is switched through the discharge portion 140, may be reduced. Also, since the filter portion 60, the fan module portion 70, and the discharge portion 140 are disposed in a straight line in the vertical direction, suctioning and filtering of air and discharging of purified air may be effectively performed while flow loss of air is minimized.

Since a diagonal flow fan is used as the fan module portion 70 according to the present invention, air blowing performance may be maximized when a discharge direction of air is the axial direction under a fixed pressure condition. Also, since the fan base 130 maintains a predetermined distance from the fan member 90 and provides a return air preventing structure, and the return air preventing structure extends to an inner wall surface of the fan housing 80, flow loss of air due to returning air may be minimized.

Also, as compared to other fan modules using a fan of the same diameter, the fan module portion 70 is customized according to the inside of the cylindrical housing portion 3300 and installed therein, and thus a size increase due to a fastening portion does not occur. Accordingly, it is possible to reduce the size of the diagonal flow fan module and the portable air caring apparatus including the same.

Also, since the safety portion 1400 is continuously installed on the fan base 130 and a protective mesh is formed on an outer side of the suction portion 136, occurrence of accidents may be prevented, and the filter portion 60 or the like may be blocked from entering the fan member 90 to improve operational reliability.

Also, according to the present invention, since the blocking portion 1410 is not installed at a position facing the suction portion 136, an area coming in contact with air moving toward the suction portion 136 is reduced, and thus an air blowing function may be improved.

### [Third embodiment - Exterior of portable air caring apparatus]

Hereinafter, a portable air caring apparatus according to a third embodiment of the present invention will be described with reference to the drawings.

For convenience of description, elements which have the same configuration and effect as in the first embodiment of the present invention will be denoted by the same reference numerals as in the first embodiment and may be described using the same drawings as in the first embodiment. As necessary, description of such elements may be omitted.

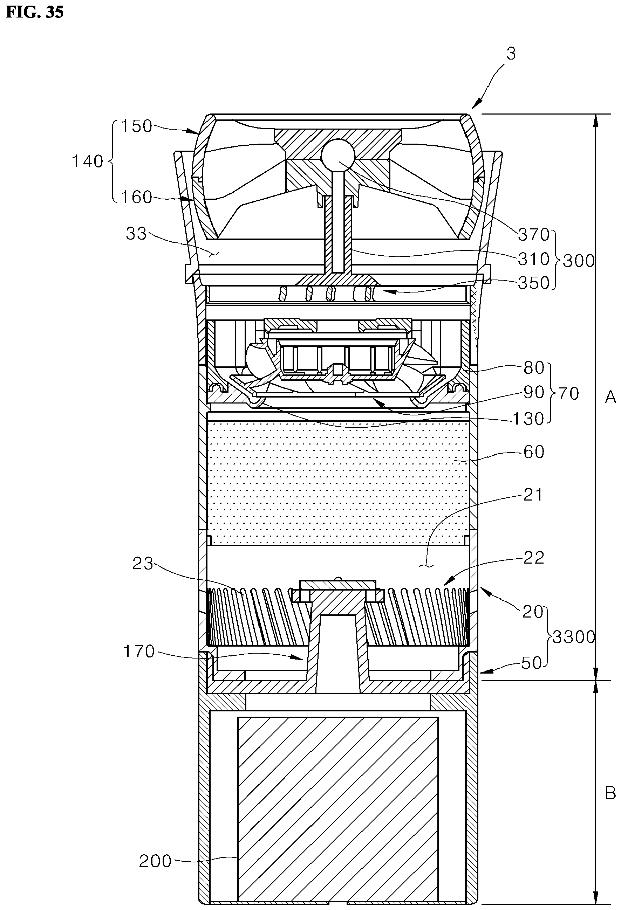

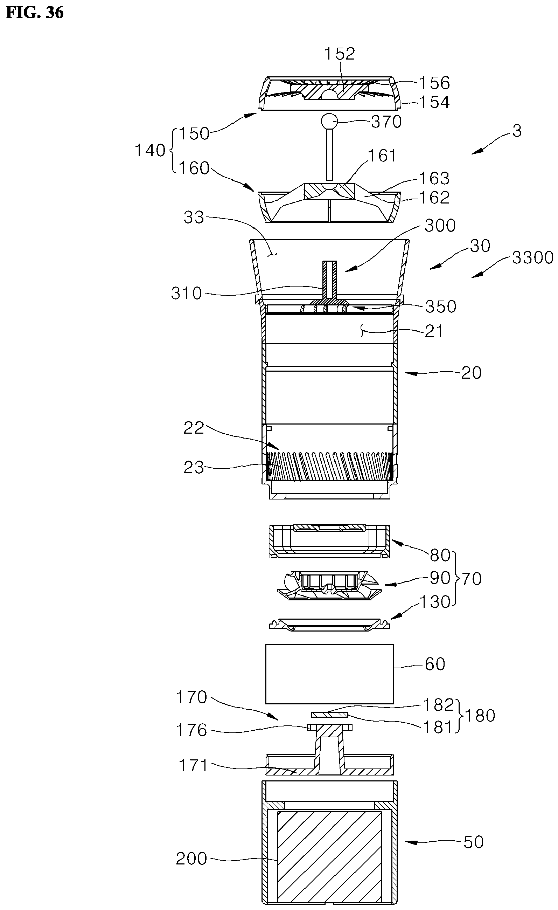

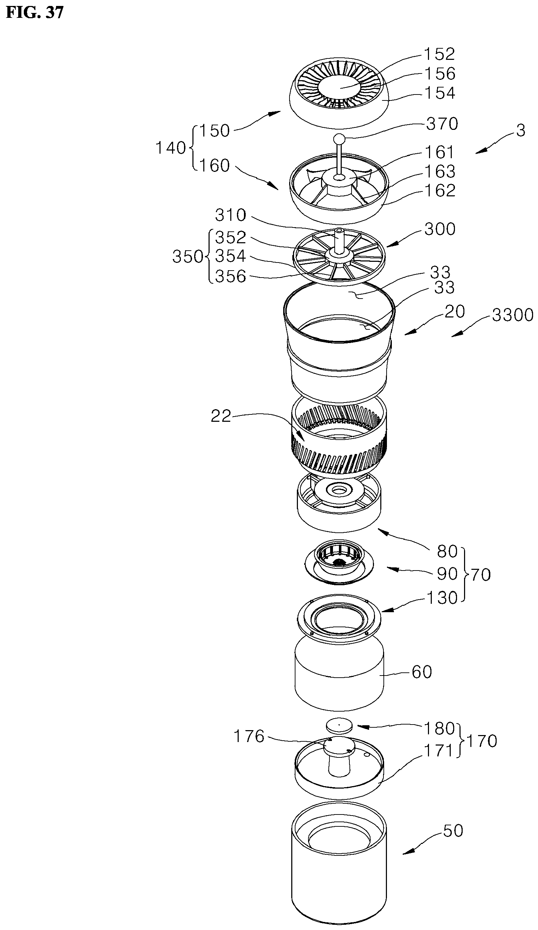

FIG. 35 is a cross-sectional view of a portable air caring apparatus 1 according to the third embodiment of the present invention, FIG. 36 is an exploded cross-sectional view of the portable air caring apparatus 1 according to the third embodiment of the present invention, and FIG. 37 is an exploded perspective view of the portable air caring apparatus 1 according to the third embodiment of the present invention.

As illustrated in FIGS. 35 to 37, the portable air caring apparatus 1 according to the third embodiment of the present invention may be formed in a substantially cylindrical shape. The portable air caring apparatus 1 may include at least any one of a housing portion 3300, a filter portion 60, a fan module portion 70, a discharge portion 140, a sanitizing portion 170, and a rotation support portion 300.

The housing portion 3300 may include an inlet portion 22, and the filter portion 60, the sanitizing portion 170, and the fan module portion 70 may be disposed in the housing portion 3300. Also, the housing portion 3300 forms an air flow path in a vertical direction. Since a cylindrical air flow path is formed inside the housing portion 3300, frictional resistance of air moving in the vertical direction may be reduced.

Also, along a rotation axis extension line E that passes through the center of the housing portion 3300 in the vertical direction, the center of the inlet portion 22, the center of the filter portion 60, the center of the sanitizing portion 170, the center of the fan module portion 70, the center of an outlet 33, and the center of a core member 310 disposed at the rotation support portion 300 may coincide in the vertical direction. Therefore, a flow of air, which moves from a lower side to an upper side along the housing portion 3300, moves in a straight line in the vertical direction, and a movement path of air is shortened. Thus, resistance of an air flow path is decreased, and air caring efficiency is improved.

The rotation axis extension line E is the same as the center of rotation of a fan member, which is disposed in the fan module portion 70, and coincides with a vertical reference line that passes through the center of the housing portion 3300 and extends in the vertical direction.

In a case in which the portable air caring apparatus 1 is installed on a horizontal surface, the vertical reference line coincides with a vertical line. Also, the housing portion 3300 may consist of a single member, but preferably, the housing portion 3300 may consist of a plurality of members.

The portable air caring apparatus 1 may be formed in a cylindrical shape that stands upright and is long in the vertical direction as a whole. Accordingly, a user may use the portable air caring apparatus 1 in a vertical state or a horizontal state. Also, in a place such as the inside of a vehicle where shaking of the portable air caring apparatus 1 may occur, since the portable air caring apparatus 1 is used in a state of being mounted in a groove portion such as a cup holder that is concave toward the lower side, the position of the portable air caring apparatus 1 may be stably maintained.

Directions will be defined. When a direction in which the discharge portion 140 is located from a first case portion 10 is referred to as "upper portion" and a direction in which a second case portion 50 is located from the first case portion 10 is referred to as "lower portion," "first direction" refers to a vertical direction or an axial direction. The first direction may also refer to a perpendicular direction. Also, "second direction" is a direction perpendicular to the first direction and refers to a lateral direction, a horizontal direction, or a radial direction.

### [Overall structure of portable air caring apparatus]

The portable air caring apparatus 1 according to the present embodiment may include the housing portion 3300, the filter portion 60, the fan module portion 70, and the discharge portion 140. Also, the portable air caring apparatus 1 may further include the sanitizing portion 170 and a battery 240.

The housing portion 3300 includes the first case portion 10 and the second case portion 50. The first case portion 10 and the second case portion 50 form the framework of the exterior of the portable air caring apparatus 1. The exterior of a side surface and a bottom surface of the portable air caring apparatus 1 are formed by the first case portion 10 and the second case portion 50. An accommodation space 21 is formed inside the first case portion 10 and the second case portion 50. The accommodation space 21 accommodates the filter portion 60, the fan module portion 70, the sanitizing portion 170, the rotation support portion 300, and electronic components including the battery 240. Preferably, the first case portion 10 and the second case portion 50 are formed to have a sufficient strength to protect the accommodated components from external impact.

The filter portion 60 is installed in the accommodation space 21 of the first case portion 10 and is disposed between the fan module portion 70 and the inlet portion 22. That is, the filter portion 60 is disposed at a lower portion of the fan module portion 70 and serves to purify air that is suctioned through the inlet portion 22 of the portable air caring apparatus 1. The air purified while passing through the filter portion 60 passes through the fan module portion 70 and the discharge portion 140 and is discharged to an upper portion of the portable air caring apparatus 1.

The filter portion 60 is installed inside the first case portion 10 and purifies air that enters through the inlet portion 22. Also, the filter portion 60 may be formed in a cylindrical shape extending in the vertical direction.

The filter portion 60 made be made of a single filter or, as necessary, installed in a state in which a plurality of filters are stacked. Also, the filter portion 60 may further include a separate filter case (not illustrated) for fixing the filter.

The filter case is fixed to the inside of the first case portion 10, and an insertion space for accommodating the filter may be formed inside the filter case.

The fan module portion 70 is accommodated in the accommodation space 21 inside the first case portion 10 and may be disposed between the discharge portion 140 and the filter portion 60. More specifically, the fan module portion 70 may be disposed between the outlet 33 and the filter portion 60. That is, the fan module portion 70 is disposed at an upper portion of the filter portion 60, and the outlet 33, the rotation support portion 300, a rotation guide portion 400, and the discharge portion 140 are disposed at an upper portion of the fan module portion 70. The fan module portion 70 serves to suction air, which enters a lower portion of the filter portion 60 through the inlet portion 22, and discharge the air to an upper portion of the first case portion 10.

The center of rotation of the discharge portion 140 may coincide with the center of the fan module portion 70 in the vertical direction. The air that enters through the inlet portion 22 moves upward, sequentially passes through the filter portion 60, the fan module portion 70, guide vanes 356, and the discharge portion 140, and is discharged to an upper side of the portable air caring apparatus 1.

In the present embodiment, the fan module portion 70 is illustrated as including a diagonal flow fan. The fan module portion 70 may suction air, which has passed through the filter portion 60, in the axial direction and discharge the air in a direction between the axial direction and radial direction.

The discharge portion 140 may be rotatably installed at the upper side of the first case portion 10 and may guide a discharge direction of air that has moved upward through the outlet 33. The rotation support portion 300 is disposed at the upper portion of the first case portion 10, and the discharge portion 140 is rotatably installed on the rotation support portion 300. Since both sides of the discharge portion 140 in the vertical direction are open, air that has moved to a lower portion of the discharge portion 140 through the guide vanes 356 may be discharged to the outside of the portable air caring apparatus 1 through an upper portion of the discharge portion 140.

The sanitizing portion 170 is disposed at the lower portion of the filter portion 60 and may be fixed to at least any one of the first case portion 10 and the second case portion 50. The sanitizing portion 170 is spaced a predetermined distance apart from the filter portion 60 and irradiates the filter portion 60 with sanitizing light. Since the sanitizing light irradiated by the sanitizing portion 170 is harmful to the human body, the installation position of the sanitizing portion 170 is set such that the sanitizing light does not leak to the outside of the portable air caring apparatus 1 through the inlet portion 22.

The battery 240 is installed in the accommodation space 21 provided inside the second case portion 50 and is disposed at a lower portion of the sanitizing portion 170. The battery 240 may supply power for driving the portable air caring apparatus 1.

### [Component arrangement structure of portable air caring apparatus]

The accommodation space 21 provided inside the portable air caring apparatus 1 may be divided into a first accommodation area A and a second accommodation area B. When the accommodation space 21 is divided in the vertical direction, an upper area is the first accommodation area A, and a lower area is the second accommodation area B. Note that the first accommodation area A and the second accommodation area B are not physically partitioned areas and are areas that are only conceptually divided.

In the third embodiment of the present invention, the accommodation space 21 of the first case portion 10 constituting the framework of the portable air caring apparatus 1 is set as the first accommodation area A, and the accommodation space 21 inside the second case portion 50 is set as the second accommodation area B.

Components relating to suctioning, purifying, and discharging air are disposed in the first accommodation area A. That is, since the inlet portion 22, the filter portion 60, the fan module portion 70, the rotation support portion 300, and the discharge portion 140 are disposed in the first accommodation area A, air flows from a lower side toward an upper side in the first accommodation area A, and a discharge direction of air is controlled through the discharge portion 140 which is rotatably installed.

In the first case portion 10, the inlet portion 22 having a plurality of inlet holes 23 formed therein is installed as a path for suctioning air. At the upper portion of the first case portion 10, the outlet 33 and the discharge portion 140 rotatably installed at the rotation support portion 300 are installed as a path for discharging air that is purified in the first accommodation area A. Therefore, in the first case portion 10, an air flow path which connects the filter portion 60, the fan module portion 70, and the discharge portion 140 is formed.

That is, the inlet portion 22, the filter portion 60, the fan module portion 70, the discharge portion 140, the rotation support portion 300, and the outlet 33 are provided in the first accommodation area A. A flow path necessary for the air, which is suctioned into the portable air caring apparatus 1, to pass through the air caring apparatus is formed in the first accommodation area A.

Components not directly related to a flow of air for purifying air are disposed in the second accommodation area B. That is, a controller, which includes a PCB, and the battery 240 may be installed in the second accommodation area B.

According to the present embodiment, the housing portion 3300 is formed in a cylindrical shape in which a length in the vertical direction is longer than a length in the lateral direction. Also, the first accommodation area A disposed at an upper portion is formed to have a longer length in the vertical direction than the second accommodation area B disposed at a lower portion. That is, when the portable air caring apparatus 1 stands upright, the first accommodation area A at the upper portion occupies a larger area than the second accommodation area B at the lower portion.

Due to being rotatably installed on the rotation support portion 300, the discharge portion 140 may easily control a discharge direction of purified air at an upper portion of the portable air caring apparatus 1. Therefore, it becomes easier for the air purified by the portable air caring apparatus 1 to reach the face of a user.

When the portable air caring apparatus 1 is used while placed on a floor surface at a lower position than the user's face, in order to increase an amount of air purified by the portable air caring apparatus 1 that reaches the user's face, using the portable air caring apparatus 1 in a vertical state is more advantageous than using the portable air caring apparatus 1 in a horizontal state.

To this end, when the portable air caring apparatus 1 stands upright, when discharge of air is performed through the discharge portion 140 which is rotated in a predetermined direction at the upper portion of the portable air caring apparatus 1, the amount of air purified by the portable air caring apparatus 1 that reaches the user's face may be further increased.

### [Detailed configuration of components of portable air caring apparatus]

The portable air caring apparatus 1 according to the third embodiment of the present invention may include at least any one of the housing portion 3300, the filter portion 60, the fan module portion 70, the discharge portion 140, and the rotation support portion 300. Also, the portable air caring apparatus 1 according to the third embodiment of the present invention may further include the sanitizing portion 170 and the battery 240.

### [First case portion]

The housing portion 3300 includes the first case portion 10 and the second case portion 50, the first case portion 10 has the accommodation space 21 formed therein, and the inlet portion 22 configured to suction air may be disposed at a side surface of a lower portion of the first case portion 10. The first case portion 10 is formed in a cylindrical shape and may be installed in a shape in which the upper and lower sides thereof are open. The first case portion 10 may consist of a single member or may consist of a plurality of members as necessary. The first case portion 10 according to the third embodiment of the present invention may consist of a plurality of members, and each member may be coupled by fitting or coupled using an adhesive or by welding, or the members may be connected to each other using a fastening member 195 such as a bolt. In this way, various modifications are possible.

Meanwhile, air is suctioned through the lower side surface of the first case portion 10, and air is discharged through the upper side of the first case portion 10. To this end, the inlet portion 22 including inlet holes 23 is installed along a periphery of a lower portion of the first case portion 10. Also, the outlet 33 configured to discharge air may be disposed at the upper side of the first case portion 10.

Also, in a state in which the inlet portion 22 configured to suction air is installed along the outer periphery of the first case portion 10, since the filter portion 60 is installed at an upper side that is spaced apart from the inlet portion 22, air may move evenly throughout the entire area of the filter portion 60.

Meanwhile, the plurality of inlet holes 23 are formed in the inlet portion 22, and the inlet holes 23 may be installed to be inclined in a diagonal shape or, as necessary, may be formed as holes each having the shape of an inequality sign in which the line is broken at the center. Also, in order to increase a flow rate of air entering the filter portion 60, the inlet holes 23 may be additionally formed in a side surface of the housing portion 3300 in which the filter portion 60 is installed. In this way, various modifications are possible.

Meanwhile, the housing portion 3300 may consist of three or more members. In this way, the housing portion 3300 may be modified to have various other shapes.

### [Second case portion]

The second case portion 50 is connected to the lower portion of the first case portion 10 and may be modified in various ways in which the second case portion 50 has a space formed therein to install electronic components including the battery 240.

At least any one of the first case portion 10 and the second case portion 50 may be made of a cylindrical case. Both the first case portion 10 and the second case portion 50 may be formed in a cylindrical shape, or only the second case portion 50 may be formed in a cylindrical shape. Alternatively, as necessary, only the first case portion 10 may be formed in a cylindrical shape.

In a case in which the second case portion 50 is formed in a cylindrical shape and extends in the vertical direction, it is convenient for a user to hold the outer periphery of the second case portion 50 with his or her hand, and the second case portion 50 may also be easily mounted on a cup holder of a vehicle that includes a groove portion having a substantially circular cross-section.

Also, in a case in which the first case portion 10 is formed in a cylindrical shape, friction, which is generated when air passing through the first case portion 10 and moving upward comes in contact with the inside of the first case portion 10 formed in a curved shape, may be reduced such that a flow of air is facilitated.

Meanwhile, since an air flow path is formed inside the first case portion 10 while an air flow path is not formed inside the second case portion 50, suction and discharge of air through the first case portion 10 may be smoothly performed even when the second case portion 50 is inserted into a cup holder or held by a user's hand. Thus, convenience in use may be improved.

### [Filter portion]

The filter portion 60 is installed inside the first case portion 10 and may be modified in various ways in which the filter portion 60 purifies air that enters through the inlet portion 22. The filter portion 60 according to the third embodiment of the present invention may be formed in a cylindrical shape.

Since the first case portion 10 is formed in the shape of a circular pipe and the filter portion 60, which is installed in the first case portion 10, is also formed in a cylindrical shape that comes in contact with the inside of the first case portion 10, impurities may be effectively removed from air passing through the first case portion 10.

Also, a transverse cross-section of the filter portion 60 is formed in a circular shape such that the filter portion 60 has the largest area in the first case portion 10. Also, when the filter portion 60 is manufactured in the form of a cylinder and an upper end and a lower end of a material constituting the filter portion 60 are cut, pressure loss may be minimized, and thus performance of the filter portion 60 may be maximized.

Also, since an outer diameter of the filter portion 60 is formed to be greater than or equal to a suction diameter of the bell mouth 132 through which air is suctioned into the fan module portion 70, the volume of the filter portion 60 may be maximized.

Also, according to the present embodiment, the filter portion 60, the fan module portion 70, the rotation support portion 300, and the discharge portion 140 may be arranged in the vertical direction along the housing portion 3300, and an air flow may also occur in the vertical direction. That is, an air flow that occurs due to the operation of the fan module portion 70 may occur in a linear direction which is the same as the direction in which the filter portion 60, the fan module portion 70, the rotation support portion 300, and the discharge portion 140 are arranged.

When the air flow occurs in the linear direction as described above, resistance related to the air flow is lowered correspondingly, and thus the air flow may occur more smoothly. In this way, since suctioning a sufficient amount of air and discharging a sufficient amount of air, which corresponds to the amount of suctioned air, may be performed by the fan module portion 70, air caring performance of the portable air caring apparatus 1 may be improved correspondingly.

### [Fan module portion]

The fan module portion 70 is disposed between the filter portion 60 and the outlet 33 and may be modified in various ways in which the fan module portion 70 rotates a fan to blow air in a direction toward the outlet 33.

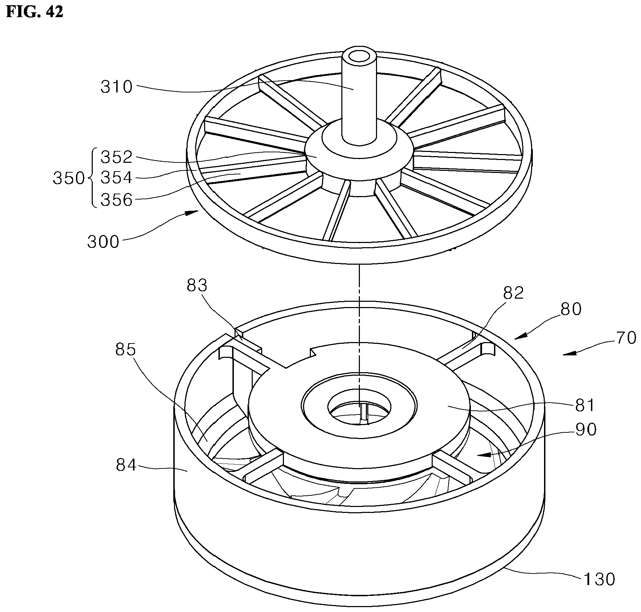

FIG. 42 is a perspective view illustrating the fan module portion 70 installed at a lower side of the rotation support portion 300 according to the third embodiment of the present invention.

As illustrated in FIG. 42, when the fan module portion 70, which is a circular diagonal flow fan module, is applied, since the shape of the fan module portion 70 matches with the inner shape of the first case portion 10, which is a cylindrical shape, or corresponds thereto, the size of the first case portion 10 is not necessarily enlarged due to fixing or fastening the fan module portion 70. In this way, reduction of product size is possible. Also, for use in a vehicle, the portable air caring apparatus 1 according to the present invention may be implemented to have a size that allows the portable air caring apparatus 1 to be inserted into a cup holder.

Also, since a circular diagonal flow fan module is applied to the fan module portion 70, a small-sized upward discharge type air cleaner that can maximize air flow performance may be provided. A type of fan of the fan module portion 70 is a diagonal flow fan, and an internal structure of the fan module portion 70 is changed to mount the diagonal flow fan.

The fan member 90 according to the present invention rotates due to operating a motor. Only a rotating shaft of the motor that rotates the fan member 90 may be connected to the fan member 90, a rotor may be installed at the fan member 90, and a stator may be installed in a fan housing 80 whose rotation is restricted. Since a magnetic field of the stator changes, the shaft that rotates along with the rotor may be connected to the fan member 90, and the rotor and the fan member 90 may rotate about the stator. Since the configuration of the motor rotating the fan member 90 is a known configuration, detailed description thereof will be omitted.

The fan module portion 70 according to the third embodiment of the present invention may include the fan housing 80, the fan member 90, and a fan base 130.

### [Fan housing]

The fan housing 80 is fixed to an inner side of the first case portion 10 and may be modified in various ways in which the fan housing 80 has a space formed therein to allow the fan member 90 to rotate. The fan housing 80 according to the third embodiment of the present invention may include at least any one of a support plate 81, a connection support 82, a wire guide 83, a side support portion 84, an inner side guiding portion 85, and a protruding boss 86.

The support plate 81 is formed in a disk shape, and a hole is formed at the center of the support plate 81. A motor may be installed at the center of the support plate 81, or a shaft connected to the motor may be installed in the first direction. The support plate 81 is disposed on the lower side of the core member 310.

The connection support 82 extends to the outside of the support plate 81 and is connected to the side support portion 84. The connection support 82 according to the third embodiment of the present invention may be provided as a plurality of connection supports 82 and may be installed in the shape of a rod. The connection supports 82 extending radially outward from the support plate 81 may be connected to the side support portion 84.

The connection support 82 according to the third embodiment of the present invention is disposed at a lower side of a core support portion 350 of the rotation support portion 300. The connection support 82 is radially installed about the support plate 81, and the rotation support portion 300 is installed at an upper side of the connection support 82.

The wire guide 83 is continuously installed on the connection support 82 and supports a lower portion of a wire of an electronic device so that the wire may move along a side surface of the connection support 82. The wire guide 83 is formed in the shape of a protrusion, which is disposed on a lower portion of a side surface of the connection support 82, and guides a wire of the motor installed on the support plate 81 to be installed to extend to the outside of the fan housing 80. The wire guide 83 may be installed at the side surface of the connection support 82 and form a concave groove to allow the wire to be disposed therein. Therefore, since the wire installed in the wire guide 83 is disposed in the concave groove disposed at the side surface of the connection support 82, and the lower portion of the wire is supported by the wire guide 83, damage to the wire may be prevented.

The side support portion 84 is formed in the shape of a cylindrical pipe, and the upper and lower sides thereof are open. An outer side of the side support portion 84 comes in contact with the inside of the housing portion 3300, and an inner side of the side support portion 84 is connected to the connection support 82.

The inner side guiding portion 85 forms an inclined surface that is installed to be inclined downward toward a radially inward side from a lower side of the side support portion 84. The inner side guiding portion 85 may be formed at the inner side of the side support portion 84 and may prevent a return air phenomenon in which air, which is blown upward by the fan member 90, moves to an inlet of the fan member 90 through an outer side surface of the fan member 90.

The protruding boss 86 extends to a lower end of the side support portion 84 and may be modified in various ways in which the protruding boss 86 includes a groove portion for inserting a coupling protrusion 134 of the fan base 130 which will be described below. The protruding boss 86 according to the third embodiment of the present invention is provided as a plurality of protruding bosses 86 that are installed in a circumferential direction of the side support portion 84.

### [Fan member]

The fan member 90 is rotatably installed in the fan housing 80 and may be modified in various ways in which the fan member 90 is able to move air in the direction toward the discharge portion 140.

A diagonal flow fan may be used as the fan member 90, but this is only the third embodiment of the present invention, and other types of fans may also be used as the fan member 90 according to the present invention. The fan member 90 according to the third embodiment of the present invention may include at least any one of a hub 100, a fan blade 110, and a shroud 120.

The hub 100 is disposed at the center of the fan housing 80 and may be modified in various ways in which the hub 100 receives external power and rotates.

The hub 100 is disposed at the center of the fan member 90 in the radial direction and may rotate along with the rotor and the shaft, which is an output shaft of the motor, that constitute the motor. The hub 100 according to the third embodiment of the present invention may include at least any one of a hub plate portion 101, an axial coupling portion 102, an inner side protruding portion 105, and a skirt portion 107.

The hub plate portion 101 may be formed in the shape of a disk that is parallel to the support plate 81. The axial coupling portion 102 may be provided on the hub plate portion 101. The axial coupling portion 102 may be disposed at the center of the hub plate portion 101 in the radial direction. The axial coupling portion 102 may be formed to protrude to an upper side and a lower side of the hub plate portion 101.

The axial coupling portion 102 may be coupled to an axial end portion of a shaft that transmits rotary power. For example, coupling between the axial coupling portion 102 and the shaft may be performed in a form in which the shaft is fitted to the axial coupling portion 102.

First reinforcing protrusions 103 are installed at predetermined intervals along the outer periphery of the axial coupling portion 102. The first reinforcing protrusions 103 are radially installed about the center of the axial coupling portion 102 and are formed as band-shaped protrusions at an outer side of the axial coupling portion 102. Therefore, since stress concentrated on the axial coupling portion 102 is distributed through the first reinforcing protrusions 103, structural rigidity of the axial coupling portion 102 may be improved.

The inner side protruding portion 105 may protrude in a direction from the hub plate portion 101 toward an upper portion on which the support plate 81 is installed. The inner side protruding portion 105 according to the third embodiment of the present invention is installed in an arc shape along the outer side edge of the hub plate portion 101. The inner side protruding portion 105 is formed in the shape of a pipe that extends in the vertical direction.

Also, second reinforcing protrusions 106 are installed at predetermined intervals along the inner periphery of the inner side protruding portion 105. The second reinforcing protrusions 106 are installed in the first direction along an inner side surface of the inner side protruding portion 105, and lower sides of the second reinforcing protrusions 106 are formed as band-shaped protrusions that extend toward the axial coupling portion 102. Therefore, since stress concentrated on the inner side protruding portion 105 is distributed through the second reinforcing protrusions 106, structural rigidity of the inner side protruding portion 105 may be improved. As necessary, the rotor of the motor may be fixed to the inner side of the inner side protruding portion 105.

The skirt portion 107 may protrude upward in a direction from an edge of the hub plate portion 101 toward the support plate 81. The skirt portion 107 may form an inclined surface that is further inclined outward in the second direction away from the hub plate portion 101 in the first direction. The skirt portion 107 is disposed at an outer side of the inner side protruding portion 105, and an inner diameter of the skirt portion 107 gradually increases from a lower side toward an upper side.

For example, the hub plate portion 101 and the skirt portion 107 may be connected in the shape of a truncated cone in which a hollow is formed and whose one side is open. The skirt portion 107 may be formed in the shape of a funnel in which an upper side is open and a lower side is blocked by the hub plate portion 101.

The shroud 120 is connected to an end portion of the fan blade 110 and installed in an annular shape and may be modified in various ways in which the shroud 120 may be spaced apart from the fan base 130.

The shroud 120 is installed along the outer periphery of the skirt portion 107, and the shroud 120 and the skirt portion 107 are connected to each other by the fan blade 110. Also, an outer diameter of the hub 100 and an inner diameter of the shroud 120 may gradually decrease in a direction from an upper side toward a lower side.

The shroud 120 may be spaced a predetermined distance apart from the hub 100 in the radial direction and may be disposed at an outer side of the hub 100 in the radial direction. Also, the shroud 120 may be spaced apart from the hub 100 as much as a distance that corresponds to a length of the fan blade 110 in the radial direction. Also, each fan blade 110 may connect the skirt portion 107, which is disposed at the hub 100, and the shroud 120 to each other.

The shroud 120 may form an inclined surface that is substantially parallel to the skirt portion 107. In the present embodiment, the skirt portion 107 and the shroud 120 are illustrated as being arranged in a form in which a distance between the skirt portion 107 and the shroud 120 gradually increases in a direction toward an upper side of the shroud 120.

The inlet protrusion 121 disposed at the lower side of the shroud 120 is a ring-shaped protrusion and extends in the first direction from the lower side of the funnel-shaped shroud 120. Since the inlet protrusion 121 is disposed at an inner side of the bell mouth 132 which will be described below, the inlet protrusion 121 may prevent the returning flow of air, which enters through an inlet provided in the lower side of the shroud 120, along an outer side of the shroud 120.

The fan blade 110 may be provided as a plurality of fan blades 110, and the plurality of fan blades 110 may be disposed to be spaced apart from each other at equal intervals along an outer peripheral surface of the hub 100. The fan blades 110 protrude to the outside of the hub 100 with respect to the center of the hub 100 and extend in a spiral shape. Also, the plurality of fan blades 110 may be disposed to be spaced apart from each other at predetermined intervals in a peripheral direction of the hub 100.

The fan blades 110 according to the third embodiment of the present invention may protrude to the outside of the skirt portion 107 in a centrifugal direction extending in a spiral shape from the center of the axial coupling portion 102. Also, when a direction from the outside of the axial coupling portion 102 toward the axial coupling portion 102 is the radial direction, the inner side of the fan blades 110 in the radial direction may be connected to the skirt portion 107, and the outer side of the fan blades 110 in the radial direction may be connected to the shroud 120 which will be described below.

The skirt portion 107 is a portion of the hub 100 that is directly connected to the fan blades 110 and is a portion that also comes in direct contact with air passing through the fan blades 110. The skirt portion 107 is also closely related to a flow path of air passing through the fan module portion 70.

Each fan blade 110 that connects the shroud 120 and the skirt portion 107 to each other may include a first end portion 111, a second end portion 112, a first edge 113, and a second edge 114.

The first end portion 111 is disposed at a front end of the fan blade 110 in a rotational direction thereof and may be formed in a linear shape that extends in the radial direction. The rotational direction is defined as a direction in which rotation of the fan member 90 occurs.

The second end portion 112 is disposed at a rear end of the fan blade 110 in the rotational direction thereof and may be radially formed about the axial coupling portion 102.

The first edge 113 may connect one end of the first end portion 111 and one end of the second end portion 112. The first edge 113 may be connected to an inner peripheral surface of the shroud 120.

The second edge 114 may connect the other end of the first end portion 111 and the other end of the second end portion 112. The second edge 114 may be connected to the outer peripheral surface of the hub 100.

That is, the one end of the first end portion 111 and the one end of the second end portion 112 may be connected to the inner peripheral surface of the shroud 120. Also, the other end of the first end portion 111 and the other end of the second end portion 112 may be connected to an outer peripheral surface of the skirt portion 107.

The one end of the first end portion 111 may be disposed closer to the center of the hub plate portion 101 in the radial direction than the one end of the second end portion 112. Also, the other end of the second end portion 112 may be disposed closer to the center of the hub plate portion 101 in the radial direction than the other end of the first end portion 111. This is because the one end and the other end of the first end portion 111 are disposed more toward the front in the rotational direction than the one end and the other end of the second end portion 112, and the skirt portion 107 is formed such that a radius thereof gradually decreases toward the front in the rotational direction.

According to the present embodiment, the fan blade 110 is connected to the skirt portion 107 of the hub 100. In order to guide a flow of air entering the fan module portion 70 in a direction that is inclined upward, the skirt portion 107 forms an inclined surface that is inclined upward.

### [Fan base]

The fan base 130 is coupled to a lower side of the fan housing 80 and may be modified in various ways in which the fan base 130 guides air, which has passed through the filter portion 60, to enter the fan member 90.

The fan base 130 may be disposed between the filter portion 60 and the fan member 90. Also, an edge of the fan base 130 may be formed in a shape that corresponds to the shape of an edge of the filter portion 60. For example, when the filter portion 60 is formed in a cylindrical shape and the edge of the filter portion 60 is formed in a circular shape, the fan base 130 may be installed in an annular shape having a hollow formed therein.

A base plate 131 may be disposed between the filter portion 60 and the fan member 90. The base plate 131 is formed in the shape of a plate that extends in an annular shape and has a hollow formed at the center to allow movement of air.

The bell mouth 132 is installed in an annular shape at an inner side of the base plate 131 that faces the hollow. The bell mouth 132 extends in the circumferential direction and has a longitudinal cross-section formed in a concave shape that surrounds a lower side of the inlet protrusion 121 of the shroud 120.

The bell mouth 132 may be formed in a shape that surrounds an outer peripheral surface of the hollow formed at the center of the base plate 131. The bell mouth 132 is formed to be convex toward the lower side and may form a groove portion that is concave toward the upper side.

At least one portion of the bell mouth 132 may be inserted into the shroud 120 in the radial direction. The bell mouth 132 may guide a suctioning flow at the inlet of the fan module portion 70 to contribute to an improvement in suctioning and discharging performance of the fan module portion 70.

The coupling protrusion 134 protrudes to an upper side of the base plate 131 and is coupled to the groove portion of the protruding boss 86, which is disposed in the fan housing 80, by being fitted thereto to fix the fan base 130 to the lower side of the fan housing 80.

The fan base 130 and the fan housing 80 may be coupled to each other at a plurality of points due to coupling performed between the coupling protrusion 134 and the protruding boss 86. When coupling between the fan base 130 and the fan housing 80 is performed as described above, the fan member 90 may be rotatably installed between the fan base 130 and the fan housing 80.

A protruding rib 133 protrudes from the base plate 131 and may be disposed at an outer side of the bell mouth 132 in the radial direction. The protruding rib 133 according to the third embodiment of the present invention is located at the outer side of the bell mouth 132 in the radial direction and is installed in an annular shape that surrounds the outer periphery of the bell mouth 132. Also, the protruding rib 133 may be integrally formed with the base plate 131. More specifically, the base plate 131, the bell mouth 132, and the protruding rib 133 may be integrally formed.

Also, the protruding rib 133 may be installed to be inclined at the same angle as an outer side surface of the shroud 120, and a distance between the protruding rib 133 and the shroud 120 may be maintained to be constant. The protruding rib 133 may protrude in a shape forming an inclined surface. The inclined surface of the protruding rib 133 may be formed as an inclined surface that is spaced a predetermined distance apart from the shroud 120 and is parallel to the inclined surface of the shroud 120.

Also, the inclined surface of the protruding rib 133 may have the same angle of inclination as an inclined surface of the inner side guiding portion 85 disposed in the fan housing 80. Therefore, it is possible to prevent a return air phenomenon in which a portion of air, which has moved upward through a space between the shroud 120 and the skirt portion 107, moves to the inlet of the fan member 90 through a space between the shroud 120 and the protruding rib 133.

### [Discharge portion]

As illustrated in FIGS. 35 to 37, the discharge portion 140 is rotatably installed on the rotation support portion 300 and may be modified in various ways in which the discharge portion 140 controls a discharge direction of air that has passed through the fan module portion 70. The discharge portion 140 according to the present invention may smoothly rotate due to being rotatably installed on a spherical ball joint 370 disposed in the rotation support portion 300.

Since the discharge portion 140 is open in the vertical direction and rotatably connected to the rotation support portion 300, the discharge portion 140 may control the discharge direction of air which has passed through the fan module portion 70. The discharge portion 140 according to the third embodiment of the present invention may include a first discharge portion 150 and a second discharge portion 160.

The first discharge portion 150 is disposed at one side (the upper side in FIG. 2) of the ball joint 370 and may be modified in various ways in which the first discharge portion 150 includes a plurality of vanes 156 configured to guide discharge of air. The first discharge portion 150 according to the third embodiment of the present invention may include a first discharge core 152, a first discharge body 154, and the vanes 156.

The first discharge core 152 is installed in a shape that surrounds the spherical upper side of the ball joint 370 and is disposed at the upper side of the core member 310. Also, the first discharge body 154 is installed in an annular shape that surrounds an outer side of the first discharge core 152, and an outer side of the first discharge body 154 is formed in a curved shape.

Also, since the first discharge core 152 and the first discharge body 154 are connected to each other by the plurality of vanes 156, the first discharge core 152, the first discharge body 154, and the vanes 156 may rotate together.

The second discharge portion 160 is disposed at the other side (the lower side in FIG. 2) of the ball joint 370 and may be modified in various ways in which the second discharge portion 160 is connected to the first discharge portion 150 and rotates about the ball joint 370 along with the first discharge portion 150. The second discharge portion 160 according to the third embodiment of the present invention may include a second discharge core 161, a second discharge body 162, and discharge supports 163.

The second discharge core 161 is installed in a shape that surrounds the spherical lower side of the ball joint 370 and is disposed at a lower side of the first discharge core 152. Also, the second discharge body 162 is installed in an annular shape that surrounds an outer side of the second discharge core 161, and an outer side of the second discharge body 162 is formed in a curved shape.

Also, since the second discharge core 161 and the second discharge body 162 are connected to each other by the plurality of discharge supports 163, the second discharge core 161, the second discharge body 162, and the discharge supports 163 may rotate together.

### [Sanitizing portion]

As illustrated in FIG. 2, the sanitizing portion 170 is disposed between the filter portion 60 and the second case portion 50 and may be modified in various ways in which the sanitizing portion 170 irradiates the sanitizing light toward the filter portion 60. The sanitizing portion 170 according to the third embodiment of the present invention may include at least any one of a sanitization support portion 210, a pedestal portion 220, and an irradiating portion 230.

### [Sanitization support portion]

The sanitization support portion 210 is disposed between the first case portion 10 and the second case portion 50 and blocks the lower portion of the first case portion 10. Also, the sanitization support portion 210 is disposed at a lower side of the irradiating portion 230 and may be modified in various ways in which the sanitization support portion 210 is connected to the housing portion 3300 such that movement of the sanitization support portion 210 is restricted.

Movement of air, which enters the first case portion 10 through the inlet portion 22, toward the second case portion 50 is blocked by the sanitization support portion 210, and thus a flow rate of air moving toward the fan module portion 70 may be increased, and air caring performance of the portable air caring apparatus 1 may be improved.

The pedestal portion 220 may be modified in various ways in which the pedestal portion 220 protrudes upward from the center of the sanitization support portion 210 to support the lower portion of the irradiating portion 230. Also, the pedestal portion 220 is disposed at the center of the inlet portion 22 in the radial direction, and a transverse cross-section of the pedestal portion 220 may be formed in a circular shape to reduce friction with air.

The pedestal portion 220 may be formed in the shape of a column that protrudes upward from the center of the sanitization support portion 210. Also, the pedestal portion 220 may be formed in a cylindrical or conical shape. The pedestal portion 220 according to the third embodiment of the present invention is formed in a shape whose transverse cross-section gradually narrows from a lower side toward an upper side, and is disposed at the center of the first case portion 10 in which the inlet portion 22 is formed. Thus, friction with air may be minimized.

A transverse cross-section of the sanitization support portion 210, which is installed to sanitize the filter portion 60, has a circular shape, and air that enters through the inlet portion 22 rotates in a spiral due to the inclined shape of the inlet holes 23, rotates along an outer side of the pedestal portion 220, and moves to an upper side where the filter portion 60 is installed. That is, since the sanitizing portion 170 is disposed at a central portion of the first case portion 10 and air, which enters through the inlet portion 22, moves upward while rotating along the outer periphery of the sanitizing portion 170, resistance of a flow path of the sanitizing portion 170 is decreased.

Since the pedestal portion 220, the center of rotation of the fan member 90, and the core member 310 of the rotation support portion 300, which will be described below, are disposed in a straight line in the perpendicular direction, resistance related to a flow of air moving from a lower side toward an upper side is decreased, and thus the air flow may occur more smoothly. As a result, air caring performance of the portable air caring apparatus 1 is improved.

The irradiating portion 230 is mounted on an upper side of the pedestal portion 220 and may irradiate the sanitizing light in a direction toward the filter portion 60. Also, the irradiating portion 230 may be disposed on a vertical reference line that passes through the radial center of the inlet portion 22 in the vertical direction. Therefore, in a case in which the filter portion 60 is disposed at an upper side of the irradiating portion 230, the entire area of a lower end of the filter portion 60 may be sanitized by a relatively small number of sanitizing light sources 232, and thus production costs and maintenance and repair costs may be reduced.

Also, the irradiating portion 230 may be modified in various ways in which the irradiating portion 230 is installed at a position that is level with or higher than an upper end of the inlet portion 22. The irradiating portion 230 according to the third embodiment of the present invention may include a PCB 231 and the sanitizing light source 232. The PCB 231 is installed on the upper side of the pedestal portion 220, and the sanitizing light source 232 configured to irradiate the sanitizing light is installed on an upper side of the PCB 231. The sanitizing light source 232 may be a UVC LED, or various other types of sanitizing apparatuses may be used as the sanitizing light source 232 in which the sanitizing light source 232 sterilizes the filter portion 60.

Meanwhile, since the sanitizing light source 232 of the sanitizing portion 170 is disposed at the upper side of the inlet portion 22, the sanitizing light source 232 may be prevented from irradiating the sanitizing light to the outside of the first case portion 10 through the inlet portion 22.

### [Rotation support portion]

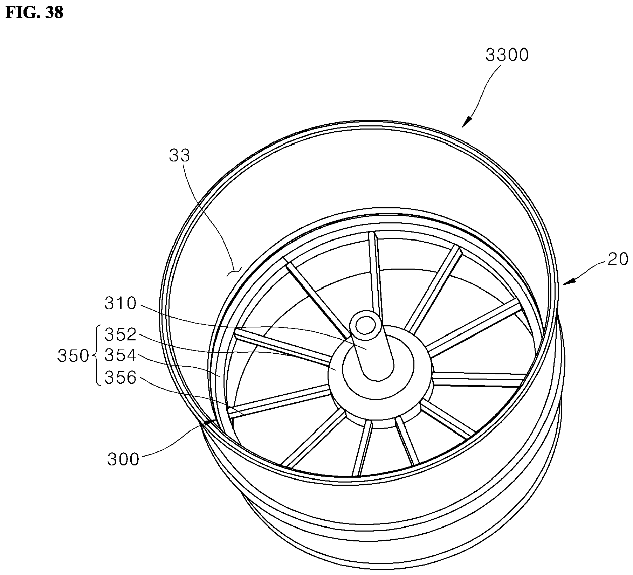

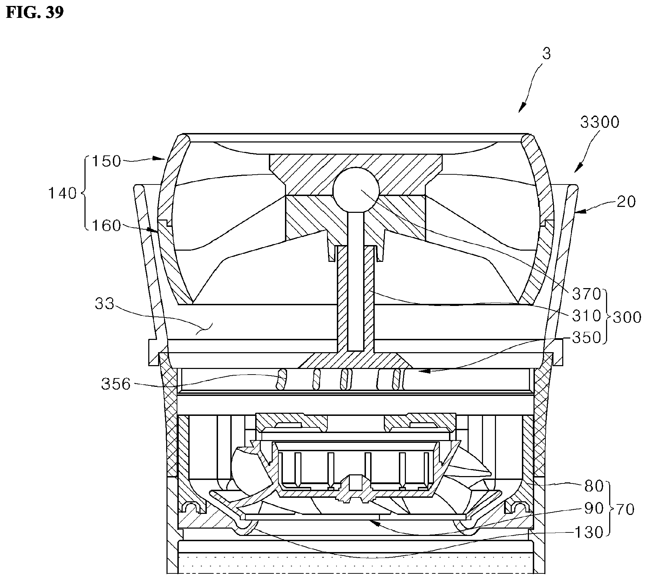

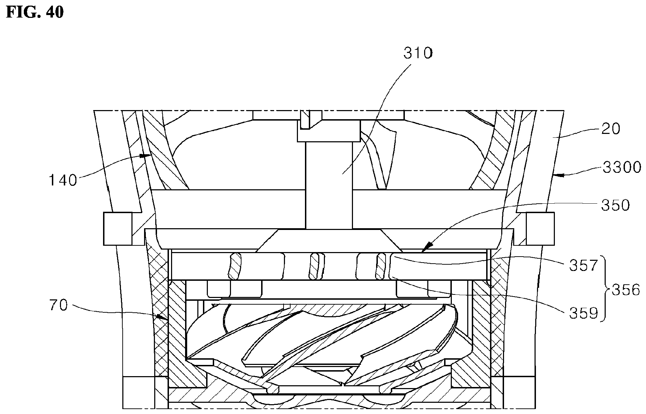

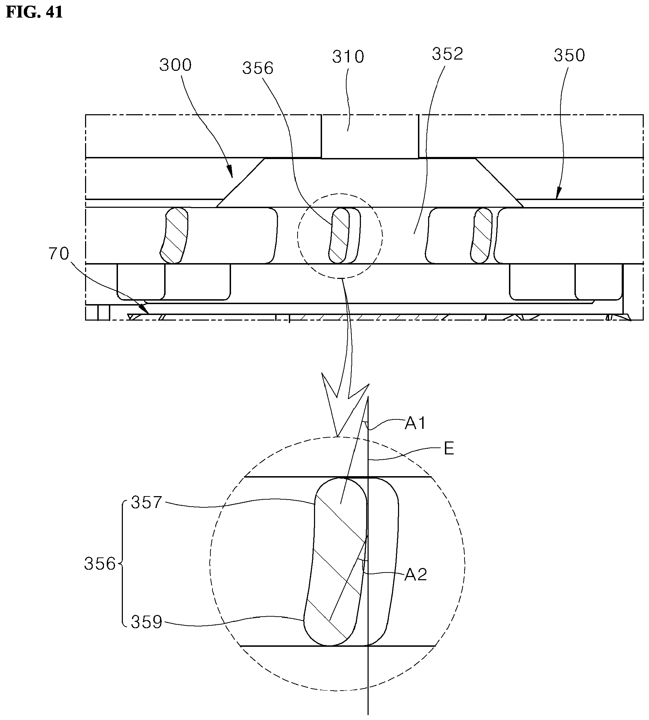

FIG. 38 is a perspective view illustrating a state in which the rotation support portion 300 is installed in the housing portion 3300 according to the third embodiment of the present invention, FIG. 39 is a cross-sectional view illustrating the state in which the rotation support portion 300 is installed in the housing portion 3300 according to the third embodiment of the present invention, and FIGS. 40 and 41 are cross-sectional views illustrating the guide vanes 356 according to the third embodiment of the present invention.

As illustrated in FIG. 36 and FIGS. 38 to 41, the rotation support portion 300 rotatably supports the discharge portion 140 which is disposed at the outlet 33 of the housing portion 3300. The rotation support portion 300 according to the third embodiment of the present invention may include the core member 310, the core support portion 350, and the ball joint 370.

### [Core member]

The core member 310 is disposed at a lower side of the discharge portion and extends in a direction toward the discharge portion. The core member 310 is disposed at a lower side of the discharge portion 140 configured to control a discharge direction of air and may extend from the center of the outlet 33 in a direction toward the discharge portion 140. Also, the core member 310 is disposed at a lower side of a second support portion 190 and may extend in a direction from the center of the outlet 33 toward the second support portion 190 to support the ball joint 370. Also, the core member 310 has an outer side formed as a curved surface, and a transverse cross-sectional area of the core member 310 gradually decreases in a direction from a lower side of the core member 310, which is connected to the core support portion 350, toward the ball joint 370. Thus, resistance of air moving from a lower side to an upper side may be minimized.

Alternatively, while the outer side of the core member 310 is formed as a curved surface, the transverse cross-sectional area of the core member 310 may vary or be maintained to be constant in the direction from the lower side of the core member 310, which is connected to the core support portion 350, toward the ball joint 370. In this way, various modifications are possible.

The core member 310 may be installed in a conical shape, and a transverse cross-sectional area of the core member 310 may gradually decrease upward. Alternatively, the core member 310 may be installed in the shape of a pipe that extends in the vertical direction.

### [Core support portion]

The core support portion 350 may include a first core support portion 352 configured to support the core member 310 and a second core support portion 354 installed along the outer periphery of the first core support portion 352 and fixed to the housing portion 3300. Also, the first core support portion 352 and the second core support portion 354 may be connected by the guide vanes 356. The core support portion 350 according to the third embodiment of the present invention supports the core member 310 and may be modified in various ways in which the core support portion 350 is fixed to the inside of the housing portion 3300 in a state in which the core support portion 350 includes the guide vanes 356.

The core support portion 350 may radially extend or be installed in a spiral shape with respect to the core member 310. In this way, various modifications are possible. The core support portion 350 according to the third embodiment of the present invention may include the first core support portion 352, the second core support portion 354, and the guide vanes 356.

The first core support portion 352 is coupled to a lower portion of the core member 310 and may be installed in a ring shape or installed in a plate shape on the lower portion of the core member 310. The core member 310 is coupled to an inner side or an upper side of the first core support portion 352, and the second core support portion 354, which has a ring shape, is installed at an outer side that is spaced apart from the first core support portion 352. The second core support portion 354 may be fixed by being fitted to the housing portion 3300 or fixed using a bolt or the like. Movement of the second core support portion 354 may be restricted using various fixing methods.

### [Guide vane]

The guide vanes 356 are disposed at an upper side of the fan module portion 70 and switch a movement direction of air, which rotates and moves upward in the fan module portion 70, to guide air in a direction toward the outlet 33. Also, an inlet angle, which is formed between a lower portion of the guide vane 356 and the rotation axis extension line E of the fan module portion 70, and an outlet angle, which is formed between an upper portion of the guide vane 356 and the rotation axis extension line E of the fan module portion 70, are formed to be different.

The first core support portion 352 and the second core support portion 354 may be connected by the guide vanes 356. Also, the guide vanes 356 may be installed in a spiral shape or installed in a radial shape about the first core support portion 352. Since the first core support portion 352 is installed in a spiral shape in a direction of wind discharged from the fan module portion 70, frictional resistance due to the guide vanes 356 coming in contact with air may be reduced.

Alternatively, the guide vanes 356 are radially installed about the core member 310 or the first core support portion 352. The guide vanes 356 according to the third embodiment of the present invention radially extend about the core member 310 and switch a movement direction of air, which moves in the fan module portion 70, to guide air in the direction toward the discharge portion. To this end, the inlet angle and outlet angle of the guide vanes 356 may be formed to be different.

The guide vane 356 installed at an upper side of the fan module portion 70 switches a movement direction of air which is discharged upward from the fan module portion 70. Since angles at which the lower side and upper side of the guide vanes 356 are installed are different from each other, air rotating and moving upward in the fan module portion 70 is guided by the guide vanes 356 and moves upward toward the outlet 33. Thus, linearity of air may be improved.

The guide vanes 356 may include a first vane 357 installed in a direction toward the outlet 33 and a second vane 359 which extends from the first vane 357 in a direction toward the fan module portion 70.

As illustrated in FIG. 41, in a longitudinal cross-section of the guide vane 356, the first vane 357 is disposed at an upper side, and the second vane 359 is connected to a lower side of the first vane 357.

An angle formed between the first vane 357, which is installed in the direction toward the discharge portion, and the rotation axis extension line E of the fan module portion 70 or the vertical reference line is A1. An angle between the rotation axis extension line E and a center line, which extends in the longitudinal direction of the first vane 357 along the center of the first vane 357, is A1.

Also, an angle formed between the second vane 359 and the rotation axis extension line E of the fan module portion 70 or the vertical reference line is A2. An angle between the rotation axis extension line E and a center line, which extends in the longitudinal direction of the second vane 359 along the center of the second vane 359, is A2. The inlet angle of the guide vanes 356 is A2, and the outlet angle of the guide vanes 356 is A1.

Here, since A1 and A2 are formed to be different, the guide vanes 356 have a bent longitudinal cross-section. A1 and A2 may form an acute angle with the rotation axis extension line E or vertical reference line. Therefore, the guide vanes 356 may be installed in an inclined shape with respect to the rotation axis extension line E. The guide vanes 356 may be modified in various ways in which the guide vanes 356 minimize an increase in frictional resistance with air discharged from the fan member of the fan module portion 70 and guide air, which rotates in a spiral shape and moves upward, in an upward direction toward the discharge portion.

The center line of the first vane 357 may coincide with the rotation axis extension line E, and a value of A1 may be changed within an acute angle range. A2 is greater than A1 in the guide vanes 356 according to the third embodiment of the present invention. Therefore, air, which is firstly guided upward due to passing through the second vane 359, is secondarily guided in an upward, perpendicular direction again due to passing through the first vane 357.

Also, a direction in which the second vane 359 is inclined while extending downward from the first vane 357 may correspond to a movement direction of air moving from the fan module portion 70 toward the guide vanes 356. When the fan member of the fan module portion 70 rotates clockwise, the second vane 359 extends to be inclined downward counterclockwise from the first vane 357. Therefore, air rotating clockwise and moving upward in the fan module portion 70 is moved to the first vane 357 through the second vane 359 extending counterclockwise. Thus, frictional resistance of air may be minimized, and a movement direction of air may be switched to an upward direction.

Alternatively, when the fan member of the fan module portion 70 rotates counterclockwise, the second vane 359 extends to be inclined downward clockwise from the first vane 357. Since air rotating counterclockwise and moving upward in the fan module portion 70 is moved to the first vane 357 through the second vane 359 extending clockwise, frictional resistance of air may be minimized, and a movement direction of air may be switched to an upward direction.

### [Ball joint]

The ball joint 370 may be modified in various ways in which a lower side of the ball joint 370 is coupled to the core member 310 so as to be restricted from moving and an upper side of the ball joint 370 is inserted into the discharge portion to rotatably support the discharge portion.

An end portion of the ball joint 370 has a spherical shape and may be disposed in the discharge portion 140 to rotatably support the discharge portion 140.

### [Air flow of portable air caring apparatus]

Since the inlet portion 22 configured to suction outside air is installed along the outer periphery of the first case portion 10, air outside the first case portion 10 may move into the first case portion 10 through the inlet portion 22, and thus a suction flow rate of air may increase.

Due to the operation of the fan module portion 70, air outside the portable air caring apparatus 1 enters the portable air caring apparatus 1. Here, the air outside the portable air caring apparatus 1 passes through the inlet holes 23 and forms a spiral air flow that rotates along the outer periphery of the sanitization support portion 210.

Air, which enters a first case 20 and moves upward while rotating in a spiral, passes through the filter portion 60, and in this process, physical particles such as dust, fine dust, and ultrafine dust, chemical substances such as odorous particles and harmful gases, and microorganisms such as bacteria and viruses that are contained in the air may be filtered.

Since the filter portion 60, the fan module portion 70, and the rotation support portion 300 are disposed in a straight line in the vertical direction, suctioning and filtering of air may be effectively performed while flow loss of air is minimized.

Air that has passed through the filter portion 60, that is, purified air, may enter the fan module portion 70. A flow of air may be guided by the bell mouth 132, and in this way, air may be effectively guided to smoothly enter the fan module portion 70.

Air entering the fan module portion 70 is discharged to the upper side of the fan module portion 70. The air discharged to the upper side of the fan module portion 70 may be discharged in a diagonal flow direction. Here, the diagonal flow direction may be defined as an upward diagonal direction.

Air suctioned into a central portion of the lower side of the fan module portion 70 is moved upward through a discharge port provided in an annular shape along an inner side of an edge of the fan module portion 70 and is moved upward through a space formed between the core support portions 350 of the rotation support portion 300. That is, since a diagonal flow fan is applied to the fan module portion 70, the air that enters the lower portion of the fan module portion 70 may be discharged in a direction that is inclined upward, and an air movement path of the rotation support portion 300 may coincide with a flow path direction. Thus, flow loss of air may be reduced.

In this way, air discharged to the upper side of the fan module portion 70 rotates and moves upward. The air discharged to the upper side of the fan module portion 70 rotates in a spiral and moves upward, and the upward movement of air is guided by the guide vanes 356 included in the rotation support portion 300.

As air discharged from the fan module portion 70 comes in contact with the second vane 359, the direction of the air is firstly switched to a direction toward an upper side where the discharge portion is disposed. Also, as the air that has passed through the second vane 359 passes through the first vane 357, the direction of the air is secondarily switched to the direction toward an upper side where the discharge portion is disposed. Since the longitudinal cross-section of the guide vanes 356 has a bent shape, and the switching of the discharge direction of air is performed in two stages, an increase in frictional resistance of air may be minimized, and the movement direction of air may be easily switched to the direction toward the discharge portion.

The air, whose direction is switched to the direction toward the upper side due to passing through the guide vanes 356, enters the discharge portion 140 and is discharged to the upper side of the discharge portion 140.

Since the discharge portion 140 rotates within a predetermined angle range, a direction in which air is discharged may be controlled according to an angle at which the discharge portion 140 is installed.

Also, since the inner side of the discharge portion 140 forms a concave groove portion, an increase in discharge resistance of air, whose direction is switched through the discharge portion 140, may be reduced. Also, since the filter portion 60, the fan module portion 70, and the discharge portion 140 are disposed in a straight line in the vertical direction, suctioning and filtering of air and discharging of purified air may be effectively performed while flow loss of air is minimized.

In a portable air caring apparatus according to the present invention, since a second case portion is mounted on a structure such as a cup holder having the shape of a groove which is concave toward the lower side, and air can be suctioned through an inlet portion provided in a first case portion without interfering with the external structure, air caring performance can be improved.

Also, according to the present invention, since the inlet portion configured to suction air is installed along the periphery of the first case portion, a suction flow rate of air increases, and thus air caring performance can be improved.

Also, according to the present invention, since the inlet portion configured to suction air is installed along the periphery of the first case portion, the position of the inlet portion is not required to be taken into account when using the product, and thus convenience in use can be improved.

Also, according to the present invention, since air entering through the inlet portion evenly passes through a filter portion and moves in a direction toward a fan module portion, air cleaning efficiency can be improved.

Also, according to the present invention, since a pedestal portion and an irradiating portion of a sanitizing portion are disposed at the center of the first case portion, and a curved surface is formed at an outer side of the pedestal portion, resistance of a flow path of the sanitizing portion decreases, and thus air caring performance can be improved.

Also, according to the present invention, since a discharge portion is rotatably installed at an upper side of the first case portion and can easily control a discharge direction of air being discharged to the upper side of the first case portion, convenience in use can be improved.

Also, according to the present invention, since a safety portion is installed to be continuous with a fan base and a protective mesh is formed at an outer side of a suction portion, occurrence of accidents can be prevented, and the filter portion or the like is blocked from entering a fan member such that operational reliability is improved.

Also, according to the present invention, since a blocking portion is not installed at a position facing the suction portion, an area coming in contact with air moving toward the suction portion is decreased, and thus an air blowing function can be improved.

Also, according to the present invention, since a rotation support portion configured to rotatably support the discharge portion guides air, which moves upward while rotating, in a direction toward the discharge portion to increase an amount of blown air, air cleaning efficiency can be improved.

Also, according to the present invention, since a guide vane configured to guide a movement direction of air is separately installed between an air blowing fan and the discharge portion, frictional resistance of air moving toward the discharge portion is reduced and the amount of blown air is increased. Thus, power consumption can be reduced.

Also, according to the present invention, since the movement direction of air can be switched while the discharge portion is rotatably supported, production costs can be reduced as compared to when two separate components are used for switching the movement direction of air and rotatably supporting the discharge portion.

In addition to the above-described effects, specific effects of the present invention have been described above along with specific details for carrying out the invention.

The present invention has been described above with reference to the accompanying drawings, but the present invention is not limited by the embodiments disclosed herein and the drawings, and it is apparent that various modifications may be made by those of ordinary skill in the art to which the present invention pertains. Further, even when the effects according to configurations of the present invention are not explicitly described while describing the embodiments of the present invention, predictable effects of the corresponding configurations should also be recognized.

## Claims

1. A portable air caring apparatus (1) comprising:
an inlet portion (22) configured to form a path along which air is suctioned;
a filter portion (60) disposed at an upper side of the inlet portion (22) and configured to purify air which enters through the inlet portion (22) and moves upward;
a sanitizing portion (170) disposed at a lower side of the filter portion (60), installed at a height allowing the sanitizing portion (170) to overlap the inlet portion (22), and configured to irradiate sanitizing light toward the filter portion (60); and
a fan module portion (70) disposed at an upper side of the filter portion (60) and configured to rotate a fan member (90) to blow air in a direction toward the upper side of the filter portion (60).

2. The portable air caring apparatus of claim 1, wherein a center of the inlet portion (22), a center of the filter portion (60), a center of the sanitizing portion (170), and a center of the fan module portion (70) coincide in a vertical direction.

3. The portable air caring apparatus of claim 1 or 2, further comprising a housing portion (3300) which includes the inlet portion (22), has the filter portion (60), the sanitizing portion (170), and the fan module portion (70) disposed therein, and forms an air flow path in a vertical direction.

4. The portable air caring apparatus of claim 3, wherein a cylindrical air flow path is formed in the housing portion (3300), and a center of the inlet portion (22), a center of the filter portion (60), a center of the sanitizing portion (170), and a center of the fan module portion (70) coincide in the vertical direction along a vertical reference line that passes through a center of the housing portion (3300) in the vertical direction.

5. The portable air caring apparatus of claim 3 or 4, wherein the housing portion (3300) includes:
a first case portion (10) in which the filter portion (60), the sanitizing portion (170), and the fan module portion (70) are installed, the inlet portion (22) is disposed at a side surface of a lower portion, and an outlet (33) configured to discharge air is disposed at an upper side; and
a second case portion (50) connected to a lower portion of the first case portion (10),
wherein an air flow path is formed in the first case portion (10), and an air flow path is not formed in the second case portion (50),wherein optionally a battery (200) is embedded in the second case portion (50).

6. The portable air caring apparatus of claim 5, further comprising a discharge portion (140) rotatably installed at an upper side of the first case portion (10) and configured to control a discharge direction of air that has passed through the fan module portion (70),
wherein a center of rotation of the discharge portion (140) coincides with the center of the fan module portion (70) in the vertical direction, and
air that enters through the inlet portion (22) moves upward and sequentially passes through the filter portion (60), the fan module portion (70), and the discharge portion (140).

7. The portable air caring apparatus of claim 6, wherein the first case portion (10) includes:
a first case (20) which includes the inlet portion (22) and is coupled to an upper side of the second case portion (50); and
a second case (30) which is disposed at an upper side of the first case (20) and includes the outlet (33),
wherein optionally the first case portion (10) further includes an intermediate case (40) which is installed in a shape surrounding an outer side of the filter portion (60) and connects the first case (20) and the second case (30) to each other, and/or
wherein optionally the second case (30) includes:
a second case body (31) installed in a shape that surrounds an outer periphery of the fan module portion (70);
a tubular expansion member (32) which extends to an upper side of the second case body (31) and in which an inner path gradually widens upward; and
a rotation support portion (35) which is connected to at least any one of the second case body (31) and the tubular expansion member (32) to rotatably support the discharge portion (140) at a center of the outlet (33) that is disposed at an inner side of the tubular expansion member (32), wherein optionally the rotation support portion (35) includes:
a core member (36) which is disposed at a lower side of the discharge portion (140) and extends in a direction from the center of the outlet (33) toward the discharge portion (140);
a support (37) which extends to an outer side of the core member (36) and is connected to at least any one of the second case body (31) and the tubular expansion member (32) to restrict movement of the core member (36); and
a ball joint (38) which is coupled to the core member (36) and configured to rotatably support the discharge portion (140),
wherein optionally:
the fan module portion (70) is disposed at a lower side of the rotation support portion (35);
the fan module portion (70) includes a fan housing (80) fixed to the first case portion (10);
the fan housing (80) includes a support plate (81) disposed at a lower side of the core member (36) and a connection support (82) extending radially outward from the support plate (81); and
the connection support (82) and the support (37) overlap in the vertical direction to decrease resistance of an air flow path.

8. The portable air caring apparatus of claim 6 or 7, wherein the discharge portion (140) includes:
a first discharge portion (150) which includes a plurality of vanes (156) configured to guide discharge of air; and
a second discharge portion (160) which is connected to the first discharge portion (150) and rotates along with the first discharge portion (150).

9. The portable air caring apparatus of any one of claims 5 to 8, wherein the inlet portion (22) is installed along an outer periphery of the housing portion (3300) and includes a plurality of inlet holes (24) formed to guide movement of air into the first case portion (10).

10. The portable air caring apparatus of any one of claims 1 to 9, wherein D1, which is a length of the inlet portion (22) in a vertical direction, is formed to be shorter than D2, which is a length of the filter portion (60) in the vertical direction.

11. The portable air caring apparatus of any one of claims 3 to 10, wherein the sanitizing portion (170) includes an irradiating portion (180) which is disposed on a vertical reference line, which passes through the center of the inlet portion (22) in the vertical direction, and configured to irradiate sanitizing light in a direction toward the filter portion (60),
wherein optionally the sanitizing portion (170) further includes:
a sanitization support portion (171) which is disposed at a lower side of the irradiating portion (180) and connected to the housing portion (3300) so as to be restricted from moving; and
a pedestal portion (176) which protrudes to an upper side of the sanitization support portion (171) to support the lower side of the irradiating portion (180),
wherein optionally:
the pedestal portion (176) is disposed at a center of the inlet portion (22), and a transverse cross-section of the pedestal portion (176) is formed in a circular shape, and/or
the irradiating portion (180) is installed at a position that is level with or higher than an upper end of the inlet portion (22), and/or
when a distance between the fan module portion (70) and the filter portion (60) is L1 and a distance between the filter portion (60) and the irradiating portion (180) is L2, L1 is shorter than L2.

12. The portable air caring apparatus of any one of claims 6 to 11, wherein the fan module portion (70) includes:
a fan housing (80) fixed to an inner side of the housing portion (3300);
a fan member (90) rotatably installed in the fan housing (80) to move air in a direction toward the discharge portion (140); and
a fan base (130) coupled to a lower side of the fan housing (80) to guide air, which has passed through the filter portion (60), to enter the fan member (90).

13. The portable air caring apparatus of claim 12, wherein the fan member (90) includes:
a hub (100) which is disposed at a center of the fan housing (80) to receive external power and rotate;
a plurality of fan blades (110) disposed to be spaced apart at equal intervals along an outer peripheral surface of the hub (100); and
a shroud (120) which is connected to an end portion of the fan blades (110), installed in an annular shape, and spaced apart from the fan base (130),
wherein optionally an outer diameter of the hub (100) and an inner diameter of the shroud (120) gradually decreases from an upper side toward a lower side.

14. The portable air caring apparatus of any one of claims 1 to 13, further comprising:
a safety portion (1400) which is installed between the filter portion (60) and the fan module portion (70) and includes a blocking portion (1410), which is configured to block movement of an external object into a suction portion (136) disposed in the fan module portion (70) , and a safety support (1430), which is configured to support the blocking portion (1410).

15. The portable air caring apparatus of claim 14, wherein the blocking portion (1410) has the fan module portion (70) which is installed at a position that does not face an air flowing area which is formed between a hub (100), which is disposed at a center of the fan member (90), and a fan base (130),
wherein optionally:
the blocking portion (1410) includes:
a central member (1411) disposed at a central portion of the hub (100);
a first blocking member (1412) whose outer diameter is greater than an outer diameter of the central member (1411) and less than an outer diameter of the hub (100);
a second blocking member (1413) whose inner diameter is greater than the outer diameter of the first blocking member (1412) and greater than an inner diameter of the fan base (130); and
an outer side member (1414) which is larger than an outer diameter of the second blocking member (1413) and is disposed on an outer periphery of the second blocking member (1413),
wherein the safety support (1430) radially extends about the central member (1411) and is connected to the first blocking member (1412), the second blocking member (1413), and the outer side member (1414); and/or
the fan base (130) includes:
a base plate (131) which is formed in the shape of a plate extending in an annular shape and has a hollow formed at a center to allow movement of air; and
a bell mouth (132) which is disposed in an annular shape along an inner side of the base plate (131), which faces the hollow, and surrounds an end portion of the fan member (90).

16. The portable air caring apparatus of any one of claims 7 to 15, wherein the rotation support portion (35) is connected to the housing portion (3300) so as to be restricted from moving and is configured to rotatably support the discharge portion (140),
wherein the rotation support portion (35) includes guide vanes (356) which radially extend about the core member (36) and switch a movement direction of air, which is moved from the fan module portion (70), to guide air in a direction toward the discharge portion (140), and
an inlet angle and an outlet angle of the guide vanes (356) are formed to be different.

17. The portable air caring apparatus of claim 16, wherein the guide vanes (356) include:
a first vane (357) installed in a direction toward the discharge portion (140); and
a second vane (359) which extends in a direction from the first vane (357) toward the fan module portion (70).

18. The portable air caring apparatus of claim 17, wherein, when an angle formed between the first vane (357) and a rotation axis extension line of the fan module portion (70) is A1, and an angle formed between the second vane (359) and the rotation axis extension line of the fan module portion (70) is A2, A1 and A2 are formed to be different from each other, wherein optionally A2 is greater than A1.

19. The portable air caring apparatus of any one of claims 16 to 18, wherein the rotation support portion (35) includes:
a core support portion (350) which supports the core member (36), includes the guide vanes (356), and is fixed to an inner side of the housing portion (3300); and
a ball joint (38) whose lower side is coupled to the core member (36) so as to be restricted from moving and whose upper side is inserted into the discharge portion (140) to rotatably support the discharge portion (140),
wherein optionally the core support portion (350) includes:
a first core support portion (352) configured to support the core member (36); and
a second core support portion (354) installed along an outer periphery of the first core support portion (352) and fixed to the housing portion (3300),
wherein the first core support portion (352) and the second core support portion (354) are connected to each other by the guide vanes (356).
